(19) Europäisches Patentamt
European Patent Office
Office européen des brevets

(11) Veröffentlichungsnummer: **0 418 648 B1**

(12) # EUROPÄISCHE PATENTSCHRIFT

(45) Veröffentlichungstag der Patentschrift: **11.01.95**

(51) Int. Cl.[6]: **C07D 309/30**, A61K 31/35

(21) Anmeldenummer: **90117043.1**

(22) Anmeldetag: **05.09.90**

(54) **4-Hydroxytetrahydropyran-2-one sowie die entsprechenden Dihydroxycarbonsäurederivate, Salze und Ester, Verfahren zu ihrer Herstellung, pharmazeutische Präparate sowie Vorprodukte.**

(30) Priorität: **08.09.89 DE 3929913**

(43) Veröffentlichungstag der Anmeldung:
**27.03.91 Patentblatt 91/13**

(45) Bekanntmachung des Hinweises auf die Patenterteilung:
**11.01.95 Patentblatt 95/02**

(84) Benannte Vertragsstaaten:
**AT BE CH DE DK ES FR GB GR IT LI LU NL SE**

(56) Entgegenhaltungen:
**EP-A- 0 283 217**
**EP-A- 0 341 681**
**DE-A- 3 722 809**

(73) Patentinhaber: **HOECHST AKTIENGESELLSCHAFT**

**D-65926 Frankfurt (DE)**

(72) Erfinder: **Jendralla, Heiner, Dr.**
**Pfortengartenweg 38**
**D-6230 Frankfurt am Main (DE)**
Erfinder: **Wess, Günther, Dr.**
**Langenselbolder Weg 35**
**D-6455 Erlensee (DE)**
Erfinder: **Kesseler, Kurt, Dr.**
**Gluckstrasse 20a**
**D-6232 Bad Soden (DE)**
Erfinder: **Beck, Gerhard, Dr.**
**Gustav-Freytag-Strasse 24**
**D-6000 Frankfurt am Main (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 418 648 B1

Rank Xerox (UK) Business Services
(3.10/3.09/3.3.3)

## Beschreibung

Das Enzym 3-Hydroxy-3-methylglutaryl-Coenzym-A- Reduktase (HMG-CoA-Reduktase) spielt eine zentrale Rolle bei der Biosynthese des Cholesterins [A. Endo, J. Med. Chem. 28, 401 (1985)]. Hemmstoffe dieses Enzyms, insbesondere Mevinolin, Synvinolin und Eptastatin wurden zur Behandlung von Hypercholesterolämikern klinisch getestet. Strukturell vereinfachte, vollsynthetische Analoga dieser Verbindungen wurden beschrieben [T.-J. Lee, TIPS 8, 442 (1987)].

Seit geraumer Zeit wird auch eine Beteiligung der Lipidperoxidation bei der Bildung arteriosklerotischer Läsionen diskutiert. So wurde beobachtet, daß oxidativ modifizierte Formen von low-density-Lipoprotein (LDL) eine große Anreicherung von Cholesterinestern in Makrophagen bewirken [M.A. Fogelman et al., Proc. Natl. Acad. Sci. USA 77, 2214 (1980)] und außerdem zur verstärkten Freisetzung einer Anzahl lysosomaler Enzyme und pathogener Mediatoren führen. Erst in jüngster Zeit wurde an Hand der Probucolanwendung gezeigt, daß die Unterdrückung der LDL-Oxidation für die Vermeidung atherosklerotischer Vorgänge wesentlicher als dessen Wirkung auf den Serumcholesterin-Spiegel ist [D. Steinberg et al., Proc. Natl. Acad. Sci. USA 84, 7725 (1987)].

In der Humanmedizin wird Probucol der Formel

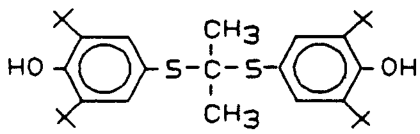

Probucol

zur Behandlung der Hyperlipoproteinämie verwendet.

Nach einem unvollständig erforschten Mechanismus senkt Probucol sowohl LDL als auch HDL. Probucol erhöht die Geschwindigkeit des Katabolismus von LDL [P.J. Nestel, T. Billington, Atherosclerosis 38, 203 (1981)] und erhöht die biliäre Cholesterinausscheidung. Verglichen mit einem potenten Cholesterinbiosyntheseinhibitor (wie Mevinolin) ist die Plasmacholesterin-senkende Wirkung von Probucol allerdings nur schwach ausgeprägt. Neuerdings wird der therapeutische Nutzen von Probucol hauptsächlich darauf zurückgeführt, daß Probucol in vivo die oxidative Veränderung der LDL verhindert [Symposium: New Developments in the Treatment of Hypercholesterolemia - Probucol, verschiedene Autoren, Am. J. Cardiol. 57, 1H-54H (1986)].

Die antioxidativen und Radikalfänger-Eigenschaften von Probucol sind darauf zurückzuführen, daß Probucol freie, eine Radikalkettenreaktion weiterführende Radikale ("chain propagating radicals") durch Abgabe der Wasserstoffatome seiner Hydroxygruppen abfängt. Probucol geht dabei in Oxy-Radikale über, die wegen der Resonanz mit den para-ständigen Schwefelatomen elektronisch stabilisiert und wegen der sterisch abschirmenden ortho-tert.-Butyl-Substituenten nicht zur Fortführung der Radikalkette in der Lage sind [W.A. Pryor et al., J. Am. Chem. Soc. 110, 2224 (1988)].

Zusammengefaßt ergibt sich somit zur Zeit folgendes Partial-Bild zur Pathogenese der Arterosklerose:

Hypercholesterolämie ist u.a. die Folge eines pathologisch verlangsamten LDL-Clearings aufgrund defekter LDL-Rezeptor-Regulation und (oder) -Struktur. Die verlängerte Halbwertszeit der LDL-Partikel im Plasma steigert die Wahrscheinlichkeit ihrer oxidativen Modifizierung.

Oxidierte LDL schädigen das Endothel aufgrund cytotoxischer Eigenschaften und werden von Makrophagen über einen speziellen Scavengerreceptor ohne Feedback-Kontrolle aufgenommen, wobei letztere in Form von Lipid-überladenen "Schaumzellen" absterben, Endothelschädigende lysosomale Enzyme freisetzen und andere pathogene Mechanismen [SMC-Proliferation (SMC = smooth muscle cell in der Gefäßwand) u.s.w] in Gang setzen. Schaumzellbildung und SMC-Proliferation gelten anatomisch-pathologisch als Frühvorgänge einer klinisch noch nicht manifesten Arterosklerose.

Folglich erscheint es äußerst wünschenswert, Wirkstoffe aufzufinden, die nach oraler Gabe und guter Resorption über eine potente Hemmung der HMG-CoA-Reduktase eine starke Senkung der Plasma-Cholesterinspiegel bewirken und gleichzeitig die Probucol-typischen antioxidativen Radikalfänger-Eigenschaften besitzen. Unter allen bisher bekannten, lipidsenkenden Wirkstoffen [Übersicht: D.R. Illingworth, Drugs 33, 259 (1987)] ist diese Kombination von Eigenschaften jedoch unbekannt.

Eine Chance für Probucol-typische Eigenschaften sollten nur diejenigen HMG-CoA-Reduktase-Inhibitoren besitzen, die die notwendige Bedingung erfüllen, daß ein geeignet substituierter Aromat über ein

Heteroatom X an den für HMG-CoA-Reduktase-Hemmung essentiellen 4(R)-Hydroxy-6(S)-methylen-3,4,5,6-tetrahydro-2H-pyran-2-on-Rest (Formel A) oder dessen ringgeöffnete Dihydroxycarbonsäure-Form (Formel B)

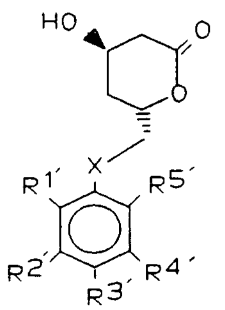

A

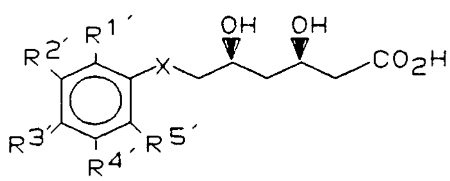

B

gebunden ist.

Verbindungen der allgemeinen Formeln A und B, in denen X Sauerstoff oder Schwefel bedeutet, wurden beschrieben in

a) Europäische Patentanmeldung A-0216127;

b) Deutsche Offenlegungsschrift 3632893 (= Derwent Referat 88-99366/15);

c) Deutsche Offenlegungsschrift 38 19 999 (entsprechend EP-A-0341681, entsprechend US Patentanmeldung Nr. 350,428);

d) C. E. Stokker et al., J. Med. Chem, 28, 347 (1985) Seite 350.

Gemäß deutscher Offenlegungsschrift 38 19 999 liegen besonders potente HMG-CoA-Reduktase-Inhibitoren vor, wenn $R^{1'}$ in Formel A bzw. B die Bedeutung Isopropyl oder Cyclopropyl und $R^{5'}$ die Bedeutung eines substituierten Phenyls, insbesondere p-Fluorphenyl, besitzt. Derartige Verbindungen waren in vitro und in vivo wirksamer als Mevinolin.

Obwohl diese ortho-Substituenten bezüglich ihres sterischen Anspruchs den beiden tert.-Butyl-Substituenten des Probucols nahe kommen, besaßen die Verbindungn der Anmeldung c) keine Probucol-analogen Eigenschaften.

Es wurde nun überraschenderweise gefunden, daß bei Ersatz des p-Substituenten $R^{3'}$ durch die Gruppierung Y-$R^3$, wobei

Y ein Schwefel- oder Sauerstoffatom bedeutet und

$R^3$ einen dem Probucol analogen Rest

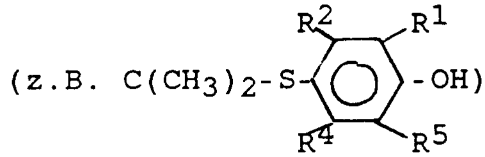

bedeutet,

Verbindungen der allgemeinen Formel I erhalten werden, die sowohl mit den antioxidativen Eigenschaften

des Probucol ausgestattet, als auch z.T. sehr starke HMG-CoA-Reduktase-Inhibitoren sind.

Es wurde gefunden, daß Verbindungen der allgemeinen Formeln I und II

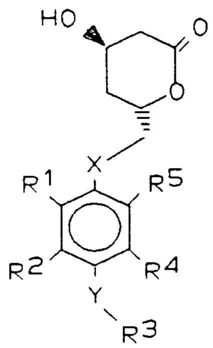

I

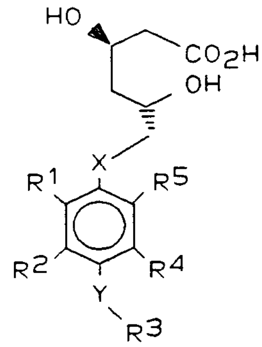

II

sowie deren pharmakologisch verträgliche Salze mit Basen und deren Ester

a) das Enzym HMG-CoA-Reduktase stärker als Mevinolin und in etwa gleichem Maße wie Verbindung C (3(R),5(S)-Dihydroxy-6[2-(4-fluorphenyl)-4,6-diisopropyl-phenoxy]-hexansäure-Natriumsalz, vergl. deutsche Offenlegungsschrift 38 19 999, Beispiel 8a) inhibieren,

b) in Zellkultur die Cholesterinbiosynthese bzw. den Cholesteringehalt stärker als Mevinolin und Verbindung C senken, insbesondere aber wesentlich stärker senken als man allein aufgrund ihrer Hemmwirkung auf die HMG-CoA-Reduktase vermuten würde.

c) nach p.o. Gabe an Kaninchen (5 mg/kg/Tag) die Plasmacholesterinspiegel wesentlich stärker senken als Mevinolin in gleicher Dosis, insbesondere aber die Plasmacholesterinspeigel wesentlich stärker senken, als man allein aufgrund ihrer Hemmwirkung auf die HMG-CoA-Reduktase vermuten würde (vgl. Tabelle 4, hier auch Angaben zu Verbindung C).

d) nach p.o. Gabe an Wistar-Ratten (100 mg/kg/Tag) eine VLDL-Senkung und eine starke LDL-Senkung bewirken, ohne daß (im Gegensatz zu Clofibrat und Probucol) das HDL erniedrigt wird. Die Plasmacholesterinsenkung im Rattenversuch ist zweifelsfrei nicht auf HMG-CoA-Reduktase-Hemmung zurückzuführen, da wegen rascher und starker Gegenregulation der Enzymsynthese ("Enzyminduktion") HMG-CoA-Reduktasehemmer an der Ratte keine Plasmacholesterinsenkung bewirken [siehe z.B. Y. Tsujita et al., Biochim. Biophys. Acta 877, 50 (1986)].

e) in vitro die mikrosomale Lipidperoxidation hemmen und folglich Radikalinhibitoren/Antioxidantien sind. In Fällen, in denen Verbindungen der Formeln I oder II diese Eigenschaften nicht (nennenswert) besaßen, wurde sie bei den zugrundeliegenden Phenolbausteinen der Formel III

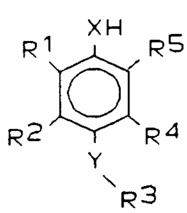

III

aufgefunden. Letztere wurden bei Umsetzung von I oder II mit Leberhomogenat als wesentlicher Metabolit aufgefunden, treten also in vivo möglicherweise als Metabolit der Verbindungen der Formeln I oder II auf.

Gegenstand der vorliegenden Erfindung sind darum Verbindungen der allgemeinen Formel I,

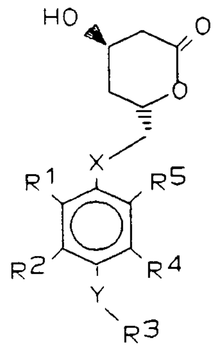

I

in der

- X und Y: gleich oder verschieden sind und ein Sauerstoffatom oder ein Schwefelatom bedeuten,
- $R^1$ und $R^5$: beide Isopropyl bedeuten oder verschieden sind und einen Isopropyl-, Cyclopropyl- oder Phenylrest bedeuten, wobei letzterer im Kern 1- bis 3-fach substituiert sein kann mit Fluor, Chlor, Brom, Trifluormethyl und/oder Alkyl oder Alkoxy mit je 1 bis 4 Kohlenstoffatomen,
- $R^2$ und $R^4$: gleich oder verschieden sind und Wasserstoff oder einen Isopropyl-, Cyclopropyl- oder Phenylrest bedeuten, wobei letzterer im Kern 1-3-fach substituiert sein kann mit Fluor, Chlor, Brom, Trifluormethyl und/oder Alkyl oder Alkoxy mit 1 bis 4 Kohlenstoffatomen,
- $R^3$:

a) Wasserstoff, Methyl oder Ethyl

b) einen geradkettigen oder verzweigten Alkylrest mit 3 bis 8 Kohlenstoffatomen, der substituiert sein kann mit dem Rest der Formel

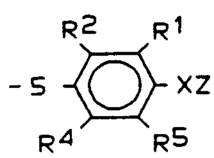

worin X, $R^1$, $R^2$, $R^4$, $R^5$ die oben angegebenen Bedeutungen haben und Z entweder ein Wasserstoffatom, ein pharmakologisch verträgliches Kation oder den 4(R)-Hydroxy-6-(S)-methylen-3,4,5,6-tetrahydro-2H-pyran-2-on-Rest der Formel

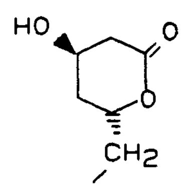

oder den entsprechenden 3(R),5(S)-Dihydroxyhexansäure-6-yl-Rest der Formel

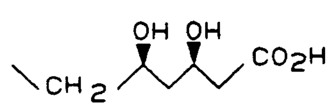

,

dessen pharmakologisch verträgliche Salze mit Basen oder dessen pharmakologisch verträgliche Ester bedeutet,

c) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 Kohlenstoffatomen, oder

d) Acetyl unter der Voraussetzung, daß Y Sauerstoff ist, bedeutet,
sowie die entsprechenden offenkettigen Dihydroxycarbonsäuren der allgemeinen Formel II

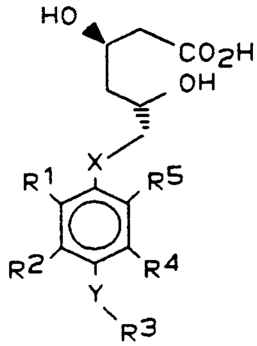
,

II

deren pharmakologisch verträgliche Salze mit Basen und deren pharmakologisch verträglich Ester.

Bevorzugt sind solche Verbindungen, in denen die Substituenten $R^1$ und $R^5$ nicht gleichzeitig Phenyl und substituiertes Phenyl oder unterschiedlich substituiertes Phenyl darstellen.

Bevorzugt haben die Reste in den allgemeinen Formeln I und II folgende Bedeutung:

X : Sauerstoff
Y : Sauerstoff oder Schwefel
$R^1$ : Isopropyl oder Cyclopropyl
$R^2$ : Wasserstoff, Isopropyl oder p-Fluorphenyl
$R^3$ : Wasserstoff, Acetyl, Isopropyl, p-Fluorphenyl,

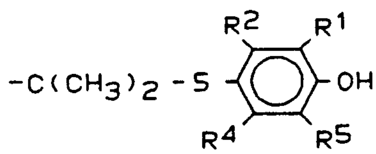
,

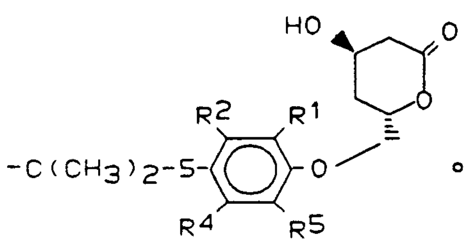
oder
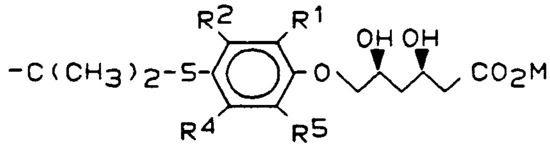

**(M = Wasserstoff oder Natrium)**

$R^4$ : Wasserstoff, Isopropyl oder p-Fluorphenyl
$R^5$ : Isopropyl oder p-Fluorphenyl

Besonders bevorzugt sind die folgenden Verbindungen der allgemeinen Formel I

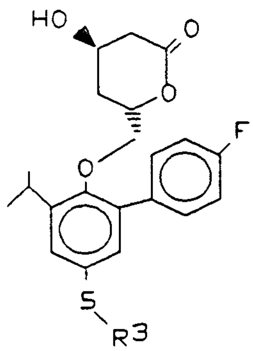

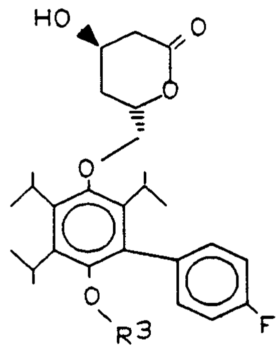

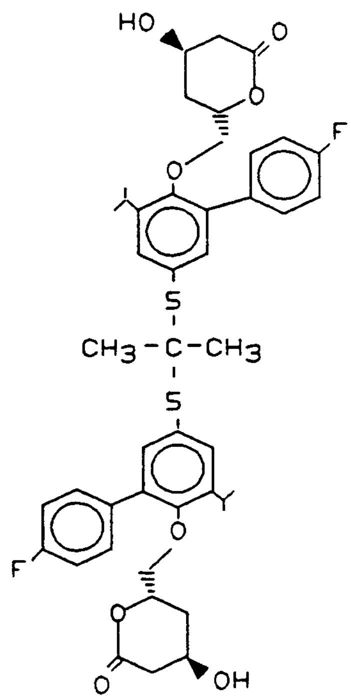

R3 = p-Fluorphenyl, p-Fluor-m-methyl-phenyl oder Isopropyl

R3 = Wasserstoff oder Acetyl

sowie die entsprechenden offenkettigen Dihydroxycarbonsäuren der allgemeinen Formel II, deren pharmakologisch verträglich Salze mit Basen und deren pharmakologisch verträgliche Ester.

Die Erfindung betrifft ferner ein Verfahren zur Herstellung der Verbindungen der Formel I, sowie der entsprechenden offenkettigen Dihydroxycarbonsäuren der Formel II, von deren pharmakologisch verträglichen Salzen mit Basen und deren pharmakologisch verträglichen Estern.

Das Verfahren ist dadurch gekennzeichnet, daß man

a) entsprechend substituierte Phenole oder Thiophenole der Formel III,

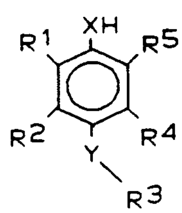

III

worin X, Y, $R^1$, $R^2$, $R^3$, $R^4$ und $R^5$ die zur Formel I angegebenen Bedeutungen haben, mit dem optisch reinen Mesylat der Formel IV

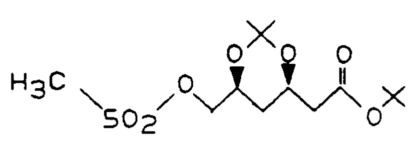

IV

zum Acetonid der Formel V

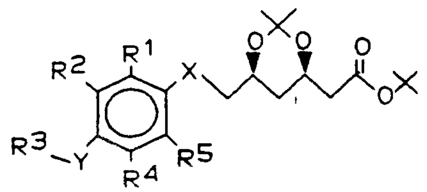

V

umsetzt,

b) Verbindungen der Formel V unter Abspaltung der Schutzgruppe in $\beta,\delta$-Dihydroxycarbonsäuretert.-butylester der Formel II/1,

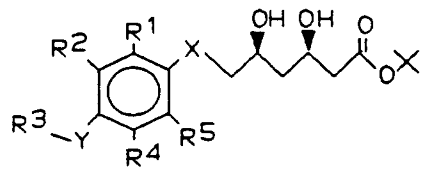

II/1

worin X, Y, $R^1$, $R^2$, $R^3$, $R^4$ und $R^5$ die zur Formel I angegebenen Bedeutungen haben, überführt

c) die tert.-Butylester der Formel II/1 zu Salzen der Formel

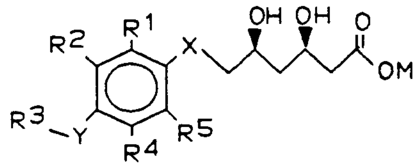

II/2

II/2, in der X, Y, $R^1$, $R^2$, $R^3$, $R^4$ und $R^5$ die zur Formel I angegebenen Bedeutungen haben und M das pharmakologisch verträgliche Kation einer Base bedeutet, vorzugsweise das Natriumkation, verseift, wobei gegebenenfalls vorhandene Schutzgruppen ebenfalls abgespaltet werden können,

d) die tert.-Butylester der Formel II/1 oder gegebenenfalls die Salze der Formel II/2 zu den $\beta$-Hydroxylaktonen der Formel I cyclisiert,

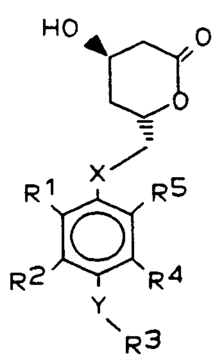

I

e) gegebenenfalls die Hydroxylaktone der Formel I in die entsprechenden offenkettigen Dihydroxycarbonsäuren der Formel II, deren Salze oder deren Ester überführt, gegebenenfalls die Salze oder Ester in die freien Dihydroxycarbonsäuren der Formel II oder gegebenenfalls die Dihydroxycarbonsäuren II in die

Salze oder Ester überführt.

Wie sich aus dem beschriebenen Verfahren ergibt, wurden die Verbindungen der allgemeinen Formeln I und II und deren Salze und Ester optisch rein mit der gezeichneten Absolutkonfiguration hergestellt. Die Verbindungen bilden in dieser Absolutkonfiguration einen besonders bevorzugten Gegenstand der Erfindung. Da die Äpfelsäure, das Ausgansmaterial zur Herstellung des Mesylats der Formel IV, auch in der umgekehrten Absolutkofiguration [D(+)-Äpfelsäure] oder als Racemat [DL-Äpfelsäure] kommerziell verfügbar ist, können auf die gleiche Weise auch die Antipoden der Verbindungen der allgemeinen Formeln I und II und deren Salze und Ester beziehungsweise die Racemate der Verbindungen der allgemeinen Formeln I und II und deren Salze und Ester hergestellt werden. Weiterhin kann man aus den Racematen der Verbindungen der allgemeinen Formeln I und II und deren Salzen und Estern durch die klassischen Methoden der Racematspaltung mehr oder weniger optisch angereicherte Verbindungen beider Absolutkonfigurationen gewinnen. Die Antipoden der Verbindungen der allgemeinen Formeln I und II und deren Salze und Ester, die Racemate, sowie optisch angereicherte Verbindungen gehören damit ebenso zum Gegenstand der Erfindung.

**Verfahrensschritt a)**

Das Mesylat der Formel IV wird durch Mesylierung der optisch reinen Hydroxyverbindung der Formel VI

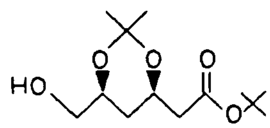

erhalten. Die Herstellung der Verbindung der Formel VI aus kommerziell verfügbarer L(-)-Äpfelsäure ist in der EP A-0 319 847 beschrieben. Die Reaktion der Hydroxyverbindung VI zum Mesylat IV erfolgt z. B. durch Umsetzung mit Methansulfonsäurechlorid in Gegenwart einer schwachen Base. Das Methansulfonsäurechlorid wird vorteilhaft im geringen Überschuß (1,05 - 1,5 Äquivalente) eingesetzt. Vorteilhaft ist der Einsatz einer Methylenchlorid/Pyridin-Mischung als Lösungsmittel. Wegen der geringen thermischen Stabilität vieler Mesylate empfiehlt es sich, die Reaktion unter Kühlung durchzuführen. Vorteilhaft wird die Reaktionstemperatur durch Eiskühlung nahe 0 °C gehalten. Bei zweckmäßiger Aufarbeitung (siehe Verfahrensbeispiel 26) kristallisiert das Mesylat IV spontan und kann durch Absaugen und Waschen in hoher Ausbeute und Reinheit erhalten werden.

Die Kupplung von III mit IV erfolgt bevorzugt in Gegenwart einer Base in aprotischen, polaren Lösungsmitteln. Besonders bewährt hat sich die Verwendung von Kaliumcarbonat als Base und DMSO oder HMPT als Lösunsgsmittel. Beim Einsatz von schwefelhaltigen Verbindungen III (d.h. X und/oder Y = S) wird zweckmäßigerweise HMPT als Lösungsmittel verwendet. Gelegentlich beschleunigt der Einsatz einer katalytischen Menge 18-Krone-6 die Reaktion und steigert die Ausbeute an V. Das Mesylat IV wird zweckmäßig in geringem Überschuß (ca. 1,1 Äquivalente) eingesetzt. Die Kupplung kann im Temperaturbereich von 40 - ca. 90 °C durchgeführt werden. Die Reinheit des Rohprodukts sinkt deutlich mit steigender Reaktionstemperatur. Die optimale Temperatur und die zugehörige Reaktionsdauer hängen von der Natur der Substituenten, insbesondere der sterischen Abschirmung der nucleophilen Gruppe XH, in der Verbindung der Formel III ab. Zur Herstellung der besonders bevorzugten Verbindungen war eine Reaktionstemperatur von 60-70 °C und eine Reaktionsdauer von ca. 12 - 18 Std. vorteilhaft. Außer mit der Acetonidgruppierung können die beiden Hydroxygruppen des Mesylats IV selbstverständlich auch mit anderen Schutzgruppen versehen sein, die gegenüber den basischen Kupplungsbedingungen stabil sind, z.B. tert.-Butyldiphenylsilyl-Gruppen.

**Verfahrensschritt b)**

Zur Herstellung der Verbindungen der Formel II/1 kann im Prinzip jede der zahlreichen Methoden herangezogen werden, die in der Literatur zur Spaltung von Ketalen beschrieben wurden. Bevorzugt ist die Verwendung von 2-normaler wäßriger Salzsäure als Katalysator in homogener organischer Lösung (THF/Ethanol) bei Raumtemperatur.

**Verfahrensschritt c)**

Dieser Schritt stellt eine basische Esterverseifung dar. Sie kann mit einer großen Anzahl pharmakologisch verträglicher Basen in wäßrigem, wäßrig-organischem oder, falls als Base Metallhydroxide verwendet werden, auch in organisch-aprotischen Lösungsmitteln durchgeführt werden. Bevorzugt ist die Verwendung eines 1:1 (Vol/Vol) Gemischs von exakt 1,0 Äquivalenten einer wäßrigen 1-normalen Natronlauge und Ethanol bei Raumtemperatur. Natronlaugeüberschüsse sollten vermieden werden, da Natriumhydroxid und das Natriumsalz II/2 beide relativ gute Wasserlöslichkeit besitzen und darum nur schwer getrennt werden können. Der Ablauf der Verseifung kann mit Kieselgel-DC (Chloroform/Methanol 5:1) verfolgt werden. Bei den meisten (aber nicht bei allen) Estern der Formel II/1 erkennt man die weitgehende Verseifung unter den genannten Bedingungen auch daran, daß die anfängliche Suspension in eine klare, homogene Lösung übergeht.

**Verfahrensschritt d)**

Die direkte Umwandlung von tert.-Butylestern der allgemeinen Formel II/1 in die Hydroxylaktone der Formel I gelingt mit einem Überschuß einer Reihe starker Säuren, bevorzugt organischer Säuren. Im Prinzip ist jedes organische Lösungsmittel geeignet, das gutes Lösungsvermögen für die tert.-Butylester II/1 besitzt und genügend inert gegenüber der starken Säure ist. Der Einsatz von Trifluoressigsäure in Methylenchloridlösung bei Raumtemperatur ist bevorzugt. Die Reaktionszeit beträgt in der Regel 1-5 Stunden. Es empfiehlt sich zur Vermeidung von Nebenreaktionen die DC-Kontrolle des Reaktionsablaufs. Es ist vorteilhaft, das Reaktionsgemisch durch Zugabe von Natriumhydrogencarbonat unter Kühlung abzustumpfen und dann mit Natriumcarbonat neutral zu stellen. Natriumcarbonat-Überschuß ist zu vermeiden, da die Lactone I auch unter schwach basischen Bedingungen sehr leicht zu den Salzen II/2 verseift werden. Die Laktone I können extraktiv in hoher Ausbeute und Reinheit gewonnen werden.

Aus den Salzen II/2 kann man die Laktone I nach verschiedenen Prozeduren erhalten. In jedem Fall werden die Salze II/2 durch vorsichtiges Ansäuern, gefolgt von Ethylacetat-Extraktion, in die freien Dihydroxycarbonsäuren der Formel II umgewandelt. Diese können durch Behandlung mit 1-1,5 Äquivalenten eines wasserabspaltenden Reagenzes, z.B. N,N'-Dicyclohexylcarbodiimid oder bevorzugt eines wasserlöslichen Carbodiimids wie N-Cyclohexyl-N'-[2'-(N''-methylmorpholinium)-ethyl] carbodiimid-para-toluolsulfonat (vgl. M. Fieser "Reagents for Organic Synthesis" 1, 181 und 11, 151) in einem inerten organischen Lösungsmittel, bevorzugt Methylenchlorid, bei 10-35 °C, bevorzugt bei Raumtemperatur, zum Lakton I cyclisiert werden. Bessere Ausbeuten und Produktreinheiten erhält man in der Regel, wenn man stattdessen die Carboxylgruppe der freien Dihydroxycarbonsäuren der Formel II zu einem intermediären Derivat umsetzt, das bezüglich einem intramolekularen nucleophilen Angriff der δ-Hydroxygruppe aktiviert ist. Die Literatur beschreibt eine große Zahl von derartigen Methoden zur Carboxylaktivierung. Sehr verbreitet ist die Bildung von Säurehalogeniden, Säureimidazoliden, Aktivestern oder gemischten Anhydriden. Vorzugsweise wird die Carbonsäure der Formel II bei 0 - -10 °C in absolutem THF mit 1,1 Äquivalenten Triäthylamin und 1,0 Äquivalenten Chlorameisensäureethylester umgesetzt. In Reaktionszeiten von 1-2 Std. bilden sich die Laktone der Formel I in fast quantitativer Ausbeute.

**Verfahrensschritt e)**

Diese Tranformationen sind trivial und sind entsprechend den Angaben im Stand der Technik durchzuführen z. B. mit Basen im Falle der Salzbildung.

Zur Durchführung des Verfahrensschritts a) werden als Ausgangsmaterialien substituierte Phenole oder Thiophenole der Formel III benötigt. Verbindungen der Formel III, worin X, Y, $R^1$, $R^2$, $R^3$, $R^4$, $R^5$ die zur Formel I angegebenen Bedeutungen haben, sind neu. Sie gehören deshalb ebenfalls zum Gegenstand der vorliegenden Erfindung.

In der deutschen Offenlegungsschrift 38 19 999 wird ein Verfahren zur Arylierung von Phenolen vorgeschlagen, das darin besteht, die Phenole zunächst einer elektrophilen aromatischen Halogenierung zu unterwerfen (Halogen = Brom oder Jod) und das Monohalogenphenol dann unter Palladium-(O)-Katalyse mit einer Arylgrignardverbindung zu behandeln, wobei es zu einem positionsspezifischen Halogen-Aryl-Austausch kommt: der Arylrest tritt nur in die Position ein, in der sich vorher das Halogenatom befand. Die Verbindungen der Formel III wurden unter Verwendung dieser Reaktion als Schlüsselschritt aufgebaut. Aus den Phenolen sind die Thiophenole entsprechend den Angaben in der DE-A 36 32 893 herstellbar.

**Schema 1 ($R^5$ = Phenyl oder substituiertes Phenyl)**

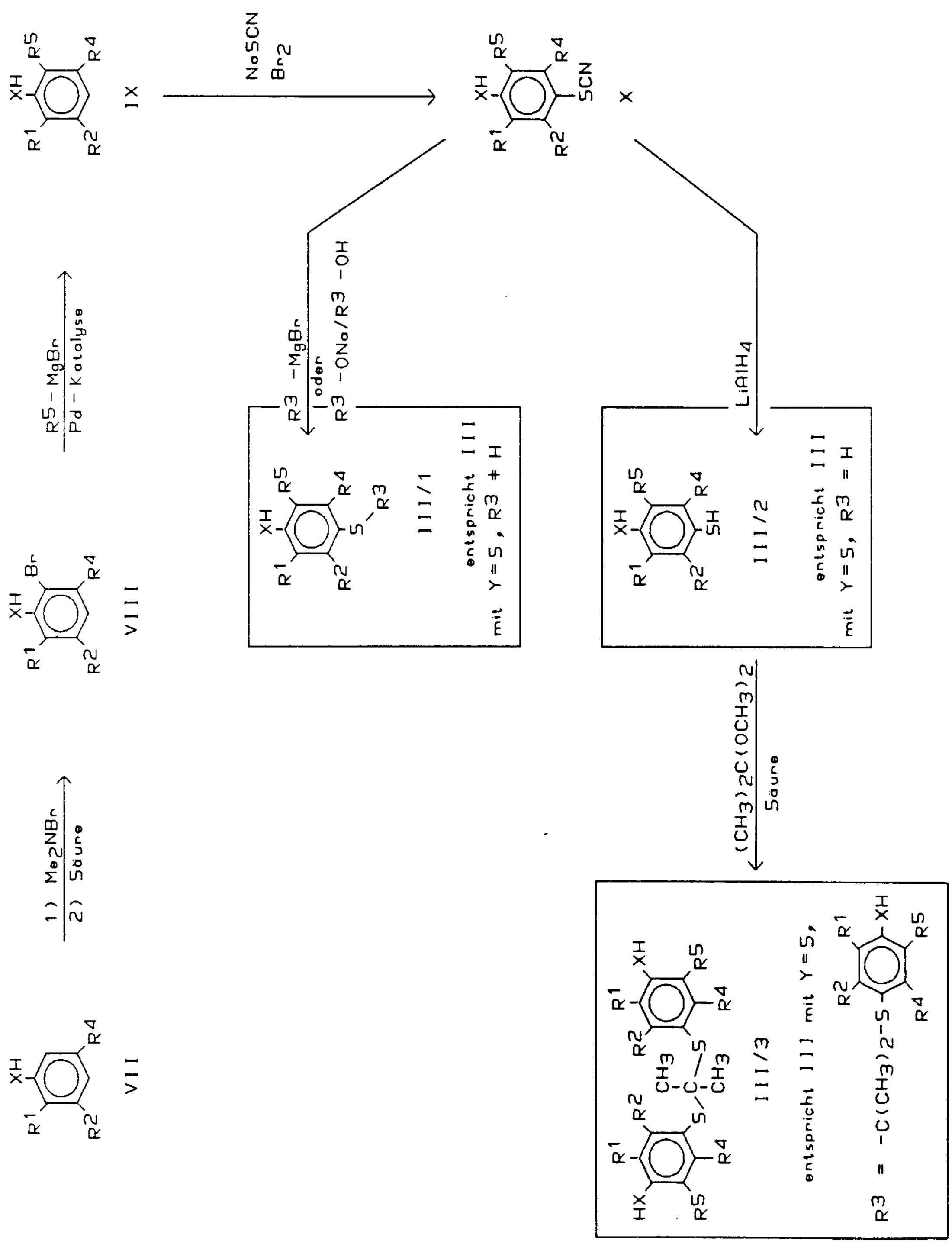


Ausgangsmaterialien für die Synthese von Verbindungen der Formel III sind gemäß Schema 1 Phenole oder Thiophenole der allgemeinen Formel VII bzw. gemäß Schema 3 Benzole der Formel XIII. Die Verbindungen VII sind kommerziell erhältlich oder literaturbekannt, soweit $R^2$ oder $R^4$ nicht die Bedeutung eines (substituierten) Phenylrests haben. Soll $R^2$ oder $R^4$ in Formel III die Bedeutung eines (substituieren) Phenylrests besitzen, so können die Verbindungen VII analog der oben beschriebenen Reaktion erhalten werden, oder es kommt die Synthese gemäß Schema 3 zur Anwendung.

Mit N-Haloaminen kann man ensprechend den Angaben in der Literatur eine regiospezifische ortho-Halogenierung von Phenolen erreichen [E. Schmitz, I. Pagenkopf, J. Prakt. Chem. 327, 998 (1985)]. Anwendungsbreite und Verfahrensvarianten wurden von Schmitz und Pagenkopf ausführlich beschrieben. Vorzugsweise werden 1,1 Äquivalente einer kommerziell erhältlichen 40 %igen wäßrigen Dimethylamin-Lösung bei ca. -10 °C mit der wäßrigen Natriumhypobromid-Lösung aus 3,2 Äquivalenten Natriumhydroxid und 1,02 Äquivalenten Brom zum N-Bromdimethylamin umgesetzt, das mit Tetrachlorkohlenstoff extrahiert, dann getrocknet wird. Diese Lösung wird dann bei ca. -10 °C zur Lösung von 1 Äquivalent der Verbindung VII in Tetrachlorkohlenstoff getropft. Das ortho-bromierte Dimethylammoniumsalz fällt aus, wird abgesaugt und durch Kochen mit 2N Schwefelsäure in die freie Verbindung VIII überführt. Da gemäß Schema 1 als Rest $R^5$ Phenyl oder substituiertes Phenyl eingeführt wird, wird Verbindung VIII unter Palladium-Katalyse mit dem Phenylgrignard-Reagenz $R^5$-MgBr umgesetzt zu Verbindungen der Formel IX. Die Durchführungsvarianten der Kupplungsreaktion und Alternativen für Palladium wurden in der deutschen Patentanmeldung 38 19 999.8 vorgeschlagen. Für die Umwandlung von VIII und IX werden bevorzugt 3 Äquivalente des Grignard-Reagenzes aus $R^5$-Br und einem geringen Überschuß Magnesiumspänen in THF hergestellt und diese Lösung wird dann bei 60 °C zu der Lösung von 1 Äquivalent der Verbindung VIII und 3-5 Mol% Tetrakis(triphenylphosphin)-Palladium(O) in THF transferiert. Vollständige Reaktion erfolgt, je nach der Natur der Substituenten $R^1$, $R^2$, $R^4$ und $R^5$ während 1 Std. - 12 Std. bei 60 °C. Die Einführung der Schwefelfunktionalität in die para-Position, wobei Verbindungen X entstehen, erfolgt durch Rhodanierung. Für diesen Schritt gibt es mehrere Verfahrensweisen, die ausführlich beschrieben und verglichen wurden [z. B. J. L. Wood in Org. React. 3, 240-266 (1946) und J.H. Clark et al., J. Chem. Soc. Chem. Commun., 81 (1989)]. Vorzugsweise werden 1 Äquivalent der Verbindung der allgemeinen Formel IX und 5 Äquivalente Natriumthiocyanat in Methanol suspendiert und bei ca. 15 °C die Lösung von 1,5 Äquivalenten Brom in Methanol langsam zugetropft. Die Reaktion beruht auf der Bildung und in situ-Umsetzung von Dirhodan $(SCN)_2$.

Tropft man eine Lösung eines Thiocyanats der allgemeinen Formel X, bevorzugt bei 25-50 °C, zu bevorzugt 6 Äquivalenten einer Alkyl- oder Arylgrignard-Verbindung $R^3$-MgBr, bevorzugt in THF als Lösungsmittel, so erhält man Thioether der allgemeinen Formel III/1. Wenn es sich bei $R^3$ um einen aliphatischen Rest handelt, erhält man die Thioether III/1 auch, wenn man eine Lösung des Thiocyanats der allgemeinen Formel X und eines Überschusses des Natriumalkoholats $R^3ONa$ (bevorzugt ca. 2 Äquivalente) im Alkohol $R^3OH$ unter Rückfluß kocht. Die Thioether der Formel III/1 entsprechen der allgemeinen Formel III, mit der Einschränkung, daß das Y Schwefel sein muß.
Es sind zahlreiche Methoden bekannt, um Thiocyanate in die entsprechenden Thiole zu überführen. Eine Übersicht findet man z. B. bei K.-D. Gundermann, K. Hümke in "Methoden der Organischen Chemie" (Houben-Weyl), Band E 11; "Organische Schwefelverbindungen, Teil 1", Thieme Verlag (Stuttgart, 1985) Seite 59: "Thiole aus Thiocyansäureestern". Vorzugsweise wird die reduktive Spaltung der Thiocyanate der allgemeinen Formel X zu Thiolen der allgemeinen Formel III/2 mit ca. 1,7 Mol-Äquivalenten Lithiumaluminiumhydrid in Tetrahydrofuran unter Rückfluß durchgeführt. Die Ausbeuten sind bei dieser Verfahrensweise sehr hoch (> 90 %). In Gegenwart von Luftsauerstoff erleiden die Thiole der allgemeinen Formel III/2 rasche oxidative Dimerisierung zu den entsprechenden Disulfiden. Ihre Herstellung erfolgt darum zweckmäßigerweise nach gründlicher Entgasung der Reaktionslösung unter einer Inertgasatmosphäre. Die Thiole der Formel III/2 entsprechen der Formel III, mit der Einschränkung, daß Y Schwefel und $R^3$ Wasserstoff ist.

Es gibt mehrere alternative Verfahren zur Einführung der Thiol-Gruppierung in Aromaten der allgemeinen Formel IX. Eine Überschicht findet man z. B. bei K.-D. Gundermann, J. Hümke in "Methoden der organischen Chemie" (Houben-Weyl), Band E11, "Organische Schwefelverbindungen, Teil 1", Thieme Verlag (Stuttgart 1985), Seite 32-63: "Herstellung von Thiolen". In Schema 1 ist ein Syntheseweg ausführlich beschrieben.

Die Umsetzung der Thiole der allgemeinen Formel III/2 zu den Aceton-dithioketalen der allgemeinen Formel III/3 ist im Prinzip mit Aceton in Gegenwart eines sauren Katalysators analog der für Probucol beschriebenen Methode möglich. [M. B. Neuworth et al., J. Med. Chem. 13, 722 (1970)].
Verbindungen der Formel III/3 werden in 85-100 %iger Ausbeute erhalten, wenn man die Thiole der Formel III/2 in einem inerten Lösungsmittel, bevorzugt Benzol, mit 1,25-1,50 Äquivalenten 2,2-Dimethoxypropan in Gegenwart katalytischer Mengen p-Toluolsulfonsäure auf ca. 80 °C erwärmt. Die Reaktionszeit hängt von der Natur der Substituenten $R^1$, $R^2$, $R^4$, $R^5$ ab und beträgt in der Regel 2-12 Std. Als Zwischenprodukt der Reaktion tritt das polare, gemischte Sauerstoff/Schwefel-Ketal der allgemeinen Formel XI auf,

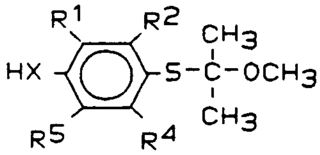

X I

das bei der Durchführung der Reaktion bei Raumtemperatur (1-2 Std.) das Hauptprodukt darstellt. Es wandelt sich im Reaktionsgemisch bei Raumtemperatur langsam, bei ca. 80 °C rasch in das weniger polare Produkt der Formel III/3 um. Die Thioketale der Formel III/3 entsprechen der allgemeinen Formel III, mit der Einschränkung, daß Y Schwefel und $R^3$ der Rest

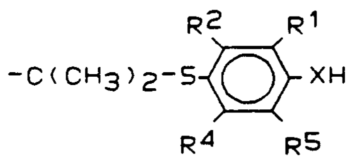

ist.

Das erfindungsgemäße Verfahren wird nachfolgend durch die Umsetzung von Verbindung III/3 zu den Endprodukten der Formel I gemäß Schema 2 erläutert.

Die Umsetzung der Thioketale der Formel III/3 mit dem Mesylat IV gemäß Verfahrensschritt a) führt zu zwei unterschiedlichen Kupplungsprodukten, nämlich dem Monokupplungsprodukt V/1 und dem Dikupplungsproudkt XII. V/1 und XII können säulenchromatographisch problemlos getrennt werden. Sie entstehen bei der Kupplung stets beide, jedoch kann man ihren Anteil über die Reaktionsbedingungen beeinflussen. Verwendet man z. B. nur 1,2 Mol des Mesylats IV pro Mol Thioketal III/3, so entstehen V/1 und XII etwa im Verhältnis 2:1 (ca. 70 % Gesamtausbeute). Werden hingegen 2,5 Mol des Mesylats IV pro Mol Thioketal III/3 eingesetzt und die Reaktionszeit verlängert, ist das Verhältnis V/1 zu XII < 1:3.

Die getrennten Produkte V/1 und XII können dann gemäß den Verfahrensschritten b), c), d) und gegebenenfalls e) in die Mono-(hydroxylaktone) der Formel I/1, die Bis-(hydroxylaktone) der Formel I/2 (beide Formeln sind Spezialfälle der allgemeinen Formel I), deren offenkettige Dihydroxycarbonsäuren der allgemeinen Formel II, deren Salze oder deren Ester überführt werden.

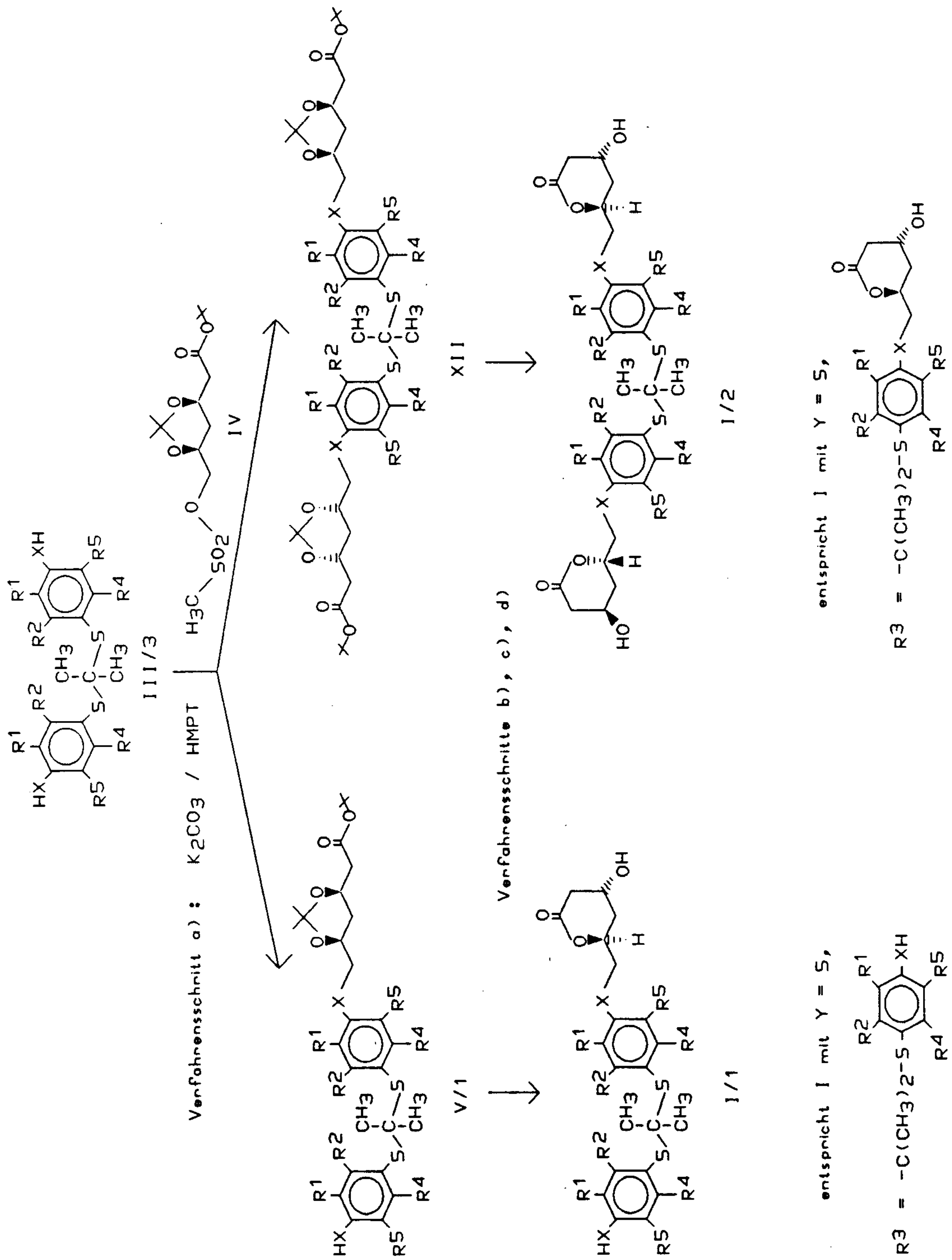
III/3
Verfahrensschritt a) : $K_2CO_3$ / HMPT
IV
XII
V/1
Verfahrensschritte b), c), d)
I/2
I/1
entspricht I mit Y = S,
R3 =
entspricht I mit Y = S,
R3 =

Schema 2

**Schema 3 (zur Bedeutung von $R^6$, $R^7$, $R^8$, $R^9$ siehe Text)**

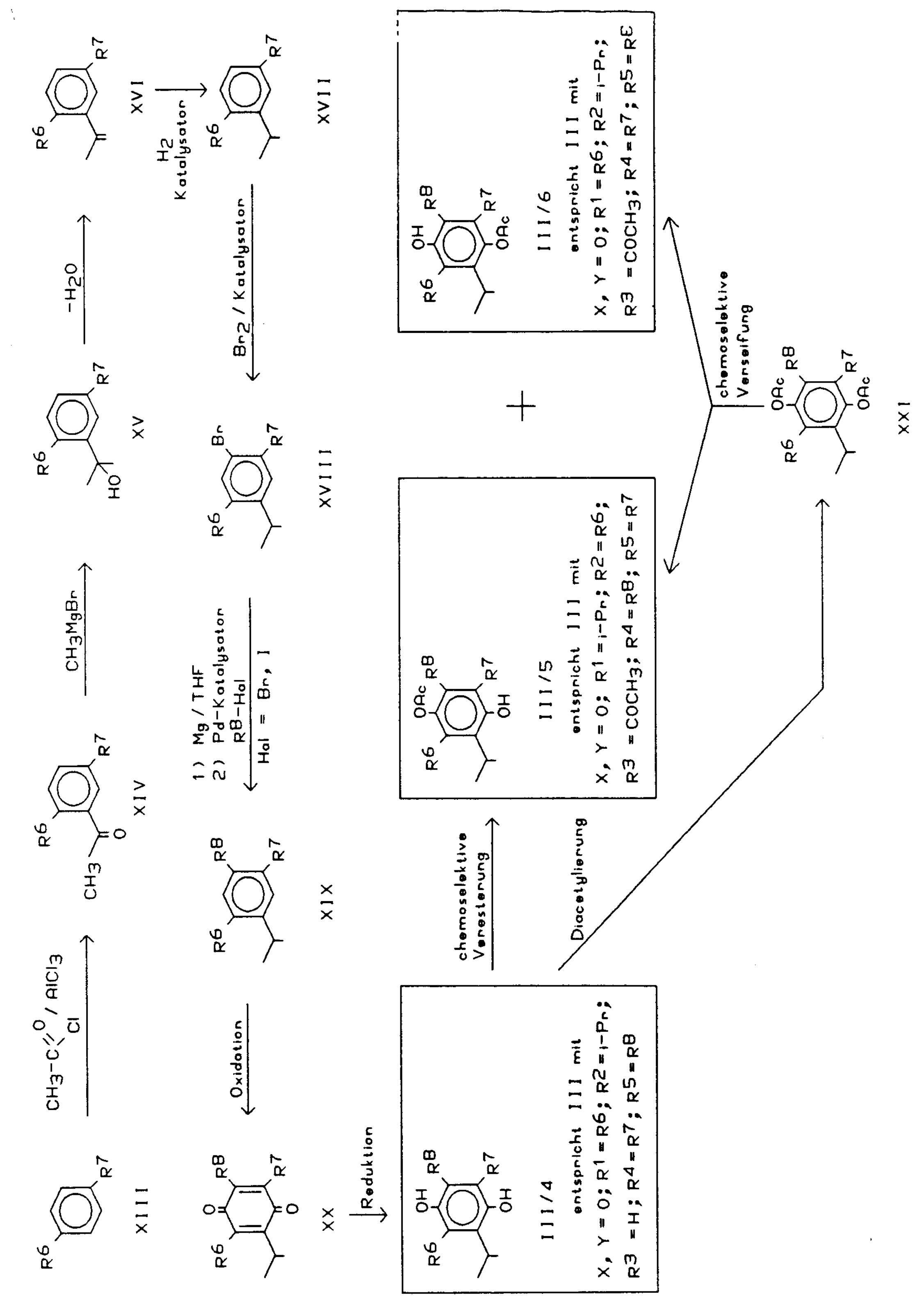


Substituierte Hydrochinone (Phenole der Formel III, worin X und Y Sauerstoff darstellen) der allgemeinen Formeln III/4, III/5 und III/6, die allesamt Spezialfälle der Bausteine mit der allgemeinen Formel III darstellen, können gemäß der in Schema 3 angegebenen Synthesesequenz hergestellt werden. Besonderheit dieses Verfahrens ist, daß die beiden Hydroxygruppen erst am Sequenzende in den aromatischen Kohlenwasserstoff der allgemeinen Formel XIX eingeführt werden. Weiterhin können die beiden Hydroxygruppen der

Verbindungen der allgemeinen Formel III/4 durch komplementäre Verfahren derart differenziert werden, daß sowohl die eine, als auch die andere Hydroxygruppe spezifisch die Kupplung mit dem Mesylat IV begehen können. In Monoacetaten der allgemeinen Formel III/5 wurde z.B. die "obere" Hydroxygruppe der Hydrochinone III/4 geschützt; nur die "untere Hydroxygruppe verbleibt kupplungsfähig für die Umsetzung mit Verbindungen der Formel IV und erhält somit die Bedeutung der XH-Gruppe der allgemeinen Formel III mit X = Sauerstoff. Die Isopropylgruppe und der Substituent $R^7$ der Formel III/5 entsprechen damit den Substituenten $R^1$ und $R^5$ der allgemeinen Formeln I bis III, während die Substituenten $R^6$ und $R^8$ von III/5 $R^2$ und $R^4$ von I bis III entsprechen.

Im Monoacetat der allgemeinen Formel III/6 ist hingegen die "untere" Hydroxygruppe der Hydrochinone III/4 geschützt. In III/6 besitzt folglich die "obere" Hydroxygruppe die Bedeutung der XH-Gruppe der allgemeinen Formel III mit X = Sauerstoff. Die Isopropylgruppe und der Substituent $R^7$ der Formel III/6 entsprechen damit den Substituenten $R^2$ und $R^4$ der allgemeinen Formeln I bis III, wahrend die Substituenten $R^6$ und $R^8$ von III/6 $R^1$ und $R^5$ von I bis III entsprechen.

Man erkennt, daß man je nach Wahl der komplementären Monoacetylierungsverfahren, den Substituentenpaaren $R^6/R^8$ und i-Pr/$R^7$ die Bedeutung von ortho-Substituenten $R^1/R^5$ oder meta-Substituenten $R^2/R^4$ in den Endprodukten der allgemeinen Formeln I und II verleihen kann.

Die direkte chemoselektive Veresterung ist an Verbindungen der allgemeinen Formel III/4 stets komplementär zum zweistufigen Verfahren der Diacetylierung, gefolgt von chemoselektiver Verseifung. Das Substituentenpaar i-Pr/$R^7$ hat entweder einen höheren oder einen geringeren sterischen Anspruch als das Substituentenpaar $R^6/R^8$.

Unter milden Bedingungen erfolgt die Monoacetylierung deutlich rascher an der weniger sterisch gehinderten der beiden Hydroxygruppen von III/4. Erzwingt man hingegen durch drastischere Reaktionsbedingungen und einen Überschuß des Acetylierungsmittel eine Diacetylierung zu XXI, so wird bei der anschließenden Verseifung die sterisch weniger gehinderte Hydroxygruppe deutlich rascher freigesetzt als die gehindertere. Man erhält also verglichen mit der direkten Monoacetylierung bevorzugt das entgegengesetzte Produkt.

Ausgangsmaterial für die Synthese der Hydrochinone III/4 bzw. der komplementär geschützten Hydrochinone der Formeln III/5 und III/6 sind die substituierten Benzole der Formel XIII, die literaturbekannt und größtenteils kommerziell erhältlich sind.

Je nachdem, welche der beiden komplementären Acetylierungen später durchgeführt wird, hat $R^6$ die Bedeutung die für $R^1$ bzw. $R^5$ in der Formel I angegeben wurde und $R^7$ die Bedeutung die für $R^2$ bzw. $R^4$ in der Formel I angegeben wurde - oder umgekehrt.

Verbindungen der allgemeinen Formel XIV erhält man durch Friedel-Crafts Acetylierung der Benzole XIII. Die Reaktion wird unter Verwendung eines geringen molaren Überschusses, bevorzugt ca. 1,05 Äquivalenten, Acetylchlorid in Gegenwart eines Überschusses einer Lewis-Säure, bevorzugt ca. 1,2 Äquivalenten Aluminiumchlorid, in einem inerten, trockenen Lösungsmittel, bevorzugt Schwefelkohlenstoff, durchgeführt. Optimale Reaktionstemperatur und -zeit hängen von der Natur der Substituenten $R^6$ und $R^7$ ab. In der Regel wird eine Temperatur nahe -10 °C und eine Zeit von 1 - ca. 6 Std. bevorzugt. Die Acetylierung erfolgt fast ausschließlich in ortho-Position zu den Substituenten $R^6$ bzw. $R^7$. Sind $R^6$ und $R^7$ identisch, so entsteht nur ein Produkt. Sind $R^6$ und $R^7$ unterschiedlich, so entstehen zwei Produkte, die getrennt werden müssen. Reinigung bzw. Trennung der Methylketone XIV können in der Regel durch Hochvakuum-Destillation erfolgen.

Die Alkohole der allgemeinen Formel XV erhält man in praktisch quantitativer Ausbeute, wenn man einen Überschuß, bevorzugt ca. 1,4 Äquivalente, einer kommerziellen etherischen Methylmagnesiumhalogenid-Lösung (z. B. einer kommerziell erhältlichen etherischen Methylmagnesiumjodid-Lösung) so zu einer etherischen Lösung des Methylketons XIV tropft, daß ein Rückfluß erhalten bleibt. Diese Reaktionen können im Prinzip extraktiv aufgearbeitet und die Alkohole XV durch Kristallisation gereinigt werden. Es ist jedoch vorteilhaft, direkt aus den rohen Alkoholen XV Wasser abzuspalten, indem man sie an einem Wasserabscheider in Gegenwart einer katalytischen Menge einer starken Säure, bevorzugt para-Toluolsulfonsäure, in einem Lösungsmittel, das mit Wasser ein niedrigsiedendes Azeotrop bildet, mit diesem aber nur wenig mischbar ist, bevorzugt Benzol oder Toluol unter Rückfluß kocht.

Die rohen Olefine der Formel XVI werden durch katalytische Hydrierung in die Verbindung der Formel XVII überführt. In der Literatur wurde eine große Anzahl von Katalysatoren beschrieben, die für derartige Reaktionen geeignet sind. Aus Gründen der Sicherheit und leichteren Durchführbarkeit wird die Hydrierung vorzugsweise bei Raumtemperatur unter 1 atm Wasserstoff durchgeführt. Dazu hat sich die Verwendung von 1-2 Gew.-% von 10 % Palladium auf Kohle und von n-Hexan als Lösungsmittel bewährt. Die Verbindungen der Formel XVII lassen sich in der Regel durch Vakuumdestillation reinigen, doch kommen selbstverständlich auch andere physikalische Trennverfahren, wie Umkristallisieren oder Chromatographie in

Betracht.

Aufgrund einer Kombination von elektronischen und sterischen Faktoren erfolgt die elektrophile aromatische Bromierung unter milden Bedingungen mit hoher Regioselektivität unter Bildung von Verbindungen der allgemeinen Formel XVIII. Zur Vermeidung von Mehrfachbromierungen soll ein Überschuß Brom vermieden werden. Als inertes Lösungsmittel hat sich Tetrachlorkohlenstoff, als Katalysator eine Spatelspitze Eisenpulver bewährt. Die Reaktion wird zur Erzielung hoher Selektivität bevorzugt bei oder unterhalb -10 °C durchgeführt.

Der Austausch des Bromatoms von XVIII gegen einen (substituierten) Arylrest $R^8$ erfolgt am besten in der Weise, daß XVIII mit einem Äquivalent Magnesiumspänen in THF unter Rückfluß zur entsprechenden Grignardverbindung umgesetzt wird. Diese Grignardlösung wird dann unter Inertgas zu einer Lösung von ca. 1,05 Äquivalenten eines (substituierten) Arylbromids oder Aryljodids $R^8$-Hal und ca. 0,01 Äquivalenten eines Palladium (0)-Katalysators, bevorzugt Tetrakis(triphenylphosphin)-palladium(0), in THF gedrückt und das Reaktionsgemisch unter Rückfluß gekocht. Häufig kann man die Reaktion auch durchführen, indem man die Grignard-Verbindung $R^8$-MgHal herstellt und diese dann zu einer Lösung des Arylbromids XVIII und einer katalytischen Menge $Pd(PPh_3)_4$ in THF gibt. Durchführungsvarianten der Kupplungsreaktion und Alternativen für Palladium wurden vorgeschlagen (vgl. deutsche Offenlegungsschrift 3 819 999). Die Produkte der allgemeinen Formel XIX können in der Regel durch Destillation im Pumpenvakuum gereinigt werden.

Die Umsetzung der Kohlenwasserstoffe der allgemeinen Formel XIX zu den Chinonen der allgemeinen Formel XX kann mit einer Reihe von Oxidationsmitteln erzielt werden. Die Ausbeute der Reaktion steigt in der Regel deutlich mit zunehmender Substitution der Ausgangsverbindung.

Bevorzugt ist die Zugabe eines großen Überschusses eines 5:1 (Vol/Vol)-Gemisches aus Trifluoressigsäure und 70 %igem wäßrigem Wasserstoffperoxid bei ca. -20 °C zu dem Kohlenwasserstoff XIX. Bei dieser Temperatur beobachtet man keine Wärmetönung oder Reaktion. Entfernt man dann das Kältebad und läßt unter Erwärmung auf Raumtemperatur rühren, so beobachtet man im Temperaturbereich von ca. +10 °C bis +20 °C ein plötzliches, außerst exothermes Anspringen der Reaktion. Intensive Kühlung des Reaktionskolbens und des Rückflußkühlers ist dann nötig, um die Reaktion unter Kontrolle zu halten. Aus dem rohen Reaktionsprodukt gewinnt man die reinen, intensiv gelben Chinone XX durch Säulenchromatographie oder einfacher durch Umkristallisieren. Die Chinone der allgemeinen Formel XX können mit verschiedenen Reagenzien zu den Hydrochinonen der allgemeinen Formel III/4 reduziert werden. Eine Übersicht geben H. Ulrich, R. Richter in "Methoden der Organischen Chemie (Houben-Weyl)", Bd. VII/3a "Chinone Teil 1", Georg Thieme Verlag, Stuttgart (1977), Seite 648-653: "Umwandlung von p-Chinonen durch Reduktion". Es ist vorteilhaft, ca. 5 Mol-Äquivalente Natriumborhydrid unter Inertgas zu einer Lösung der Chinone XX in Ethanol zu geben. Den Ablauf der Reaktion erkennt man an der Entfärbung der ursprünglich gelben Lösung. Dann wird das Ethanol im Vakuum entfernt und der Rückstand unter intensiver Kühlung mit entgaster Salzsäure zersetzt. Die wäßrige Phase wird sofort mit entgastem Diethylether extrahiert und der Ether im Vakuum entfernt. Die Hydrochinone der allgemeinen Formel III/4 verbleiben als farblose Pulver, die mit z. B. n-Pentan gewaschen und unter Inertgas abgesaugt werden. Diese Hydrochinone erleiden, insbesondere in Lösung, sehr leicht Oxidation, wobei sie sich in die gelben Chinone XX zurückverwandeln. Bei der Herstellung von III/4 sollten darum alle Lösungsmittel sauerstofffrei sein. Die Hydrochinone III/4 werden am besten direkt, ebenfalls unter sauerstofffreien Bedingungen, zu den Monoacetaten III/5 oder III/6 umgesetzt, die kaum oxidationsempfindlich sind.

Zur regioselektiven Herstellung der Monoacetate der Formeln III/5 und (oder) III/6 setzt man z.B. bei 0 °C die Hydrochinone der Formel III/4 mit 1,1-1,5 Äquivalenten Acetanhydrid in Anwesenheit einer Base, bevorzugt in wasserfreiem Pyridin als Lösungsmittel um. Die Reaktionszeit beträgt 1-3 Tage in Abhängigkeit von der Natur der Substituenten $R^6$, $R^7$, $R^8$. Neben wenig Ausgangsmaterial III/4 und Diacetat XXI erhält man die beiden Monoacetate III/5 und III/6 mit einer Selektivität, die in Abhängigkeit von der Natur der Substituenten $R^6$, $R^7$, $R^8$, bei 4:1 bis ca. 20:1 liegt.

Die beiden Monoacetate können säulenchromatographisch auf Kieselgel voneinander, sowie vom Chinon XX und vom Diacetat XXI getrennt werden. Im Laufmittel Cyclohexan/Ethylacetat 9:1 hat das sterisch stärker gehinderte (in geringerem Umfang gebildete) Monoacetat einen etwas größeren $R_f$-Wert als das sterisch weniger gehinderte (in größerem Umfang gebildete) Monoacetat (Hauptprodukt). Das Diacetat XXI ist deutlich polarer.

Mono- und Diacetate der allgemeinen Formeln III/5, III/6 und XXI sind Feststoffe, die bei Bedarf durch Umkristallisieren weiter gereinigt werden können.

Zur gezielten Herstellung der Diacetate der allgemeinen Formel XXI setzt man die Hydrochinone der Formel III/4 mit einem Überschuß, bevorzugt 3-4 Äquivalenten Acetanhydrid, in Anwesenheit einer Base, bevorzugt in wasserfreiem Pyridin als Lösungsmittel, um. Die Reaktionstemperatur liegt in Abhängigkeit von

der Natur der Substituenten $R^6$, $R^7$, $R^8$ bei ca. 0-40 °C, die Reaktionszeit bei ca. 1 Stunde bis mehreren Tagen.

Sowohl bei der Mono- als auch der Diacetylierung können anstatt Acetanhydrid auch andere Acetylierungsmittel eingesetzt werden, z.B. Acetylchlorid, gemischte Anhydride der Essigsäure, Essigsäureimidazolid etc..

Gelegentlich werden die Ausbeuten bei der Mono- oder Diacetylierung deutlich gesteigert, wenn man als Katalysator 4-Dimethylaminopyridin (DMAP, bevorzugt 5-10 Mol-%), Triethylamin als Base und Acetanhydrid als Acetylierungsmittel einsetzt [G. Höfle, W. Steglich, H. Vorbrüggen, Angew. Chem. Int. Ed. 17, 569 (1978)]. Zur chemoselektiven (regioselektiven) Verseifung der Diacetate der allgemeinen Formel XXI sind im Prinzip alle milden Methoden der Esterspaltung verwendbar. Diese Methoden sind Stand der Technik. Entscheidend ist, daß nur wenig mehr als 1 Äquivalent der Base eingesetzt wird (um die Verseifung beider Acetylgruppen weitgehend zu vermeiden) und daß die Reaktionstemperatur so niedrig gehalten wird, daß die Verseifung mehrere Tage erfordert (maximale Selektivität).

Zweckmäßig ist die Verwendung von 1,1 Äquivalenten Lithiumhydroxid in 1,2-Dimethoxyethan/Wasser 3:1 (Vol/Vol). Optimale Reaktionstemperatur und -zeit hängen von der Natur der Substituenten $R^6$, $R^7$, $R^8$ ab. In der Regel führt eine Verseifung bei Raumtemperatur, die meistens 1-5 Tage erfordert, zu Selektivitäten von 2-5 zu 1.

Hohe Cholesterinspiegel, sowie die oxidative Modifizierung der LDL und die daraus resultierende Bildung von "Schaumzellen" mit pathogenen Folgeprozessen wurden mit einer Reihe von Erkrankungen in Verbindung gebracht, die als Folgen der Arteriosclerose betrachtet werden, z.B. koronare Herzkrankheit und Herzinfarkt. Daher ist die Senkung erhöhter Cholesterinspiegel und die Vermeidung der LDL-Oxidation zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG-CoA-Reduktase blockieren die Cholesterin-Biosynthese auf einer frühen Stufe. Sie eignen sich daher zur Vorbeugung und Behandlung von Erkrankungen, die durch einen erhöhten Cholesterinspiegel verursacht werden. Eine Reduktion bzw. Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäurebindender Stoffe wie Anionenaustauscher erzielen. Die erhöhte Gallensäurenausscheidung führt zu einer verstärkten Neusynthese und damit zu einem erhöhten Cholesterinabbau (M.S. Brown, P.T. Kovanen, J.L. Goldstein, Science 212, 628 (1981); M.S. Brown, J.L. Goldstein, Spektrum der Wissenschaft 1985, 96). Die erfindungsgemäßen Verbindungen sind Hemmstoffe der HMG-CoA-Reduktase. Sie eignen sich daher zur Hemmung bzw. Verminderung der Cholesterin-Biosynthese und damit zur Vorbeugung oder Behandlung von Erkrankungen, die durch erhöhte Cholesterinspiegel im Blut verursacht werden, insbesondere koronare Herzkrankheit, Atherosklerose und ähnliche Erkrankungen. Es gibt Hinweise dafür (vergl. Tab. 1-4), daß die erfindungsgemäßen Verbindungen darüber hinaus eine Plasmacholesterin-senkende Wirkung nach einem andersartigen Mechanismus haben, der die nach dem Mechanismus der HMG-CoA-Reduktase-Hemmung erzielte Plasmacholesterin-Senkung verstärkt. Die erfindungsgemäßen Verbindungen und (oder) deren Bausteine der allgemeinen Formel III haben darüber hinaus eine antioxidative, radikalinhibitorische Wirkung. Bei den Bausteinen der allgemeinen Formel III handelt es sich darüber hinaus um Metaboliten der erfindungsgemäßen Verbindungen.

Die Erfindung betrifft daher auch pharmazeutische Präparate auf Basis der Verbindungen der Formel I bzw. der entsprechenden Dihydroxycarbonsäuren der Formel II, deren Salze und Ester, sowie die Verwendung dieser Verbindungen als Arzneimittel, insbesondere zur Behandlung der Hypercholesterinämie.

Die Verbindungen der Formel I bzw. II sowie die entsprechenden Salze oder Ester werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 1 mg bis 2500 mg, vorzugsweise jedoch im Dosisbereich 10 bis 100 mg.

Die erfindungsgemäßen Verbindungen können als Laktone der allgemeinen Formel I, in Form der freien Säuren der Formel II oder in Form pharmazeutisch annehmbarer Salze oder Ester zur Anwendung kommen, und zwar gelöst oder suspendiert in pharmakologisch unbedenklichen organischen Lösungsmitteln wie ein- oder mehrwertigen Alkoholen wie z.B. Ethanol oder Glycerin, in Triacetin, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder anderen pharmazeutisch annehmbaren Zusatzstoffen wie Stärke, Cyclodextrin oder Polysacchariden. Ferner können die erfindungsgemäßen Verbindungen kombiniert werden mit Zusatzstoffen, die Gallensäuren binden, insbesondere nicht toxische, basische Anionenaustauscherharze, die Gallensäuren in einer nichtresorbierbaren Form im Gastrointestinaltrakt binden. Die Salze der Dihydroxycarbonsäuren können auch als wäßrige Lösung verabreicht werden.

Die Hemmung der Cholesterinbiosynthese bzw. die Plasmacholesterinsenkung durch die erfindungsgemäßen Verbindungen der Formeln I und II wurden an verschiedenen in vitro- und in vivo-Testsystemen bestimmt.

**1) Inhibierung der HMG-CoA-Reduktase-Aktivität an solubilisierten Enzympräparationen aus Ratten-Lebermikrosomen**

Die HMG-CoA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden. Als Substrat diente (S,R) $^{14}C$-HMG-CoA, die Konzentration von NADPH wurde während der Inkubation durch ein regenerierendes System aufrechterhalten. Die Abtrennung von $^{14}C$-Mevalonat von Substrat und anderen Produkten (z.B. $^{14}C$-HMG) erfolgte über Säulenelution, wobei das Elutionsprofil jeder Einzelprobe ermittelt wurde. Auf die ständige Mitführung von $^{3}H$-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen zusammen behandelt. Als Präparat wurden bei diesem Test stets die Natriumsalze der Dihydroxycarbonsäuren der allgemeinen Formel II eingesetzt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt. Nach der oben beschriebenen Methode wurden von den Natriumsalzen der erfindungsgemäßen Verbindungen der allgemeinen Formel II z.B. folgende Hemmwerte auf die HMG-CoA-Reduktase ermittelt ($IC_{50}$-(mol/l); molare Konzentration der Verbindung pro Liter, die für eine 50 %ige Hemung erforderlich ist).

Tabelle 1

| | $IC_{50}$ (Mol/l) |
|---|---|
| Standard Mevinolin-Natriumsalz | $8 \cdot 10^{-9}$ |
| Verbindung C (deutsche Offenlegungsschrift 3 819 999 dort Beispiel 8a) | $2{,}3 \cdot 10^{-9}$ |
| Beispiel | $IC_{50}$ (Mol/l) |
| 1 | $6 \cdot 10^{-9}$ |
| 2 | $2 \cdot 10^{-9}$ |
| 3 | $4 \cdot 10^{-9}$ |
| 4 | $8 \cdot 10^{-9}$ |
| 5 | $8 \cdot 10^{-9}$ |
| 6 | $3 \cdot 10^{-8}$ |
| 7 | $1 \cdot 10^{-8}$ |
| 8 | $2 \cdot 10^{-8}$ |
| 9 | $4 \cdot 10^{-9}$ |

**2) Hemmung der Cholesterinbiosynthese in Zellkulturen (HEP G2-Zellen)**

Bestimmung der Inhibition des Einbaus von $^{14}C$-Natriumacetat in Cholesterin

Monolayers von HEP G2-Zellen in lipoproteinfreiem Medium wurden 1 Stunde mit verschiedenen Konzentrationen der Natriumsalze der Dihydroxycarbonsäuren der Formel II präinkubiert. Nach Zugabe von $^{14}C$-markiertem Natriumacetat wurde die Inkubation 3 Stunden fortgesetzt. Tritium-markiertes Cholesterin wurde als interner Standard zugefügt und ein Aliquot der Zellen alkalisch verseift. Die Lipide wurden mit Chloroform/Methanol 2:1 extrahiert. Nach Zugabe von Carrier-Cholesterin wurde das Lipidgemisch präparativ auf DC-Platten mit Chloroform/Aceton 9:1 getrennt. Die Cholesterin-Zone wurde durch Anfärben mit Joddampf sichtbar gemacht, außerdem mit einem DC-Radioscanner detektiert und dann abgeschabt. Die gebildete Menge $^{14}C$-Cholesterin wurde szintigraphisch bestimmt und auf mg Zell-Protein bezogen. Dieselbe Prozedur wurde mit Zellen der gleichen Kultur ohne Präinkubation mit einer Testverbindung durchgeführt (sogenannte "Lösungsmittelkontrolle"). Die Wirkstärke der Testverbindungen wurde bestimmt durch Vergleich des biosynthetisierten $^{14}C$-Cholesterins in Testläufen und bei "Lösungsmittelkontrolle". Externer

Standard war Mevinolin-Natriumsalz. Die $IC_{50}$- und $IC_{70}$-Werte ($IC_{50}$ bzw $IC_{70}$ ist die molare (Mol/Liter) Konzentration der Verbindung, die für eine 50 bzw. 70 %ige Hemmung erforderlich ist) variierten etwas für unterschiedliche Versuchsansätze. Die Mittelwerte für Mevinolin-Natriumsalz waren $IC_{50} = 5 \cdot 10^{-8}$ M, $IC_{70} = 1{,}5 \cdot 10^{-7}$ M.

Die gemessen IC's für Testverbindungen (Natriumsalze der Dihydroxycarbonsäuren der Formel II) (Tabelle 2) wurden um die Abweichung von Mevinolin-Natrium von seinem Durchschnittswert korrigiert. Mevinolin-Natrium wurde eine relative Wirkstärke von 100 zugewiesen.

Tabelle 2

| | $IC_{50}$ (M) | $IC_{70}$ (M) | relative Wirkstärke in % |
|---|---|---|---|
| Standard Mevinolin-Natriumsalz | $5{,}0 \cdot 10^{-8}$ | $1{,}5 \cdot 10^{-7}$ | 100 |
| Verbindung C | $2{,}7 \cdot 10^{-8}$ | $7 \cdot 10^{-8}$ | 185 (214) |
| Beispiel | | | |
| 1 | $4{,}3 \cdot 10^{-9}$ | $\sim 8 \cdot 10^{-10}$ | 1163 (18750) |
| 2 | $2{,}2 \cdot 10^{-9}$ | | 2273 |
| 3 | $3{,}8 \cdot 10^{-9}$ | | 1316 |
| 4 | $8{,}0 \cdot 10^{-9}$ | | 625 |
| 5 | $9{,}2 \cdot 10^{-9}$ | | 543 |
| 6 | $1{,}7 \cdot 10^{-6}$ | | 3 |
| 7 | $9{,}2 \cdot 10^{-7}$ | | 5 |
| 8 | $4{,}0 \cdot 10^{-9}$ | | 1250 |
| 9 | $7{,}5 \cdot 10^{-9}$ | $1{,}2 \cdot 10^{-8}$ | 667 (1250) |

**3) Wirkung auf Serumlipoproteine und andere Stoffwechselparameter männlicher Ratten im subchronischen Versuch**

Methode:

Gruppen männlicher Ratten des Stammes HOE: WISKf (SPF 71) mit einem Ausgangsgewicht über 180 g, erhielten täglich morgens die Prüfpräparate (Natriumsalze der Dihydroxycarbonsäuren der allgemeinen Formel II) in Polyäthylenglykol 400 per Schlundsonde; die jeweilige Kontrollgruppe erhielt nur das Vehikel. Die letzte (7.) Applikation erfolgte 24 Stunden vor Blutentnahme und Tötung. Zu Futter und Wasser bestand freier Zugang während des Versuches. 24 Stunden vor der Blutentnahme, die retroorbital unter leichter Äthernarkose vor und nach der Behandlungsperiode (also am 1. und 8. Tag) erfolgte, wurde das Futter entzogen. Im Serum jedes einzelnen Tieres wurde Totalcholesterin bestimmt [CHODPAP high performance Methode von Boehringer Mannheim], aus dem Serumpool aller Tiere einer Gruppe wurde, als Maß für die Triglyceride, Gesamtglycerin ebenfalls analytisch bestimmt [GPO-PAP high performance Methode, Boehringer Mannheim].

Unmittelbar nach der Blutentnahme wurden die Tiere durch Distorsion der Wirbelsäule getötet, und das relative Lebergewicht, die Körpergewichtsentwicklung und der Futterverbrauch bestimmt.

Zur Analyse der Serum Lipoproteine wurde das Serum aller Ratten einer Gruppe gepoolt. Die Serum-Lipoproteine wurden mit der präparativen Ultrazentrifuge getrennt.

Folgende Bedingungen wurden für die Trennung der Fraktionen VLDL, LDL und HDL verwendet:

| | | | |
|---|---|---|---|
| 1. | VLDL | Dichte | < 1,006 |
| 2. | LDL | Dichte | 1,006 bis 1,04 |
| 3. | HDL | Dichte | 1,04 bis 1,21 |
| 4. | Subnatant der HDL (VHDL) | Dichte | > 1,21 |

Die Bestimmung des Proteins erfolgte nach der Methode von Lowry et al. LOWRY, O.H., ROSEBOROUGH, N.J., FARR, A.L., und RANDELL, R. J.: J. Biol. Chem. 193, 265 (1951)]

Tabelle 3:

| Testsubstanz Beispiel | Dosis mg/kg | % Veränderung gegenüber Kontrolle: Totalcholesterin VLDL | LDL | HDL | Protein VLDL | LDL | Glyz. VLDL |
|---|---|---|---|---|---|---|---|
| 7 | 100 | -23 | -52 | + 9 | -15 | - 8 | - 3 |
| 6 | 100 | +13 | -16 | - 3 | - 2 | + 3 | - 8 |
| 6 | 30 | +12 | -26 | +13 | - 4 | -24 | + 8 |
| Standard Clofibrat | 100 | -40 | -19 | -29 | -16 | - 9 | + 4 |
| Standard Probucol | 30 | + 4 | -24 | -11 | - 6 | -23 | +24 |
| keine (Kontrolle) | - | - | - | - | - | - | - |

Fortsetzung Tabelle 3:

| Testsubstanz Beispiel | % Veränderungen des Mittelwertes, bez. auf Kontrolle: Lebergew. | Futterverbrauch | bez. auf Ausgangsw.: Körpergewicht | Cholesterin HDL/LDL* |
|---|---|---|---|---|
| 7 | - 1 | ±0 | +5 | 2,25 |
| 6 | + 1 | -1 | +5 | 1,15 |
| 6 | + 3 | -1 | +5 | 1,27 |
| Standard Clofibrat | +15 | +1 | +8 | 0,88 |
| Standard Probucol | + 5 | ±0 | +5 | 0,97 |
| keine (Kontrolle) | - | - | +4 | 1,00* |

*: Kontrolle normiert auf 1,00

**4) Hypocholesterolämische Aktivität bei Kaninchen nach p.o.-Administration**

Normolipämische, männliche, weiße Neuseeland-Kaninchen, Körpergewicht 3-3,5 kg, wurden in Gruppen von 5 Tieren eingeteilt. Die Gruppen erhielten jeweils eine der Testverbindungen (Natriumsalze der Verbindungen der allgemeinen Formel II), suspendiert in 1 % wäßriger Methyl-Cellulose (®Tylose MH 300), 5,10 oder 20 mg/kg/Tag, täglich morgens per Schlundsonde. Die Tiere der Kontrollgruppen erhielten nur Tylose MH 300. Alle 3-4 Tage wurden 20 Stunden nach der oralen Administration allen Tieren Proben venösen Blutes abgenommen. In diesen wurde mit der Test-Kombination von Boehringer-Mannheim (CHOD-PAP-high performance method) enzymatisch das Serumtotalcholesterin bestimmt. Die Serumcholesterinspiegel der behandelten Tiere wurden mit denen der Kontrollgruppen verglichen. An die 20-tägige "Behandlungsphase" schloß sich eine "Auslaßphase" an, in der die Veränderung der Serumcholesterinspiegel weiter verfolgt wurde.

Vor, dreimal während der Behandlungsphase und in der Auslaßphase wurden auch die Safety-Parameter (SGOT, SGPT, aP, Bilirubin und Kreatinin im Serum) der Tiere in der Kontrollgruppe und in den behandelten Gruppen bestimmt. Sie zeigten keine signifikanten Veränderungen.

Vor, sechsmal während der Behandlungsphase und in der Auslaßphase wurde auch das Körpergewicht der Tiere in der Kontrollgruppe und in den behandelten Gruppen bestimmt. Es erfolgte keine signifikante Veränderung gegenüber dem Ausgangswert (± 1 %).

**Tabelle 4:**

| Testverbindung | Dosis mg/kg Tag | Serumtotalcholesterin % Veränderung gegenüber Kontrollgruppe: Zahl der Applikation 3 | 10 | 14 | 17 | 20 | Auslaßphase 3 | 6 | 11 | 22 |
|---|---|---|---|---|---|---|---|---|---|---|
| Bsp. 1 | 5 mg (konstant) | -44 | -46 | -43 | -42 | -48 | -22 | -17 | - 6 | - |
| Bsp. 9 | 5 mg (konstant) | - 8 | -12 | -15 | - | - | - 4 | - 4 | + 6 | - |
| Verbindung C | 10 mg (bis 10. Tag) | + 7 | -32 | | | | | | | |
| | 20 mg (ab 11. Tag) | | | -31 | -37 | -36 | -13 | -23 | -16 | + 18 |
| Mevinolin | 10 mg (bis 10. Tag) | -12 | -30 | | | | | | | |
| | 20 mg (ab 11. Tag | | | -27 | -24 | -26 | +14 | - 1 | + 2 | 0 |

Aus Tabelle 4 ist ersichtlich, daß die Verbindung aus Beispiel 1 trotz niedrigerer Dosierung eine wesentlich stärkere Plasmacholesterin-Senkung bewirkt, als Mevinolin oder die Verbindung C. Weiterhin ist ersichtlich, daß die Wirkung der Verbindung aus Beispiel 1 sehr früh einsetzt. Bereits bei der ersten Messung nach 3 Behandlungstagen war die Maximalwirkung praktisch erreicht.

### 5) Hemmung der mikrosomalen Lipidperoxidation (in vitro)

Die Hemmung der Lipidperoxidation wurde unter den von H. Wefers, H. Sies, Eur. J. Biochem. 174, 353-357 (1988) beschriebenen Versuchsbedingungen gemessen. Die Mikrosomen wurden gemäß der dort zitierten Literatur gewonnen. $IC_{50}$ bedeutet die Konzentration der Testverbindung in Mol pro Liter, die eine 50 %ige Hemmung der Lipidperoxidation bewirkt.

Tabelle 5

| Verfahrensbsp./Beispiel | | $IC_{50}$ | Hemmung bei $10^{-5}$ Mol/l |
|---|---|---|---|
| 4 | | $4{,}4 \cdot 10^{-6}$ | 90 % |
| 5 | | - | 11 % |
| 6 | | $3{,}0 \cdot 10^{-6}$ | 95 % |
| 7 | | $4{,}0 \cdot 10^{-6}$ | 92 % |
| 8 | | - | 1 % |
| 12 | | $1{,}5 \cdot 10^{-6}$ | 99 % |
| 22 | | $1{,}6 \cdot 10^{-6}$ | 99 % |
| 23 | | $2{,}8 \cdot 10^{-6}$ | 98 % |
| 25 | | $4{,}8 \cdot 10^{-6}$ | 83 % |
| | 1 | - | <10 % |
| | 2 | - | <10 % |
| | 5 | - | 0 % |
| | 6 | $2{,}7 \cdot 10^{-6}$ | 98 % |
| | 7 | - | <10 % |
| | 8 | - | 27 % |
| | 9 | - | <10 % |

### 6) Hemmung der $Cu^{2+}$-katalysierten LDL-Oxidation in vitro

LDL wurde aus Schweineplasma, welches EDTA (1 mg/ml) enthielt, durch Ultrazentrifugation in Salzlösungen von NaCl/NaBr zwischen den Dichten 1.019 und 1.063 g/ml isoliert (vergl. R.J. Havel et al., J. Clin. Invest. 43, 1345 (1955)). LDL wurde anschließend gegen mit Phosphat gepufferte Salzlösung (160 mM NaCl, 10 mM $NaH_2PO_4$), pH 7,4 dialysiert und unter Stickstoff bei 4 °C aufbewahrt. Vor dem Oxidationsprozeß wurden die LDL-Fraktionen mit Phosphat-gepufferter Salzlösung auf eine Protein-Endkonzentration von 0,1 mg/ml verdünnt und 2,5 mL aliquoter Teile wurden mit den Testverbindungen (25 µL ethanolischer Lösung) über 1 Stunde bei 37 °C unter Stickstoff vorinkubiert (vergl. Mc. Lean et al., Biochemistry 1989, 28, 321). Für die $Cu^{2+}$-katalysierte Oxidation von LDL wurden zu jeder Probe 12,5 µL einer 1 mM $CuSO_4$-Lösung gegeben, wobei eine 5 µM $Cu^{2+}$-Konzentration bewirkt wurde. Die Inkubation wurde über 2 Stunden bei 37 °C und Luftatmosphäre durchgeführt. Die Fluoreszenzintensität wurde bei 430 nm (Anregungswellenlänge 365 nm) gemessen (vergl. Steinbrecher, U. P., J. Biol. Chem. 1987, 262, 3603). Der $IC_{50}$-Wert (Konzentration der Testverbindung in Mol pro Liter, die eine 50 %ige Hemmung der LDL-Oxidation bewirkt) wurde aus der Abnahme der relativen Fluoreszenzintensität ($LDL_{Ox} = 100$) ermittelt. Die Werte sind in Tabelle 6 zusammengestellt. Außerdem ist die Hemmung mit $10^{-5}$ M Konzentration der Testverbindung in % angegeben.

Tabelle 6

| Verfahrensbeispiel | Beispiel | $IC_{50}$ µMol/l | Hemmung bei $10^{-5}$ M |
|---|---|---|---|
| 6 | | 1,0 | |
| 25 | | 0,15 | |
| | 1 | > 10 | 41 % |
| | 8 | 1,1 | |
| | 9 | 6,0 | |
| | 10 | > 10 | 23 % |
| Standard Probucol | | 0,50 | |

In den folgenden Verfahrensbeispielen wird die Synthese von Vorprodukten beschrieben, die zur Herstellung der erfindungsgemäßen Verbindungen der allgemeinen Formeln I und II benötigt werden. In den Beispielen wird die Herstellung von erfindungsgemäßen Verbindungen der allgemeinen Formeln I und II, deren Estern und Salzen beschrieben. Die Verfahrensbeispiele und die Beisipele haben keinen limitierenden Charakter für den Umfang der Erfindung.

## Allgemeine Arbeitstechnik

Reaktionen wurden in Glasapparaturen unter Stickstoff-Inertgasatmosphäre durchgeführt. Wenn nicht anders angegeben, wurden technische Lösungsmittel ohne weitere Reinigung oder Trocknung für Reaktionen und Chromatographie verwendet. Reagenzien hatten eine Reinheit von mindestens 97 % (meistens >99 %). Trocknungen von Reaktionsextrakten wurden, wenn nicht anders angegeben, mit Magnesiumsulfat durchgeführt. Dünnschichtchromatographische Analysen (DC) wurden auf Glas-DC-Fertigplatten Kieselgel 60 mit Fluoreszenzindikator $F_{254}$ (Firma Merck) durchgeführt. Die Detektion der Produktflecken erfolgte mittels UV, sowie durch Verwendung von Sprühreagenzien zur Anfärbung.

Säulenchromatographische Trennungen wurden in Glassäulen und unter Bedingungen durchgeführt, wie sie für die Flash-Chromatographie beschrieben wurden [W.C. Still et al., J. Org. Chem. 43, 2923 (1978)-]. Verwendet wurde Kieselgel der Korngröße 35-70 µm, Porendurchmesser 60Å bzw. 70-200 µm, Porendurchmesser 60Å der Firma Amicon.

$^1$H-NMR Spektren wurden mit einem ®Bruker WP60 oder WM270 Spektrometer aufgenommen. Wenn nicht anders angegeben, diente $CDCl_3$ als Lösungsmittel. Chemische Verschiebungen sind in ppm, bezogen auf Tetramethylsilan als interner Standard, angegeben.

Massenspektren wurden mit einem ®Kratos MS9 (FAB) oder MS80 (Cl)-Spektrometer aufgenommen.

Schmelzpunkte wurden mit einem Büchi Kapillar-Schmelzpunktapparat (nach Dr. Tottoli) ermittelt und sind unkorrigiert.

## Verfahrensbeispiel 1

### 2-Brom-6-isopropyl-phenol (Schema 1, Formel VIII)

198,1 ml (3,85 Mol) Brom wurden bei -5 bis 0°C zur Lösung von 470 g Natriumhydroxid in 2 l Wasser getropft. Das Gemisch wurde noch 10 Min. bei dieser Temperatur gerührt. Die entstandene Natriumhypobromid-Lösung wurde bei -5 bis 0°C zur Lösung von 464 g einer 40 %igen wäßrigen Dimethylamin-Lösung (4,11 Mol) in 50 ml Wasser getropft.

Das Gemisch wurde noch 30 Min. gerührt, dann wurde die organische Phase abgetrennt und die wäßrige Phase mit 2 mal 750 ml Methylenchlorid extrahiert. Die vereinigten organischen Phasen wurden kurz über Magnesiumsulfat getrocknet und filtriert. Das Filtrat wurde bei -10°C zur Lösung von 500 g (3,67 Mol) ortho-Isopropylphenol in 900 ml Methylenchlorid getropft. Nach Zugabe von ca. 2/3 des Filtrats bildete sich ein Feststoff und das Reaktionsgemisch wurde viskos und schwer rührbar. 500 ml Methylenchlorid wurden bei -10°C zugegeben und das Gemisch wurde eine weitere Stunde gerührt. Der Feststoff wurde abgesaugt, mit wenig kaltem Methylenchlorid gewaschen, in 1,5 l 2N Schwefelsäure suspendiert und bei Raumtemperatur gerührt, bis sich sämtlicher Feststoff in ein Öl umgewandelt hatte. Die organische Phase wurde abgetrennt, die wäßrige Phase mit Methylenchlorid extrahiert. Die vereinigten organischen Phasen wurden mit Kochsalzlösung gewaschen, getrocknet und das Lösungsmittel wurde im Vakuum entfernt. Der Rückstand wurde im Wasserstrahlvakuum durch eine 30 cm Vigreux-Kolonne destilliert.

391,7 g (1,82 Mol) farbloses Öl, Sdp. 122-124 °C/2793 Pa (21 Torr);
Ausbeute 49,6 %

NMR (60 MHz): δ = 1,20 (d, 6H, $CH_3$), 3,23 (sept., 1H, CH), 5,42 (s, 1H, OH), 6,4-7,2 (m, 3H, arom.-H).

**Verfahrensbeispiel 2**

**2-(p-Fluorphenyl)-6-isopropyl-phenol (Schema 1, Formel IX)**

**a)** Zu 18,7 g (0,77 Mol) Magnesiumspänen wurden drei Jodkristalle gegeben und die Zugabestelle mit einem Heißluftgerät (®Fön) erhitzt, bis Joddämpfe im Kolben sichtbar waren. Es wurde auf Raumtemperatur abgekühlt und 20 ml absol. THF zugegeben. 131,3 g (0,75 Mol) p-Bromfluorbenzol wurden in einen 500 ml Tropftrichter gefüllt und davon ca. 2 ml in den Reaktionskolben gegeben. Die braune Farbe des Reaktionsgemischs verschwand rasch und es erfolgte starke Wärmeentwicklung zum Rückfluß. Sofort wurden weitere 50 ml absol. THF zum Reaktionsgemisch gegeben und das p-Bromfluorbenzol im Tropftrichter mit 200 ml THF verdünnt. Diese Lösung wurde dann so zugetropft, daß ein gelinder Rückfluß aufrecht erhalten wurde. Anschließend wurde das Reaktionsgemisch noch 1 Stunde unter Rückfluß gekocht und dann auf 50 °C abgekühlt.

**b)** In einem zweiten Kolben wurde aus der Lösung von 52,0 g (0,24 Mol) 2-Brom-6-isopropyl-phenol in 150 ml absol. THF mittels 20 minütigem Durchleiten von Stickstoff der gelöste Sauerstoff vertrieben. 1,7 g (1,5 mMol) Tetrakis(triphenylphosphin)-palladium(O) wurden unter Minimierung des Sauerstoffkontakts zugegeben.

Dann wurde die Grignard-Lösung aus Stufe a) mittels einer Doppelkanüle ("®Flex-Needle", Firma Aldrich) unter Stickstoffdruck in diese Lösung transferiert, wobei eine Wärmeentwicklung auftrat. Die Geschwindigkeit des Transfers wurde so gewählt, daß ein gelinder Rückfluß aufrecht erhalten wurde. Anschließend wurde noch 6 Stunden zum Rückfluß erhitzt. Das Reaktionsgemisch wurde abgekühlt und auf 500 g Eis/100 ml konz. Salzsäure gegossen. Die organische Phase wurde abgetrennt und die wäßrige Phase wurde mit 3 x 100 ml Ether extrahiert. Die vereinigten organischen Phasen wurden mit 100 ml gesättigter Kochsalzlösung gewaschen, getrocknet und die Lösungsmittel abgezogen. Der Rückstand wurde im Pumpenvakuum durch eine 30 cm Vigreux-Kolonne destilliert. Nach einem Vorlauf (30-65 °C/26,6 Pa (0,2 Torr)) destillierte das reine Produkt (Sdp. 107-109 °C/66,5 Pa (0,5 Torr)) als farbloses Öl, das in der Vorlage und z.T. auch schon in der Destillationsbrücke kristallisierte (Schmp. 44-46 °C). Um ein Verstopfen der Brücke zu vermeiden, wurde diese auf ca. 50 °C temperiert. Ausbeute 37,8 g Titelverbindung (164 mMol); 68,4 % d. Th. GC-Analyse (30 m fused silica-Säule DB-5 "Polydiphenyldimethylsiloxan", Schichtdicke 0,25 µm, Innendurchmesser 0,32 mm, 180 °C, Inj. 240 °C, 1 bar $H_2$): $t_{ret}$: 4,46 Min; Reinheit > 99,9 %.

NMR (270 MHz): δ = 1,28 (d, 6H, $CH_3$); 3,32 (sept., 1H, CH); 5,08 (s, 1H, OH); 6,9-7,5 (m, 7H, arom.-H).

MS (DCI, Isobutan): m/e = 231 ($M+H^+$), 230 ($M^+$), 215 ($M^+$-$CH_3$)

**Verfahrensbeispiel 3**

**2-(p-Fluorphenyl)-4-thiocyanato-6-isopropyl-phenol (Schema 1, Formel X)**

Die Suspension von 70,9 g (838 mMol, 5,0 Äquivalenten) Natriumthiocyanat in 200 ml Methanol wurde 20 Min. bei Raumtemperatur gerührt. 40,0 g (173,8 mMol, 1,0 Äquiv.) 2-(p-Fluorphenyl)-6-isopropyl-phenol wurden zugegeben und das Gemisch wurde 20 Minuten gerührt. 14,32 ml (277,8 mMol, 1,6 Äquiv.) Brom wurden in 50 ml Methanol gelöst (exotherm) und diese Lösung wurde bei 15-20 °C wahrend 20 Minuten zu obiger Reaktionslösung getropft. Das Reaktionsgemisch färbte sich gelb und das Phenol löste sich vollständig. Das Reaktionsgemisch wurde 30 Min. gerührt. DC (Toluol/Cyclohexan 1:1) zeigte vollständigen Umsatz des Ausgangsmaterials ($R_f = 0{,}54$) an. Neben der Titelverbindung ($R_f = 0{,}32$) entstand als Verunreinigung nur eine geringe Menge der entsprechenden para-Brom-Verbindung, die mit dem Ausgangsmaterial cochromatographiert ($R_f = 0{,}54$), aber aufgrund anderer Anfärbung unterschieden werden kann. Das Reaktionsgemisch wurde auf 400 g Eis/400 ml 2 N Salzsäure gegossen und mit 4 x 200 ml Toluol extrahiert. Die Extrakte wurden mit wäßriger Natriumsulfit-Lösung gewaschen, filtriert, mit gesättigter Kochsalzlösung gewaschen, getrocknet und im Vakuum eingeengt.

Der zurückbleibende gelbe Feststoff wurde in 500 ml heißem Cyclohexan gelöst und es wurden 5 g Aktivkohle zugesetzt. Danach wurde 5 Minuten unter Rückfluß gekocht und die heiße Suspension im Vakuum filtriert. Die abgesaugte Aktivkohle wurde mit 20 ml heißem Cyclohexan nachgewaschen. Das fast

farblose Filtrat kühlte langsam ab und wurde dann noch 12 Std. auf 10 °C gekühlt.

Die farblosen Kristalle (Titelverbindungen) wurden abgesaugt und im Vakuum getrocknet. 47,6 g (165,7 m Mol) Ausbeute entsprech. 95,3 %; Schmp.: 94,5-96 °C.

NMR (60 MHz): δ = 1,26 (d, 6H, $CH_3$), 3,32 (sept., 1H, CH); 5,46 (s, 1H, OH); 7,0-7,6 (m, 6H, arom.-H).

MS (DCI, Isobutan) : m/e = 288 ($M+H^+$), 272 ($M^+$-$CH_3$), 261 ($M^+$-CN)

**Verfahrensbeispiel 4**

**2-(p-Fluorphenyl)-4-mercapto-6--isopropyl-phenol (Schema 1, Formel III/2)**

Zu einer Suspension von 7,5 g (198 mMol) Lithiumaluminiumhydrid in 20 ml absol. THF tropfte man die Lösung von 32,5 g (113 mMol) 2-(p-Fluorphenyl)-4-thiocyanato-6-isopropyl-phenol in 150 ml absol. THF. Das Reaktionsgemisch wurde 90 Min. unter Rückfluß gekocht. DC (CH/EE 9:1; $R_f$ des Thiocyanats: 0,16; $R_f$ des Mercaptans: 0,26) zeigte quantitative Reaktion an. Zum Gemisch wurden unter Trockeneiskühlung vorsichtig 100 ml konz. Salzsäure getropft. Dabei gingen die Aluminiumsalze in Lösung. Das Reaktionsgemisch wurde mehrmals mit Ether extrahiert. Die vereinigten Extrakte wurden mit gesättigter Kochsalzlösung gewaschen, getrocknet und die Lösungsmittel im Vakuum entfernt. Der Rückstand wurde unter Stickstoffdruck mit obigem Laufmittel über eine Kieselgelsäule filtriert. Man erhielt 27,3 g (104 mMol) des reinen Produkts (Titelverbindung) als farbloses Öl (Ausbeute 92,1 %).

NMR (60 MHz): δ = 1,27 (d, 6H, $CH_3$), 3,30, (sept., 1H, CH), 3,40 (s, 1H, SH), 5,06 (s, 1H, OH) 6,93-7,63 (m, 6H, arom.-H).

MS (DCI, Isobutan): m/e = 262 ($M^+$), 247 ($M^+$-$CH_3$)

IR ($CHCl_3$): 3555 (OH), 2560 (schwach, SH), 1510, 1455, 1223, 840 $cm^{-1}$

Das Produkt ist oxidationsempfindlich und muß unter weitgehendem Sauerstoffausschluß gehandhabt werden.

**Verfahrensbeispiel 5**

**4,4-(Isopropylidendithio)-bis-[(2-isopropyl-6-p-fluorphenyl)phenol] (Schema 1, Formel III/3)**

27,3 g (104 mMol) 2-(p-Fluorphenyl)-4-mercapto-6-isopropyl-phenol wurden in 100 ml Benzol gelöst, das zuvor mittels Durchperlen von Stickstoff vom Sauerstoff befreit worden war. 13,5 g (16 ml, 130 mMol) 2,2-Dimethoxypropan, gefolgt von ca. 100 mg (~ 0,5 mMol) p-Toluolsulfonsäure-Monohydrat wurden zugegeben. Das Reaktionsgemisch wurde 30 Min. bei Raumtemperatur, dann 8 Stunden unter Rückfluß gerührt. Es wurde mit wäßriger Natriumacetat-Lösung, dann mit gesättigter Kochsalzlösung gewaschen, getrocknet und im Vakuum eingeengt. Das zurückbleibende Öl (29,0 g) wurde mit Cyclohexan/Toluol 1:1 + 1 o/oo Triethylamin über Kieselgel chromatographiert und ergab 26,0 g Titelverbindung (46,0 mMol, Ausb. 88,5 %) als ein gelbliches Öl, das im Kühlschrank kristallisierte. Es schmolz nahe Raumtemperatur.

NMR (60 MHz): δ = 1,28 (d, 12H, $C(CH_3)_2$); 1,53 (s, 6H, -S-$C(CH_3)_2$-S); 3,32 (sept., 2H, CH); 5,26 (s, 2H, OH); 7,0-7,7 (m, 12H, arom.-H)

MS (FAB, 3-NBA/LiI): m/e = 571 ($M+Li^+$);

303 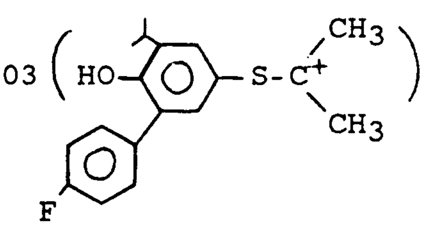 .

**Verfahrensbeispiel 6**

**2-(p-Fluorphenyl)-4-(p-fluorphenylthio)-6-isopropyl-phenol (Schema 1, Formel III/1)**

Eine THF-Lösung (100 ml) von p-Fluorphenylmagnesiumbromid [aus 3,11 g (128 mMol) Magnesium und 22,0 g (13,8 ml, 126 mMol) p-Bromfluorbenzol] wurde wie in Verfahrensbeispiel 2 hergestellt. Die Lösung von 6,04 g (21 mMol) 2-(p-Fluorphenyl)-4-thiocyanato-6-isopropyl-phenol (aus Verfahrensbeispiel 3) in 50 ml THF wurde bei 50 °C zugetropft und noch 2 Stunden bei 40-50 °C gerührt. Das Gemisch wurde abgekühlt und auf 500 ml eiskalte 2N Salzsäure gegossen. Es wurde dreimal mit 200 ml Ether extrahiert. Die vereinigten Extrakte wurden mit Kochsalzlösung gewaschen, getrocknet und das Lösungsmittel im Vakuum entfernt.

Das zurückbleibende Öl (Titelverbindung) (7,5 g, 21 mMol, Ausbeute ~ 100 %) war gemäß DC (Cyclohexan/Ethylacetat 9:1) und $^1$H-NMR rein.

NMR (60 MHz): $\delta$ = 1,25 (d, 6H, $CH_3$); 3,31 (sept., 1H, CH); 5,22 (s, 1H, OH); 6,8-7,8 (m, 10H, arom.-H)

MS (DCI, Isobutan): m/e = 357 ($M+H^+$); 356 ($M^+$)

**Verfahrensbeispiel 7**

**2-(p-Fluorphenyl)-4-(phenylthio)-6-isopropyl-phenol (Schema 1, Formel III/1)**

In Analogie zu Verfahrensbeispiel 6 ergab die Einwirkung von 6 Äquivalenten Phenylmagnesiumbromid auf 2-(p-Fluorphenyl)-4-thiocyanato-6-isopropyl-phenol (aus Verfahrensbeispiel 3) in THF bei 40-50 °C in 95 %iger Ausbeute die Titelverbindung.

NMR (60MHz): $\delta$ = 1,25 (d, 6H, $CH_3$); 3,30 (sept., 1H, CH); 5,20 (s, 1H, OH); 6,9-7,8 (m, 11H, arom.-H)

MS (DCI, Isobutan): m/e = 339 ($M+H^+$); 338 ($M^+$)

**Verfahrensbeispiel 8**

**2-(p-Fluorphenyl)-4-(isopropylthio)-6-isopropyl-phenol (Schema 1, Formel III/1)**

Zu 20 ml Isopropanol wurden 910 mg (39,6 mMol) Natriumstücke gegeben und man rührte, bis das Metall vollständig verschwunden war. Zur erhaltenen Lösung wurde während einer Stunde die Lösung von 2,5 g (8,7 mMol) 2-(p-Fluorphenyl)-4-thiocyanato-6-isopropyl-phenol (aus Verfahrensbeispiel 3) in 50 ml Isopropanol getropft. Das Reaktionsgemisch wurde 30 Min. unter Rückfluß gekocht, dann in 100 ml 2N Schwefelsäure gegossen und mit 3x 100 ml Ether extrahiert. Die vereinigten Extrakte wurden mit gesättigter Kochsalzlösung gewaschen, getrocknet und im Vakuum eingeengt. Der ölige Rückstand (2,42 g) wurde mit Cyclohexan/Ethylacetat 2:1, später 1:1 chromatographiert und lieferte 640 mg (2,1 mMol, Ausbeute 24,1 %) eines zähen, gelblichen Öls (Titelverbindung).

NMR (60MHz): $\delta$ = 0,97 (d, 6H, $SC(CH_3)_2$); 1,20 (d, 6H, $C(CH_3)_2$); 3,00-3,90 (2x sept., 2H, CH); 5,20 (s, 1H, OH); 6,9-7,7 (m, 6H, arom.-H)

MS (DCI, Isobutan): m/e = 305 ($M+H^+$); 304 ($M^+$)

**Verfahrensbeispiel 9**

**2-Brom-6-cyclopropyl-phenol (Schema 1, Formel VIII)**

In Analogie zu Verfahrensbeispiel 1 wurde aus ortho-Cyclopropylphenol Y.S. Shaborov, V.K. Potapov, R.Y. Levina, J. Gen. Chem. USSR 34, 3171 (1964)] die Titelverbindung in 38 % Ausbeute als farbloses Öl erhalten.

NMR (60MHz): $\delta$ = 0,73 (m, 4H, $CH_2$); 1,77 (qui, 1H, CH); 5,42 (s, 1H, OH), 6,4-7,2 (m, 3H, arom.-H)

MS (DCI, Isobutan): m/e = 213/215 ($M+H^+$); 212/214 ($M^+$)

**Verfahrensbeispiel 10**

**2-(p-Fluorphenyl)-6-cyclopropyl-phenol (Schema 1, Formel IX)**

In Analogie zu Verfahrensbeispiel 2 wurde aus 2-Brom-6-cyclopropyl-phenol die Titelverbindung in 47 % Ausbeute als farbloser Feststoff erhalten.

NMR (60 MHz): δ = 0,78 (m, 4H, $CH_2$); 1,85 (qui, 1H, CH); 5,00 (s, 1H, OH); 6,7-7,5 (m, 7H, arom.-H)

MS (DCI, Isobutan): m/e = 229 ($M+H^+$), 228 ($M^+$)

**Verfahrensbeispiel 11**

**2-(p-Fluorphenyl)-4-thiocyanato-6-cyclopropyl-phenol (Schema 1, Formel X)**

In Analogie zu Verfahrensbeispiel 3 wurde aus 2-(p-Fluorphenyl)-6-cyclopropyl-phenol die Titelverbindung in 62 % Ausbeute als blaßgelber Feststoff (Schmp. 82-85 ° C) erhalten.

NMR (60 MHz): δ = 0,79 (m, 4H, $CH_2$); 1,88 (qui, 1H, CH); 5,36 (s, 1H, OH), 6,9-7,6 (m, 6H, arom.-H)

MS (DCI, Isobutan): m/e = 286 ($M+H^+$); 259 ($M^+$-CN)

**Verfahrensbeispiel 12**

**2-(p-Fluorphenyl)-4-(p-fluorphenylthio)-6-cyclopropyl-phenol (Schema 1, Formel III/1)**

In Analogie zu Verfahrensbeispiel 6 wurde aus 2-(p-Fluorphenyl)-4-thiocyanato-6-cyclopropyl-phenol die Titelverbindung als zähes, blaßgelbes Öl erhalten.

NMR (60 MHz): δ = 0,78 (m, 4H, $CH_2$); 1,87 (qui, 1H, CH); 5,12 (s, 1H, OH); 6,7-7,7 (m, 10H, arom.-H)

MS (DCI, Isobutan): m/e = 355 ($M+H^+$); 354 ($M^+$)

**Verfahrensbeispiel 13**

2-(p-Fluor-m-methyl-phenyl)-6-isopropyl-phenol (Schema 1, Formel IX)

In Analogie zu Verfahrensbeispiel 2 wurde aus 2-Brom-6-isopropyl-phenol (aus Verfahrensbeispiel 1) und dem Grignard-Reagenz aus p-Fluor-m-methyl-brombenzol die Titelverbindung in 68 % Ausbeute als farbloser Feststoff erhalten.

NMR (60 MHz): δ = 1,25 (d, 6H, $CH_3$); 2.35 (s, 3H, $CH_3$); 3,30 (sept., 1H, CH), 5,00 (s, br, 1H, OH), 6,7-7,5 (m, 6H, arom.-H)

MS (DCI, Isobutan): m/e = 245 ($M+H^+$), 244 ($M^+$); 229 ($M^+$-$CH_3$)

**Verfahrensbeispiel 14**

**2-(p-Fluor-m-methyl-phenyl)-4-thiocyanato-6-isopropyl-phenol (Schema 1, Formel X)**

In Analogie zu Verfahrensbeispiel 3 wurde aus 2-(p-Fluor-m-methyl-phenyl)-6-isopropyl-phenol die Titelverbindung in 59 % Ausbeute als blaßgelber Feststoff (Schmp. 96-98 ° C) erhalten.

NMR (60 MHz): δ = 1,26 (d, 6H, $CH_3$); 2.35 (s, 3H, $CH_3$); 3,32 (sept., 1H, OH); 5,47 (s, 1H, OH); 7,0-7,6 (m, 5H, arom.-H)

MS (DCI, Isobutan): m/e = 302 ($M+H^+$), 286 ($M^+$-$CH_3$),275 ($M^+$-CN)

**Verfahrensbeispiel 15**

**2-(p-Fluor-m-methyl-phenyl)-4-(p-fluorphenylthio)-6-isopropyl-phenol (Schema 1, Formel III/1)**

In Analogie zu Verfahrensbeispiel 6 wurde aus 2-(p-Fluor-m-methyl-phenyl)-4-thiocyanato-6-isopropylphenol die Titelverbindung als zähes, blaßgelbes Öl erhalten.

NMR (60 MHz): δ = 1,25 (d, 6H, $CH_3$); 2,34 (s, 3H, $CH_3$); 3,31 (sept., 1H, CH); 5,23 (s, 1H, OH);

6,8-7,8 (m, 9H, arom.-H)
MS (DCI, Isobutan): m/e = 371 ($M+H^+$), 370 ($M^+$).

**Verfahrensbeispiel 16**

**1-Acetyl-2,5-diisopropyl-benzol (Schema 3, Formel XIV)**

Zu einer auf -10°C gekühlten Suspension von 200 g (1,5 Mol) Aluminiumtrichlorid in 260 ml Schwefelkohlenstoff tropfte man während 2 Std. die Lösung von 142 ml (157 g, 2,0 Mol) Acetylchlorid in 362 ml (310 g, 1,91 Mol) 1,4-Diisopropylbenzol. Man rührte weitere 1,5 Std. bei -10°C, wobei die Chlorwasserstoff-Entwicklung stark abnahm, setzte dann weitere 100 g (0,75 Mol) Aluminiumtrichlorid in Portionen zu und rührte eine weitere Stunde. Das Reaktionsgemisch wurde vorsichtig auf 1 kg Eis/100 ml 2 N Salzsäure geschüttet (stark exotherm!). Die ölige organische Phase wurde abgetrennt und die Wasserphase 2mal ausgeethert. Die vereinigten organischen Phasen wurden mit verdünnter Natriumcarbonatlösung, dann mit Wasser gewaschen und über Calciumchlorid getrocknet. Die Lösungsmittel wurden abgezogen und der Rückstand wurde im Pumpenvakuum durch eine 30 cm Vigreux-Kolonne destilliert. Nach einem Vorlauf (Sdp. 70-91°C/79,8 Pa (0,6 Torr), farbloses Öl, 10,9 g) erhielt man die Titelverbindung (Sdp. 92-95°C/79,8 Pa (0,6 Torr), farbloses Öl, 344,3 g, 1,69 Mol), Ausbeute 88,4 %. Beim Nachlauf (Sdp. 97-104°C/133 Pa (1,0 Torr), 10.25 g) handelt es sich um ein farbloses Öl, das sofort erstarrt.
Das Produkt ist rein gemäß DC (Cyclohexan/Ethylacetat 5:1, $R_f$ = 0,45).

**Verfahrensbeispiel 17**

**1-(2-Hydroxy-2-propyl)-2,5-diisopropyl-benzol (Schema 3, Formel XV)**

Zu 468 ml (1,4 Mol) einer kommerziellen, 3-molaren Lösung von Methylmagnesiumjodid in Ether tropfte man 197 g (0,97 Mol) 1-Acetyl-2,5-diisopropyl-benzol so zu, daß ein gelinder Rückfluß aufrecht erhalten wurde. Man kochte noch 1 Std. unter Rückfluß, goß dann vorsichtig auf 1,5 l eiskalte, wäßrige Ammoniumchlorid-Lösung, trennte die organische Phase ab und etherte noch zweimal aus. Die vereinigten organischen Phasen wurden mit gesättigter Kochsalzlösung gewaschen, getrocknet und das Lösungsmittel wurde im Vakuum entfernt.

Man erhielt 213,1 g (Ausbeute 100 %) eines zähen Öls (DC: Cyclohexan/Ethylacetat 5:1, $R_f$ = 0,26). Aus diesem Rohprodukt kann man reines 1-(2-Hydroxy-2-propyl)-2,5-diisopropylbenzol durch Kristallisation aus ca. 400 ml Petrolether bei -20°C erhalten. Die Kristallbildung erfordert häufig mehrere Tage.

NMR (60 MHz): $\delta$ = 1,25 (d, 12H, $CH_3$), 1,68 (s, 6H, $CH_3$), 1,74 (s, 1H, OH), 2,86 (sept. 1H, CH), 3,82 (sept., 1H, CH), 6,96-7,42 (m, 3H, arom.-H)
Es ist vorteilhaft, den rohen Alkohol direkt weiter umzusetzen.

**Verfahrensbeispiel 18**

**1,2,5-Triisopropylbenzol (Schema 3, Formel XVII)**

Der rohe Alkohol aus Verfahrensbeispiel 17 (213,1 g, 0,97 Mol) wurde mit 800 ml Toluol und zwei Spatelspitzen para-Toluolsulfonsäure-Hydrat am Wasserabscheider unter Rückfluß gekocht. Innerhalb 1 Stunde wurden 15 ml Wasser abgeschieden. DC (100 % Toluol) zeigte vollständige Umsetzung des Alkohols ($R_f$ = 0,17) zum Olefin ($R_f$ = 0,79) an. Das Toluol wurde im Vakuum bei 20°C Badtemperatur entfernt. Der ölige Rückstand wurde in 160 ml n-Hexan gelöst. Unter Stickstoff wurden 4,7 g 10 % Palladium auf Kohle zugesetzt und das Gemisch bei Raumtemperatur unter 1 atm Wasserstoffdruck geschüttelt. Innerhalb 12 Stunden wurden 21,2 l Wasserstoff aufgenommen. Der Katalysator wurde über Kieselgur (z.B. ®Celite) abgesaugt und mit n-Hexan gewaschen. Das Filtrat wurde im Vakuum bei 20°C Badtemperatur eingeengt. Der ölige Rückstand wurde im Wasserstrahlvakuum durch eine 30 cm Vigreux-Kolonne destilliert. Das Destillat im Siedebereich 70-128°C/12 Torr (178,5 g) wurde in Fraktionen gesammelt. Alle Fraktionen enthielten nach DC-Analyse (100 % Cyclohexan) das Produkt ($R_f$ = 0,65) neben einer polaren Verunreinigung ($R_f$ = 0,05), deren Anteil mit fortschreitender Destillation zunahm. Die Verunreinigung wurde durch Säulenchromatographie (100 % Cyclohexan) entfernt.
Erhalten wurden 166, 2 g (0,81 Mol) der Titelverbindung als farbloses Öl. Ausbeute 83,8 %.

NMR (60 MHz): $\delta$ = 1,18 (d, 18H, $CH_3$); 2,63-3,60 (3xsept., 3H, CH), 6,87-7,30 (m, 3H, arom.-H)
MS (DCI, Isobutan): m/e = 205 ($M+H^+$), 204 ($M^+$), 189 ($M^+$-$CH_3$)

GC (30 m fused silica-Säule DB-5 "Polydiphenyldimethylsiloxan", Schichtdicke 0,25 µm, Innendurchmesser 0,32 mm, 160 °C, Inj. 240 °C, 1 bar Helium):$t_{ret}$ 4,39 Min., Reinheit 97,1 %

**Verfahrensbeispiel 19**

**1-Brom-2,4,5-triisopropylbenzol (Schema 3, Formel XVIII)**

Zur Lösung von 301,3 g (1,47 Mol) 1,2,5-Triisopropylbenzol in 600 ml Tetrachlorkohlenstoff gab man bei -10 °C eine Spatelspitze Eisenpulver und tropfte dann unter weitgehendem Lichtausschluß die Lösung von 76 ml (236,2 g, 1,48 Mol) Brom in 600 ml Tetrachlorkohlenstoff zu. Zunächst erfolgte keine nennenswerte Bromwasserstoff-Entwicklung und die Bromfarbe der Reaktionslösung blieb bestehen. Nach vollständiger Zugabe ließ man auf 0 °C erwärmen, worauf eine kräftige Bromwasserstoff-Entwicklung einsetzte. Man rührte noch weitere 90 Min. bei Raumtemperatur, worauf DC (100 % Cyclohexan) weitgehendes Verschwinden des Ausgangsmaterials ($R_f$ = 0,51) anzeigte (Produkt $R_f$ = 0,57; Nebenprodukt $R_f$ = 0,64).

Die Reaktionslösung wurde zwischen Methylenchlorid und 10 %iger Natriumthiosulfatlösung verteilt. Die organische Phase wurde abgetrennt und die wäßrige Phase noch einmal mit Methylenchlorid extrahiert. Die vereinigten organischen Phasen wurden mit Natriumchloridlösung gewaschen, getrocknet und im Vakuum eingeengt. Der ölige Rückstand wurde im Pumpenvakuum durch eine 20 cm-Vigreux-Kolonne fraktioniert:

1) Vorlauf, 5,3 g farbloses Öl, Sdp. 71-103 °C/133 Pa (1 Torr)
2) Hauptlauf, 265,4 g (0,94 Mol) der Titelverbindung, Sdp. 105-112 °C/ 133 Pa (1 Torr), sehr blaß gelbes Öl, Ausbeute 64,0 %
3) Nachlauf 35,8 g gelbes Öl, Spd. 114-120 °C/133 Pa (1 Torr), Titelverbindung + Nebenprodukt.

GC des Hauptlaufs (Säule DB-5, Bedingungen wie in Verfahrensbeispiel 18):$t_{ret}$ 8,81 Min., Reinheit: 98,4 %

| | |
|---|---|
| NMR (60 MHZ): | δ = 1,24 (d, 18H, $CH_3$); 3,19 (sept., 1H, CH); 7,12 (s, 1H, arom.-H); 7,33 (s, 1H, arom.-H) |
| MS (PCI, Isobutan): | m/e = 285/283 ($M+H^+$), 284/282 ($M^+$), 269/267 ($M^+$-$CH_3$), 243/241 ($M^+$-∧.) |

**Verfahrensbeispiel 20**

**1-(p-Fluorphenyl)-2,4,5-triisopropylbenzol (Schema 3, Formel XIX)**

Zu 22,3 g (0,92 Mol) Magnesiumspänen gab man einige Jodkristalle und erhitzte mit einem Heißluftgerät (®Fön) bis zur Bildung der violetten Joddämpfe. Man gab ca. 50 ml absol. THF zu, gefolgt von ca. 20 ml von insgesamt 251 g (0,89 Mol) 1-Brom-2,4,5-triisopropylbenzol. Sobald die Reaktion angesprungen war (evtl. Erhitzen zum Rückfluß notwendig) gab man 150 ml absol. THF durch den Rückflußkühler zu, verdünnte die restliche Bromverbindung im Tropftrichter mit ca. 300 ml THF und tropfte diese Lösung so zu, daß ein gelinder Rückfluß erhalten blieb. Nach vollständiger Zugabe kochte man noch 30 Min. unter Rückfluß und erhielt eine klare, schwach grünliche Lösung, die auf ca. 40 °C abgekühlt wurde.

In einer zweiten Apparatur vertrieb man aus der Lösung von 162,6 g (0,93 Mol) 4-Bromfluorbenzol in 800 ml absol. THF den Sauerstoff mit durchperlendem Stickstoff (30 Min.). Man gab 10 g (8,6 mMol) Tetrakis(triphenylphosphin)-Palladium(O) zu und rührte die Lösung 10 Min. bei Raumtemperatur. Dann drückte man die obige Grignardlösung mittels einer Doppelkanüle mit Stickstoff in diese Lösung (ca. 15 Min.). Man erhitzte 2 Stunden unter Rückfluß, wobei aus der anfangs klaren Reaktionslösung ein weißer Niederschlag ausfiel. Man ließ abkühlen und goß das Reaktionsgemisch auf Ether und 2N Salzsäure, wobei das ausgefallene Magnesiumbromid weitgehend dekantierend entfernt wurde. Die organische Phase wurde abgetrennt und nacheinander mit 2N Salzsäure, Wasser, gesättigter Natriumhydrogencarbonatlösung und gesättigter Kochsalzlösung gewaschen, dann getrocknet, filtriert und im Vakuum eingeengt.

Der Rückstand wurde im Pumpenvakuum ohne Kolonne unter Verwendung einer kurzen, luftgekühlten Brücke destilliert. Nach einem Vorlauf (25,5 g, Sdp. 25-101 °C/19,95 Pa (0,15 Torr)) destillierte das Produkt (187,5 g, 628 mMol, Sdp. 114 °C/6,65 Pa (0,05 Torr)), das sofort zu einem farblosen, harten Teststoff (Titelverbindung) kristallisierte (Schmp. 75-78 °C).

| | |
|---|---|
| NMR (60 MHz): | δ = 1,10-1,36 (3xd, 18H, $CH_3$); 2,80-3,56 (m, 3H, CH); 6,86-7,40 (m, 6H, arom.-H) |
| MS (DCI, Isobutan): | m/e = 299 ($M+H^+$), 298 ($M^+$), 257 ($M+$-$C_3H_5$·) |

**Verfahrensbeispiel 21**

**2-(p-Fluorphenyl)-3,5,6-triisopropyl-1,4-benzochinon (Schema 3, Formel XX)**

60,0 g (201 mMol) 1-(p-Fluorphenyl)-2,4,5-triisopropylbenzol wurden in einem 2 l-Kolben mit mechanischem Rührer, leistungsfähigem Rückflußkühler, Tropftrichter, Innenthermometer und Inertgaszuleitung/Blasenzähler vorgelegt. Bei -20 °C wurde die Lösung von 68 ml 70 %igen wäßrigem Wasserstoffperoxid in 333 ml Trifluoressigsäure zugetropft. Das Kältebad wurde entfernt, aber zum sofortigen erneuten Einsatz bereit gehalten. Das Reaktionsgemisch erwärmte sich innerhalb 30 Min. auf ca. +20 °C. Hier setzte eine sehr exotherme Reaktion ein, deren Kontrolle die sofortige Kühlung mit einem Trockeneis/Kältebad erforderte. Trotz dieser Kühlung erwärmte sich das Reaktionsgemisch zum Rückfluß. Die Kühlung wurde dann so gehandhabt, daß ein geringer Rückfluß aufrecht erhalten wurde. Nach 10-15 Min. flaute die exotherme Reaktion ab und DC (Cyclohexan/Toluol 1:1) zeigte die vollständige Umsetzung des Ausgangsmaterials ($R_f$ = 0,72) zum gelben Reaktionsprodukt ($R_f$ = 0,45) und polaren Nebenprodukten an.

Das Reaktionsgemisch wurde vorsichtig auf eiskalte Natriumhydrogencarbonatlösung gegossen und 3mal mit Ether extrahiert. Die vereinigten Etherphasen wurden 2mal mit Natriumhydrogencarbonatlösung, dann mit Kochsalzlösung gewaschen, getrocknet und eingeengt. Der Rückstand wurde mit Cyclohexan/Toluol 2:1 durch Kieselgel chromatographiert und ergab 14,6 g (44,5 mMol, Ausb. 22,1 %) eines intensiv gelben Feststoffs (Titelverbindung), Schmp. 122-124 °C.

Gemäß $^1$H-NMR und MS enthielt die so hergestellte Titelverbindung ca. 10 % einer chromatographisch nicht abtrennbaren Verunreinigung (MG = 344).

NMR (270 MHz): δ = 1,15-1,40 (m, 18H, $CH_3$); 1,98 (sept., 1H, CH); 2,63 (sept., 1H, CH); 3,24 (sept., 1H, CH); 7,08 (AA'BB'-System, 4H, arom.-H).

MS (CDI, Isobutan): m/e = 329 ($M+H^+$)

**Verfahrensbeispiel 22**

**2-(p-Fluorphenyl)-3,5,6-triisopropyl-1,4-hydrochinon (Schema 3, Formel III/4)**

Zur Lösung von 11,1 g (33,8 mMol) des Chinons aus Verfahrensbeispiel 21 in 740 ml Ethanol wurden bei Raumtemperatur unter Stickstoff 6,3 g (166,5 mMol) Natriumborhydrid gegeben. Nach einstündigem Rühren trat Entfärbung der gelben Lösung ein. Das Ethanol wurde im Vakuum entfernt und mit Stickstoff belüftet (bei Sauerstoff-Zutritt erfolgt Rückoxidation des gebildeten Hydrochinons unter Gelbfärbung). Der Rückstand wurde unter Eiskühlung vorsichtig mit 500 ml stickstoffgespülter, 2-normaler Salzsäure versetzt (starke Wasserstoffentwicklung, Wärmetönung) und mit 500 ml Ether geschüttelt, der von Lithiumaluminiumhydrid abdekantiert wurde. Die Etherphase wurde abgetrennt, im Vakuum eingeengt und mit Stickstoff belüftet. Der Rückstand wurde im Hochvakuum getrocknet, mit n-Pentan verrührt, unter Stickstoff abgesaugt und mit n-Pentan gewaschen, dann im Hochvakuum getrocknet. Erhalten wurden 9,65 g (29,2 mMol, 86,4 % Ausbeute) farbloser Feststoff (Titelverbindung), Schmp. 193-196 °C.

NMR (270 MHz): δ = 1,23 (d, 6H, $CH_3$); 1,34 (d, 6H, $CH_3$); 1,42 (d, 6H, $CH_3$); 2,66 (sept. 1H, CH); 3,40-3,70 (s, sehr breit, wahrscheinlich eingeschränkte Rotation der Isopropylgruppen, 2H, CH); 4,12 (s, 1H, OH); 4,39 (s, 1H, OH); 7,12-7,28 (m, 4H, arom.-H)

MS (DCI, Isobutan): m/e = 330 ($M^+$)

**Verfahrensbeispiel 23**

**2,5,6-Triisopropyl-3-(p-fluorphenyl)-4-acetoxy-phenol (Schema 3, Formel III/5)**

Zu 4,04 g (12,23 mMol) des Hydrochinons aus Verfahrensbeispiel 22 gab man unter Eiskühlung 1,73 ml (18,34 mMol, 1,5 Äquiv.) Essigsäureanhydrid, gefolgt von 23 ml entgastem, trockenem Pyridin. Man ließ das Reaktionsgemisch unter Argon und Feuchtigkeitsausschluß 3 Tage im Kühlschrank bei 0 °C stehen. DC (Cyclohexan/Diisopropylether 4:1) zeigte weitgehende Umsetzung des Ausgangsmaterials ($R_f$ = 0,60) und Bildung der Titelverbindung ($R_f$ = 0,36) als Hauptprodukt, neben dem Diacetat (Schema 3, Formel XXI) ($R_f$ = 0,21) und dem regioisomeren Monoacetat (Schema 3, Formel III/6) ($R_f$ = 0,41) als Nebenprodukten an. Das Reaktionsgemisch wurde auf 200 ml 2N Salzsäure und 500 ml Ether gegossen und geschüttelt. Die Etherphase wurde nacheinander mit 2mal 100 ml 2N Salzsäure, 100 ml Natriumhydrogencarbonat-Lösung, 50 ml gesättigter Kochsalzlösung gewaschen, getrocknet, filtriert und eingeengt.

Der Rückstand (4,10 g) wurde unter Erwärmen in wenig Toluol gelöst, diese Lösung auf eine Kieselgelsäule aufgetragen und mit Cyclohexan/Diisopropylether 5:1 eluiert. Nach wenig Ausgangsmaterial (200 mg, 0,61 mMol) wurden zunächst 150 mg (0,40 Mol) 2,3,5-Triisopropyl-4-acetoxy-6-(p-fluorphenyl)-phenol (Schema 3, Formel III/6) eluiert, gefolgt von 2,14 g (5,75 mMol, Ausbeute 47,0 %) der Titelverbindung, gefolgt von 940 mg (2,27 mMol) 1,4-Diacetoxy-2,3,5-triisopropyl-6-(p-fluorphenyl)-benzol (Schema 3, Formel XXI). Gesamtausbeute aller Acetylierungsprodukte, bezogen auf umgesetztes Ausgangsmaterial 72,5 %.

Die Titelverbindung wurde als farbloser Feststoff, Schmp. 192-195 °C erhalten.

| | |
|---|---|
| NMR (60 MHz): | $\delta$ = 1,15-1,53 (3xd, 18H, $CH_3$); 1,72 (s, 3H, CO-$CH_3$); 2,3-3,8 (m, 3H, CH); 4,81 (s, 1H, OH), 6,98-7,26 (AA'BB'-System, 4H, arom.-H) |
| MS (DCI, Isobutan): | m/e = 373 ($M+H^+$), 372 ($M^+$), 330 ($M+H^+-CH_3CO$) |

**Verfahrensbeispiel 24**

**1,4-Diacetoxy-2,3,5-triisopropyl-6-(p-fluorphenyl)-benzol (Schema 3, Formel XXI)**

Zu 4,04 g (12,23 mMol) des Hydrochinons aus Verfahrensbeispiel 22 gab man unter Eiskühlung 3,46 ml (36,7 mMol, 3,0 Äquiv.) Essigsäureanhydrid, gefolgt von 23 ml trockenem Pyridin. Reaktionsdurchführung, Aufarbeitung und chromatographische Reinigung erfolgte wie in Verfahrensbeispiel 23 angegeben. Erhalten wurden neben geringen Mengen der regioselektiven Monoacetate 3,06 g (8,22 mMol, Ausbeute 67,2 %) der Titelverbindung als farbloser Feststoff, Schmp. 172-173 °C.

| | |
|---|---|
| NMR (60 MHz): | $\delta$ = 0,9-1,6 (m, 18H, $CH_3$, gehinderte Rotation), 1,70 (s, 3H, CO-$CH_3$), 2,35 (s, 3H, CO-$CH_3$), 2,43-3,73 (m, 3H, CH), 6,97-7,20 (AA'BB'-System, 4H, arom.-H). |
| MS (DCI, Isobutan): | m/e = 415 ($M+H^+$), 414 ($M^+$), 373 ($M+H^+-CH_2=C=O$), 372 ($M^+-CH_2=C=O$), 331 ($M+H^+$-2 $CH_2=C=O$), 330 ($M^+$-2 $CH_2=C=O$). |

**Verfahrensbeispiel 25**

**2,3,5-Triisopropyl-4-acetoxy-6-(p-fluorphenyl)-phenol (Schema 3, Formel III/6)**

Zur Lösung von 1,8 g (4,34 mMol) des Diacetats aus Verfahrensbeispiel 24 in 30 ml 1,2-Dimethoxyethan wurde die Lösung von 114,2 mg (4,77 mMol, 1,1 Äquivalente) Lithiumhydroxid in 9,83 ml Wasser gegeben. Das Reaktionsgemisch wurde bei Raumtemperatur gerührt. Nach kurzer Zeit fiel ein farbloser Feststoff aus, nach dreitägigem Rühren wurde eine klare Lösung erhalten. DC (vgl. Verfahrensbeispiel 20) zeigte nur noch Spuren des Diacetats an, die Titelverbindung als Hauptprodukt neben dem regioisomeren Monoacetat als Nebenprodukt.

Das Reaktionsgemisch wurde auf 2 N Salzsäure gegossen und mit Ether extrahiert. Das Extrakt wurde mit Natriumhydrogencarbonat-Lösung, dann mit gesättigter Kochsalzlösung gewaschen, getrocknet und eingeengt. Säulenchromatographie (vgl. Verfahrensbeispiel 23) ergab 980 mg (2,63 mMol, 60,6 % Ausbeute) der Titelverbindung als farblosen Feststoff, Schmp. 174-177 °C. Darüber hinaus wurden 480 mg (1,29 mMol, 29,7 % Ausbeute) einer Verbindung erhalten, die identisch mit der aus Verfahrensbeispiel 23 war.

| | |
|---|---|
| NMR (60 MHz): | $\delta$ = 0,85-1,53 (m, 18H, $CH_3$, gehinderte Rotation), 2,32 (s, 3H, CO-$CH_3$), 2,40-3,65 (m, 3H, CH), 4,40 (s, 1H, OH), 7,0-7,36 (m, 4H, arom.-H). |

**Verfahrensbeispiel 26**

**(3R,5S)-6-Methylsulfonyloxy-3,5-O-isopropyliden-3,5-dihydroxyhexansäure-tert.-butylester (Formel IV)**

Zur Lösung von 175,7 g (676 mMol) (3R,5S)-6-Hydroxy-3,5-O-isopropyliden-3,5-dihydroxyhexansäure-tert.-butylester (siehe EPA 0 319 847) in 1,7 l absol. Methylenchlorid und 1,7 l absol. Pyridin tropfte man bei 0-5 °C 116,2 g (1,01 Mol, 1,5 Äquiv.) Methansulfonsäurechlorid. Man rührte 90 Min. unter Eiskühlung, engte dann das Reaktionsgemisch bei 30 °C im Vakuum ein und entfernte die Hauptmenge des restlichen Pyridins nach Aufnehmen in Toluol durch Abziehen im Vakuum. Der Rückstand wurde in Toluol aufgenommen und zweimal mit Wasser, einmal mit gesättigter Natriumhydrogencarbonat-Lösung, einmal mit gesättigter Natriumchloridlösung gewaschen, dann getrocknet, filtriert und im Vakuum eingeengt. Das zurückbleibende Öl kristallisierte bei Raumtemperpatur innerhalb weniger Minuten praktisch vollständig. Die Kristalle wurden abgesaugt,

auf der Nutsche zerstoßen, mit kaltem Petrolether gewaschen und im Vakuum getrocknet.

Man erhielt 192,0 g (568 mMol) farblosen Feststoff, Schmp. 75-76 °C. Einengen des Filtrats, Absaugen der Kristalle und Waschen mit wenig kaltem Petrolether lieferte weitere 34,8 g (103 mMol) leicht verunreinigtes Produkt, Schmp. 69-73 °C. Gesamtausbeute an Titelverbindung: 226,8 g (671 mMol, 99,3 %).

NMR (270 MHz, $CD_2Cl_2$): δ = 1,18-1,33 (m, 1H, $CH_2$, axial), 1,36 (s, 3H, $CH_3$), 1,42 (s, 9H, tert.-Bu), 1,46 (s, 3H, $CH_3$), 1,56 (dt, 1H, $CH_2$, äquat.), 2,36 (AB-Teil von ABX-System, 2H, $CH_2$), 3,03 (s, 3H, $CH_3$-$SO_2$), 4,09-4,23 (m, 3H, $OCH_2$ und O-CH), 4,24-4,37 (m, 1H, OCH).

MS (DCI-Isobutan): m/e = 283 ($M + H^+$ - > = )

**Verfahrensbeispiel 27**

**{2,2-Dimethyl-4(S)-[(2-p-fluorphenyl-4-p-fluorphenylthio-6-isopropyl)-phenoxymethyl]-6(R)-tert.-butoxycarbonylmethyl}-1,3-dioxolan (Formel V)**

Zur Lösung von 4,0 g (11,2 mMol) 2-(p-Fluorphenyl)-4-p-fluorphenylthio)-6-isopropyl-phenol (Verfahrensbeispiel 6) in 25 ml trockenem Hexamethylphosphorsäuretriamid (HMPT) gab man 2,02 g (14,6 mMol, 1,3 Äquiv.) gepulvertes Kaliumcarbonat und ca. 10 mg Kronen-Ether 18-Crown-6 (Firma Aldrich). Man rührte die Suspension 20 Min. bei Raumtemperatur, gab dann 4,55 g (13,5 mMol, 1,2 Äquiv.) (3R,5S)-6-Methylsulfonyloxy-3,5-O-isopropyliden-3,5-dihydroxyhexansäure-tert.-butylester (Verfahrensbeispiel 26) hinzu und rührte 2 Tage bei 75-80 °C. Das Reaktionsgemisch färbte sich dunkel und wurde dickflüssiger. Es wurde in 200 ml wäßrige Natriumdihydrogenphosphat-Lösung gegossen und mehrmals mit Ether extrahiert. Die vereinigten Extrakte wurden mit gesättigter Kochsalzlösung gewaschen, getrocknet und im Vakuum eingeengt und ergaben 8,64 g eines bräunlichen Öls. Säulenchromatographie (Cyclohexan/Ethylacetat 10:1 plus 1 o/oo Triethylamin) gab 4,96 g (8,28 mMol, 74,0 % Ausbeute) eines blaßgelben, zähen Öls (Titelverbindung).

NMR (270 MHz, $C_6D_6$): δ = 0,98-1,07 (m, 2H, $CH_2$), 1,19 + 1,20 (2xd, 6H, CH($CH_3$)$_2$), 1,38 (s, 9H, tert.-Bu), 1,39 + 1,41 (2xs, 6H, OC($CH_3$)$_2$), 2,12 (dd, 1H, $CH_2CO_2$),2,42 (dd, 1H, $CH_2CO_2$), 3,27 (dd, 1H, O-$CH_2$), 3,37 (dd, 1H, O-$CH_2$), 3,65 (sept., 1H, CH($CH_3$)$_2$), 3,65-3,76 (m, 1H, O-CH), 4,10-4,21 (m, 1H, O-CH), 6,60 + 6,82 (AA'BB'-System, 4H, arom.-H), 7,12-7,18 (m, 2H, arom.-H), 7,22-7,29 (m, 3H, arom.-H), 7,45 (d, 1H, arom-H)

MS (DCI, Isobutan): m/e = 598 ($M^+$), 543 (M + $H^+$ - > = ), 485.

**Verfahrensbeispiel 28**

**3(R),5(S)-Dihydroxy-6-[(2-p-fluorphenyl-4-p-fluorphenylthio-6-isopropyl)-phenoxy]-hexansäure-tert.-butylester(Formel II/1)**

Die Lösung von 4,47 g (7,47 mMol) des Acetonids aus Verfahrensbeispiel 27 in 50 ml Tetrahydrofuran, 50 ml Ethanol und 5 ml 2 N Salzsäure wurde 16 Std. bei Raumtemperatur gerührt. DC (Cyclohexan/Ethylacetat 1:1) zeigte fast quantitative Umwandlung des Ausgangsmaterials ($R_f$ = 0,78) ins Produkt ($R_f$ = 0,59) an. Das Reaktionsgemisch wurde in wäßrige Natriumhydrogencarbonat-Lösung geschüttet und mehrmals mit Ether extrahiert. Die Extrakte wurden mit gesättigter Kochsalzlösung gewaschen, getrocknet und im Vakuum eingeengt. Der Rückstand (4,46 g bräunliches Öl) wurde durch Säulenchromatographie (Cyclohexan/Ethylacetat 2:1) gereinigt und ergab 3,37 g (6,03 mMol) Titelverbindung als farbloses Öl (Ausbeute 80,8 %).

NMR (270 MHz, $C_6D_6$): δ = 1,1 - ca. 1,4 (m, partiell verdeckt durch starke Singuletts, 2H, $CH_2$), 1,18 (d, 6H, CH($CH_3$)$_2$, 1,31 (s, 9H, tert.-Bu), 2,00 (dd, 1H, $CH_2$-$CO_2$), 2,13 (dd, 1H, $CH_2$-$CO_2$), 3,15 (s, breit, 1H, OH), 3,36 (AB-Teil von ABX-Systemen, 2H, $OCH_2$), 3,52 (s, breit, 1H, OH), 3,56 (sept., 1H, CH($CH_3$)$_2$, 3,76-3,96 (m, 2H, 2x CHOH), 6,61 + 6,79 (AA'BB'-System, 4H, arom.-H), 7,14-7,27 (m, 5H, arom.-H), 7,45 (d, 1H, arom.-H).

MS (FAB, 3-NBA): m/e = 558 ($M^+$), 519, 503 ($M^+$ - > = + $H^+$), 356 ($M^+$ des Phenolbausteins).

**Verfahrensbeispiel 29**

**{2,2-Dimethyl-4(S)-[(2-p-fluorphenyl-4-p-fluorphenylthio-6-cyclopropyl)-phenoxymethyl]-6(R)-tert.-butoxycarbonylmethyl}-1,3-dioxolan (Formel V)**

In Analogie zu Verfahrensbeispiel 27 wurden aus 2,0 g (5,6 mMol) 2-(p-Fluorphenyl)-4-(p-fluorphenylthio)-6-cyclopropylphenol (Verfahrensbeispiel 12) 2,44 g (4,09 mMol, 73,0 % Ausbeute) der Titelverbindung als blaßgelbes, zähes Öl erhalten.

NMR (270 MHz, $C_6D_6$): δ = 0,78 (m, 4H, $CH_2$), 0,98-1,07 (m, 2H, $CH_2$), 1,38 (s, 9H, tert.-Bu), 1,39 + 1,41 (2xs, 6H, $OC(CH_3)_2$), 1,87 (qui, 1H, CH), 2,13 (dd, 1H, $CH_2CO_2$), 2,42 (dd, 1H, $CH_2CO_2$), 3,32 (AB-Teil von ABX-System, 2H, $OCH_2$), 3,64-3,77 (m, 1H, O-CH), 4,10-4,22 (m, 1H, O-CH), 6,61 + 6,83 (AA'BB'-System, 4H, arom.-H), 7,10-7,30 (m, 5H, arom.-H), 7,46 (d, 1H, arom.-H).

MS (DCI, Isobutan): m/e = 596 ($M^+$), 541 ($M + H^+$ - > = ).

**Verfahrensbeispiel 30**

**3(R),5(S)-Dihydroxy-6-[(2-p-fluorphenyl-4-p-fluorphenylthio-6-cyclopropyl)-phenoxy]-hexansäure-tert.-butylester (Formel II/1)**

In Analogie zu Verfahrensbeispiel 28 wurden aus 2,44 g (4,09 mMol) des Acetonids aus Verfahrensbeispiel 29 1,64 g (2,95 mMol, 72,1 % Ausbeute) der Titelverbindung als farbloses, zähes Öl erhalten.

NMR (270 MHz, $C_6D_6$): δ = 0,77 (m, 4H, $CH_2$), 1,10-1,35 (m, partiell verdeckt, 2H, $CH_2$), 1,31 (s, 9H, tert.-Bu), 1,87 (qui, 1H, CH), 1,95-2,18 (AB-Teil von ABX-System, 2H, $CH_2CO_2$-), 3,15 (s, breits, 1H, OH), 3,37 (AB-Teil von ABX-System, 2H, $OCH_2$), 3,53 (s, breit, 1H, OH), 3,75-3,97 (m, 2H, CHOH), 6,61 + 6,80 (AA'BB'-System, 4H, arom.-H), 7,13-7,28 (m, 5H, arom.-H), 7,45 (d, 1H, arom.-H).

MS (FAB, 3-NBA): m/e = 556 ($M^+$), 354 ($M^+$ des Phenolbausteins)

**Verfahrensbeispiel 31**

**{2,2-Dimethyl-4(S)-[[2-(p-fluor-m-methyl-phenyl)-4-p-fluorphenylthio-6-isopropyl]-phenoxymethyl]-6-(R)-tert.-butoxycarbonylmethyl}-1,3-dioxolan (Formel V)**

In Analogie zu Verfahrensbeispiel 27 wurden aus 4,2 g (11,35 mMol) 2-(p-Fluor-m-methyl-phenyl)-4-(p-fluorphenylthio)-6-isopropyl-phenol (Verfahrensbeispiel 15) und 4,55 g (13,5 mMol) des Mesylats (Verfahrensbeispiel 26) nach Chromatographie 5,15 g (8,41 mMol, 74,1 % Ausbeute) der reinen Titelverbindung als farbloses, zähes Öl erhalten.

NMR (270 MHz, $C_6D_6$): δ = 0,97 -1,09 (m, 2H, $CH_2$), 1,20 (d, fein aufgespalten, 6H, $CH(CH_3)_2$), 1,38 (s, 9H, tert.-Bu), 1,39-1,41 (2xs, 6H, $OC(CH_3)_2$), 2,13 (dd, 1H, $CH_2CO_2$), 2,35 (s, 3H, $CH_3$), 2,43 (dd, 1H, $CH_2CO_2$), 3,28 (dd, 1H, O-$CH_2$), 3,38 (dd, 1H, O-$CH_2$), 3,65 (sept., 1H, $CH(CH_3)_2$), 3,65-3,76 (m, 1H, O-CH), 4,09-4,22 (m, 1H, O-CH), 6,60-7,44 (m, 9H, arom.-H).

MS (DCI, Isobutan): m/e = 612 ($M^+$), 557 ($M + H^+$ - > = ).

**Verfahrensbeispiel 32**

**3(R),5(S)-Dihydroxy-6-[(2-p-fluor-m-methyl-phenyl-4-p-fluorphenylthio-6-isopropyl)-phenoxy]-hexansäure-tert.-butylester (Formel II/1)**

In Analogie zu Verfahrensbeispiel 28 wurden aus 5,10 g (8,33 mMol) des Acetonids aus Verfahrensbeispiel 31 nach Chromatographie 3,70 g (6,47 mMol, Ausbeute 77,7 %) farbloses Öl erhalten.

MS (FAB, 3-NBA): m/e = 572 ($M^+$), 517 ($M^+$ -> = + $H^+$), 370 ($M^+$ des Phenolbausteins)

**Verfahrensbeispiel 33**

**{2,2-Dimethyl-4(S)-[(2-p-fluorphenyl-4-phenylthio-6-isopropyl-phenoxymethyl]-6(R)-tert.-butoxycarbonylmethyl}-1,3-dioxolan (Formel V)**

In Analogie zu Verfahrensbeispiel 27 erhielt man aus 4,7 g (13,90 mMol) 2-p-Fluorphenyl-4-phenylthio-6-isopropyl-phenol (Verfahrensbeispiel 7) nach Chromatographie 5,4 g (9,31 mMol, 67,0 % Ausbeute) eines farblosen, zähen Öls.

NMR (270 MHz, $C_6D_6$): $\delta$ = 0,97 -1,08 (m, 2H, $CH_2$), 1,20 (2xd, 6H, $CH(\underline{CH_3})_2$), 1,37 (s, 9H, tert.-Bu), 1,39 + 1,41 (2xs, 6H, $OC(CH_3)_2$), 2,13 (dd, 1H, $CH_2CO_2$),2,42 (dd, 1H, $CH_2CO_2$), 3,32 (AB-Teil von ABX-System, 2H, $OCH_2$), 3,66 (sept., 1H, $C\underline{H}$-$(CH_3)_2$), ~3,65-3,77 (m, 1H, O-CH), 4,09-4,22 (m, 1H, O-CH), 6,62-7,28 (m, 11H, arom.-H).

MS (DCI, Isobutan): m/e = 580 ($M^+$), 525 ($M + H^+$ - > =), 467.

**Verfahrensbeispiel 34**

**3(R),5(S)-Dihydroxy-6-[(2-p-fluorphenyl-4-phenylthio-6-isopropyl)-phenoxy]-hexansäure-tert.-butylester (Formel II/1)**

In Analogie zu Verfahrensbeispiel 28 erhielt man aus 5,35 g (9,2 mMol) des Acetonids aus Verfahrensbeispiel 33 nach Chromatographie 3,65 g (6,76 mMol, Ausbeute 73,3 %) farbloses, zähes Öl.

MS (FAB, 3-NBA): m/e = 540 ($M^+$), 501, 485 ($M^+$ -> = + $H^+$), 338 ($M^+$ des Phenolbausteins).

**Verfahrensbeispiel 35**

**{2,2-Dimethyl-4(S)-[(2-p-fluorphenyl-4-isopropylthio-6-isopropyl-phenoxymethyl]-6(R)-tert.-butoxycarbonylmethyl}-1,3-dioxolan (Formel V)**

Die Suspension von 206 mg (0,68 mMol) 2-(p-Fluorphenyl)-4-(isopropylthio)-6-isopropyl-phenol (Verfahrensbeispiel 8), 271 mg (0,80 mMol) (3R,5S)-6-Methylsulfonyloxy-3,5-O-isopropyliden-3,5-dihydroxyhexansäure-tert.-butylester (Verfahrensbeispiel 26), 221 mg (1,60 mMol) gepulvertem Kaliumcarbonat und einer Mikrospatelspitze (1-2 mg) Kronenether 18-Crown-6 in 6.8 ml trockenem Hexamethylphosphorsäuretriamid wurde 12 Std. auf 65-70 °C, dann noch 6 Std. auf 80 °C erwärmt. DC (Cyclohexan/Ethylacetat 5:1) zeigte vollständige Umsetzung des Ausgangsphenols an. Das Reaktionsgemisch wurde abgekühlt, auf Natriumhydrogencarbonatlösung gegossen und zweimal mit Ether extrahiert. Die vereinigten Etherphasen wurden zweimal mit Wasser, einmal mit gesättigter Kochsalzlösung gewaschen, getrocknet, filtriert und eingeengt. Der Rückstand wurde mit Toluol/Ethylacetat 30:1, später 20:1 über Kieselgel chromatographiert und ergab:

in Frkt. 6-8 40,1 mg eines nicht identifizierten Reaktionsprodukts, $R_f$ (Toluol/Ethylacetat, 20:1):0,40,
in Frkt. 10-15 110,3 mg der Titelverbindung, $R_f$: 0,32, blaßgelbes, zähes Öl,
in Frkt. 23-27 32,5 mg einer Verbindung, der auf Grund von $^1$H-NMR und MS die Struktur

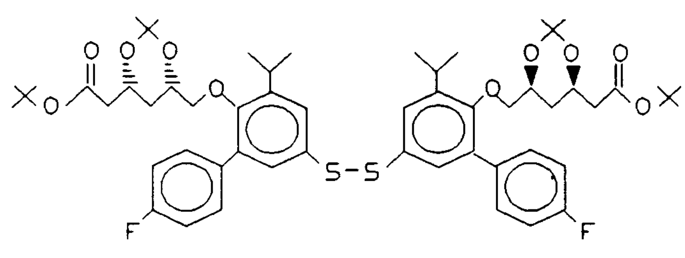

zugeordnet wird. $R_f$: 0,14, blaßgelbes, zähes Öl
in Frkt. 30-35 40,7 mg einer Verbindung, der auf Grund von $^1$H-NMR und MS die Struktur

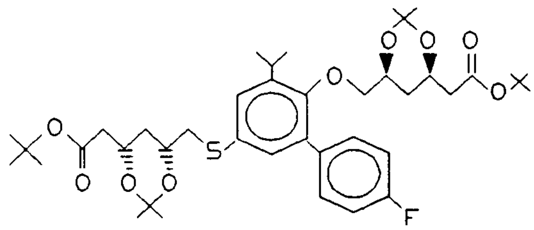

zugeordnet wird. $R_f$: 0,08, blaßgelbes, zähes Öl.

Spektren der Titelverbindung (Frkt. 10-15):

NMR (270 MHz): $\delta$ = 0,97 (d, 6H, $SC(CH_3)_2$, 1,22 (d, fein aufgespalten, 6H, $CH(\underline{CH_3})_2$), 1,15-1,33 (m, 1H, $CH_2$), 1,38 (s, 3H, $CH_3$), 1,42 (s, 3H, $CH_3$), 1,45 (s, 9H, tert.-Bu), 1,82 (dt, 1H, $CH_2$), 2,37 (AB-Teil von ABX-System, 2H, $CH_2$), 2,87 (dd, 1H, $OCH_2$), 3,09 (dd, 1H, $OCH_2$), 3,45 (sept. 1H, CH), 3,64 (sept. 1H, CH), 4,02 (m, 1H, CH), 4,23 (m, 1H, CH), 7,03 - 7,53 (m, 6H, arom.-H).

MS (DCI, Isobutan): m/e = 546 ($M^+$), 489 ($M^+$-tert.-Bu), 433

Spektren von Frkt. 23-27:

NMR (270 MHz): $\delta$ = 1,06 (AB-System, 4H, $CH_2$), 1,20 + 1,21 (2xd, 12H, $CH(\underline{CH_3})_2$), 1,32 (s, 6H, C-$(\underline{CH_3})_2$),1,40 (s, 6H, $C(\underline{CH_3})_2$), 1,43 (s, 18H, tert.-Bu), 2,33 (AB-Teil von ABX-System, 4H, $CH_2CO_2$), 3,20-3,51 (m, 6H, $OCH_2$ und $\underline{CH}(CH_3)_2$), 3,89 (m, 2H, CH), 4,19 (m, 2H, CH), 7,00-7,51 (m, 12H, arom.-H).

MS (FAB): m/e = 1006 ($M^+$), 223.

Spektren von Frkt. 30-35:

NMR (270 MHz): $\delta$ = 1,07 (~ qua, 1H, $CH_2$), 1,16-~1,30 (verdeckt, 2H, $CH_2$), 1,24 (2xd, 6H, CH-$(\underline{CH_3})_2$, 1,32 (s, 3H, $CH_3$), 1,37 (s, 3H, $CH_3$), 1,39 (s, 3H, $CH_3$), 1,41 (s, 3H, $CH_3$), 1,43 + 1,44 (2xs, 18H, tert.-Bu), 1,80 (dt, 1H, $CH_2$), 2,21-2,48 (2xAB-Teil von ABX-System, 4H, $CH_2CO_2$), 2,87 (dd, 1H, $SCH_2$), 3,08 (dd, 1H, $SCH_2$), 3,26 (dd, 1H, $OCH_2$), 3,37 (dd, 1H, $OCH_2$), 3,47 (sept., 1H, $\underline{CH}(CH_3)_2$), 3,89 (m, 1H, CH), 4,02 (m, 1H, CH), 4,22 (m, 2H, CH), 7,03-7,26 (m, 4H, arom.-H), 7,45-7,52 (m, 2H, arom.-H).

MS (DCI, Isobutan): m/e = 748 ($M^+$), 689 ($M^+$ - tert.-Bu), 577, 519.

**Verfahrensbeispiel 36**

**3(R),5(S)-Dihydroxy-6-[2-p-fluorphenyl-4-isopropylthio-6-isopropyl)-phenoxy]-hexansäure-tert.-butylester (Formel II/1)**

In Analogie zu Verfahrensbeispiel 28 erhielt man aus 100 mg des Acetonids (Verfahrensbeispiel 35, Frkt. 10-15) nach Chromatographie 74 mg farbloses, zähes Öl.

MS (FAB, 3-NBA): m/e = 506 ($M^+$), 451 ($M^+$ - >= + $H^+$), 304 ($M^+$ des Phenolbausteins).

**Verfahrensbeispiel 37**

**⟨2,2-Dimethyl-4(S)-{2-isopropyl-4-[(3-isopropyl-4-hydroxy-5-p-fluorphenyl-1-phenylthio)-2-propyl-2-thio]-6-p-fluorphenyl-phenoxymethyl}-6(R)-tert.-butoxycarbonylmethyl⟩-1,3-dioxolan (Schema 2, Formel V/1)**

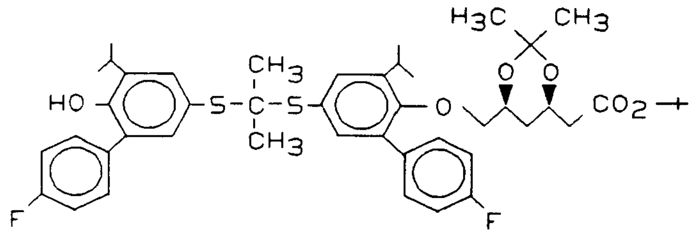

"Mono-Kupplungsprodukt"

und

**4,4-(Isopropylidendithio)-bis-⟨{1-[(2S,4R)-2,4-O-isopropyliden-2,4-dihydroxy-5-tert.-butoxycarbonyl]-pentoxy-2-isopropyl-6-p-fluorphenyl}-benzol⟩ (Schema 2, Formel XII)**

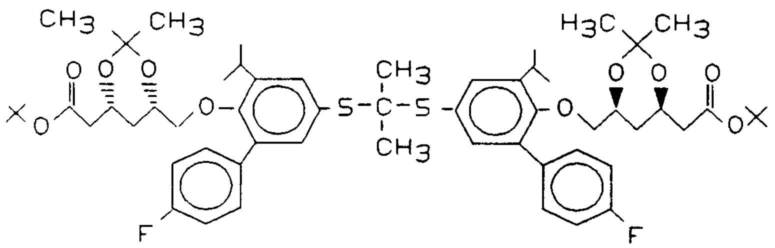

"Doppel-Kupplungsprodukt"

und

**Ansatz 1: Bevorzugte Bildung des Monokupplungsprodukts**

Die Suspension aus 4,30 g (7,61 mMol) 4,4-(Isopropylidendithio)-bis-[2-isopropyl-6-p-fluorphenyl)-phenol] (Verfahrensbeispiel 5), 2,50 g (18,09 mMol) gepulvertem Kaliumcarbonat und 10 mg Kronenether 18-Crown-6 in 50 ml trockenem HMPT wurde 30 Min. bei Raumtemperatur gerührt. 3,09 g (9,13 mMol, 1,2 Äquivalente) (3R,5S)-6-Methylsulfonyloxy-3,5-O-isopropyliden-3,5-dihydroxyhexansäure-tert.-butyl ester (Verfahrensbeispiel 26) wurde zugegeben und das Reaktionsgemisch wurde 12 Std. bei 80-85 °C gerührt. Das abgekühlte Gemisch wurde in 25%ige wäßrige Natriumdihydrogenphosphatlösung gegossen und zweimal mit Ether extrahiert. Die Extrakte wurden mit gesättigter Kochsalzlösung gewaschen, getrocknet und eingeengt. Der Rückstand zeigte bei DC-Analyse (Cyclohexan/Toluol/Ethylacetat/Triethylamin 10:10:1:$10^{-3}$) neben einer Spur des Ausgangsphenols ($R_f$ = 0,49) zwei Produkte ($R_f$ = 0,34 und 0,26). Säulenchromatographie über Kieselgel mit diesem Laufmittel ergab 2,598 g (3,22 mMol) des weniger polaren Monokupplungsprodukts, Ausb. 42,3 %, als farblosen Feststoff, Schmp. 47-50 °C und 1,704 g (1,62 mMol) des polaren Doppelkupplungsprodukts, Ausb. 21,3 %, als farblosen Feststoff, Schmp. 48-51 °C.

Spektren des Monokupplungsprodukts:

| | |
|---|---|
| NMR (270 MHz, $C_6D_6$): | δ = 0,98-1,07 (m, 2H, $CH_2$), 1,24/1,28/1,30 (3xs, 12H, CH($\underline{CH_3}$)$_2$), 1,38 (s, 9H, tert.-Bu), 1,38/1,39 (2xs, 6H, OC($CH_3$)$_2$), 1,67 (s, 6H, S-C($\underline{CH_3}$)$_2$-S), 2,12 (dd, 1H, $CH_2CO_2$), 2,42 (dd, 1H, $CH_2CO_2$), 3,22-3,42 (m, 3H, $\overline{OCH_2}$ + C$\underline{H}$($CH_3$)$_2$), 3,62-3,68 (m, 2H, CH + C$\underline{H}$($CH_3$)$_2$), 4,10-4,22 (m, 1H, CH), 6,62-7,15 (m, 6H, arom.-H), 7,32-7,41 (m, 2H, arom.-H), 7,51/7,67/7,78/7,87 (4xd, 4x1H, arom.-H). |
| MS (FAB, 3-NBA/LiI): | m/e = 813 (M + $Li^+$), 625, 303. |

Spektren des Doppelkupplungsprodukts:

| | |
|---|---|
| NMR (270 MHz, $C_6D_6$): | δ = 0,90-1,08 (m, 4H, $CH_2$), 1,28 und 1,32 (d, 12H, CH($\underline{CH_3}$)$_2$), 1,39 (s, 18H, tert.-Bu), 1,40 (2xs, 12H, OC($CH_3$)$_2$), 1,66 (s, 6H, S-C($CH_3$)$_2$-S), 2,12 (dd, 2H, $CH_2CO_2$), 2,43 (dd, 2H, $CH_2CO_2$), 3,27 (dd, 2H, $OCH_2$), 3,39 (dd, 2H, $OCH_2$), 3,63-3,78 (m, 4H, C$\underline{H}$($CH_3$)$_2$ und CH), 4,11-4,23 (m, 2H, CH), 6,82-6,91 (m, 4H, arom.-H), 7,32-7,42 (m, 4H, arom.-H), 7,68 (d, 2H, arom.-H), 7,88 (d, 2H, |

arom.-H).
MS (FAB, 3-NBA/LiI): m/e = 1055 ($M + Li^+$), 746, 625, 431, 303.

**Ansatz 2: Bevorzugte Bildung des Doppelkupplungsprodukts**

Die Suspension aus 26,0 g (46 mMol) des Phenols (Verfahrensbeispiel 5), 15,3 g (110,4 mMol, 2,4 Äquivalenten) Kaliumcarbonat und 50 mg 18-Crown-6 in 150 ml trockenem HMPT wurde 15 Min. bei Raumtemperatur gerührt. 23,3 g (69 mMol, 1,5 Äquivalente) des Mesylats (Verfahrensbeispiel 26) wurden zugegeben und das Reaktionsgemisch wurde 15 Std. bei 85 °C gerührt. Das zähflüssige Reaktionsgemisch wurde mit weiteren 100 ml HMPT verdünnt und noch 4 Std. auf 85 °C erwärmt. Das abgekühlte Reaktionsgemisch wurde auf 2 N Salzsäure/Eis (1:1) gegossen, mit Ether extrahiert, die Extrakte mit Kochsalzlösung gewaschen, getrocknet und eingeengt. Säulenchromatographie (Cyclohexan/Toluol/Ethylacetat 30:10:1 → 10:10:1) gab
11,84 g (31,9 % Ausbeute) des Monokupplungsprodukts und
19,80 g (41,0 % Ausbeute) des Doppelkupplungsprodukts.

**Verfahrensbeispiel 38**

**3(R),5(S)-Dihydroxy-6-{2-isopropyl-4-[(3-isopropyl-4-hydroxy-5-p-fluorphenyl-1-phenylthio)-2-propyl-2-thio]-6-p-fluorphenyl-phenoxy}-hexansäure-tert.-butylester (Formel II/1)**

Die Lösung von 11,84 g (14,7 mMol) des Monokupplungsprodukts (Verfahrensbeispiel 37, Ansatz 2) in 50 ml THF, 50 ml Ethanol und 10 ml 2 N Salzsäure wurde 16 Std. bei Raumtemperatur gerührt. Das Reaktionsgemisch wurde in Natriumhydrogencarbonatlösung gegossen, dreimal mit Ether extrahiert und die Extrakte mit Kochsalzlösung gewaschen, getrocknet und eingeengt. Der Rückstand wurde mit Toluol/Ethylacetat (20:1 → 5:1) über Kieselgel chromatographiert und ergab 7,8 g (10,17 mMol, 69,3 % Ausbeute) eines farblosen, zähen Öls.

NMR (270 MHz, $C_6D_6$): δ = 1,08-1,22 (m, 2H, $CH_2$), 1,26/1,27 (2xd, 12H, $CH(\underline{CH_3})_2$), 1,32 (s, 9H, tert.-Bu), 1,67 (s, 6H, $S\text{-}C(CH_3)_2\text{-}S$), 2,00 (dd, 1H, $\underline{CH_2CO_2}$), 2,14 (dd, 1H, $CH_2CO_2$), 3,08 (d, 1H, OH), 3,28-3,42 (m, 3H, $OCH_2$ und $\underline{CH}(CH_3)_2$), 3,51 (d, 1H, OH), 3,59 (sept., 1H, $\underline{CH}(CH_3)_2$), 3,77-3,96 (m, 2H, CH), 4,97 (s, 1H, OH), 6,63-6,98 (m, 6H, arom.-H), 7,32 (m, 2H, arom.-H), 7,51/7,67/7,78/7,87 (4xd, 4x1H, arom.-H).

MS (FAB, 3-NBA/LiI): m/e = 773 ($M + Li^+$), 415, 303.

**Verfahrensbeispiel 39**

**4,4-(Isopropylidendithio)-bis-⟨{1-[(2S,4R)-dihydroxy-5-tert.-butoxycarbonyl]-pentoxy-2-isopropyl-6-p-fluorphenyl}-benzol⟩**

Die Lösung von 12,2 g (11,3 mMol) des Doppelkupplungsprodukts (Verfahrensbeispiel 37, Ansatz 2) in 250 ml THF, 250 ml Ethanol und 20 ml 2 N Salzsäure wurde 12 Std. bei Raumtemperatur gerührt. Das Reaktionsgemisch wurde in wäßrige Natriumdihydrogenphosphatlösung gegossen, dreimal mit Ether extrahiert und die Extrakte wurden mit Kochsalzlösung gewaschen, getrocknet und eingeengt. Säulenchromatographie (Kieselgel) des Rückstands mit Toluol/Ethylacetat 6:1 ergab 798 mg (6,8 % Ausbeute) eines farblosen Öls, bei dem nur eine der beiden Acetonidgruppen hydrolysiert war und folgende Formel aufwies:

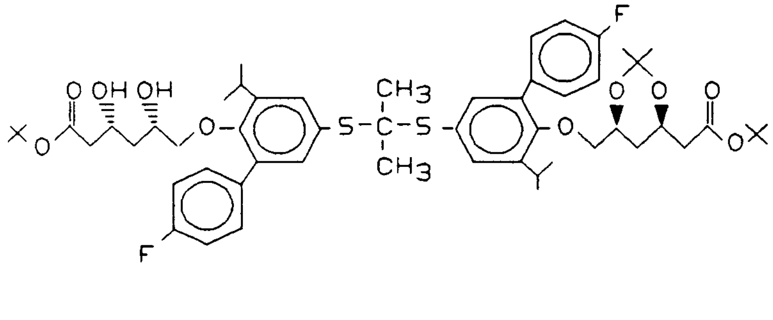

MS (FAB, 3-NBA/LiI): m/e = 1015 ($M+Li^+$), 449, 303.

Weiteres Eluieren mit Methylenchlorid/Methanol 10:1 gab 8,67 g (77 % Ausbeute) der Titelverbindung als zähes Öl.

NMR (270 MHz, $C_6D_6$): δ = 1,12-1,23 (m, 2H, $CH_2$), 1,28 (d, 12H, $CH(\underline{CH_3})_2$), 1,32 (s, 18H, tert.-Bu), ~1,35-1,42 (m, 2H, $CH_2$), 1,66 (s, 6H, $S-C(\underline{CH_3})_2-S$), 2,01 (dd, 2H, $CH_2CO_2$), 2,14 (dd, 2H, $CH_2CO_2$), 3,13 (d, 2H, OH), 3,36 (AB-Teil vom ABX-System, 4H, $OCH_2$), 3,53 (d, 2H, OH), 3,60 (2H, sept., $C\underline{H}(CH_3)_2$, 3,78-3,96 (4H, m, CH), 6,82 (m, 4H, arom.-H), 7,32 (m, 4H, arom.-H), 7,56 (d, 2H, arom.-H), 7,87 (d, 2H, arom.-H).

MS (FAB, 3-NBA/LiI): m/e = 975 ($M+Li^+$), 455, 449, 415.

**Verfahrensbeispiel 40**

**{2,2-Dimethyl-4(S)-[(2,3,5-triisopropyl-4-acetoxy-6-p-fluorphenyl)-phenoxymethyl]-6(R)-tert.-butoxycarbonylmethyl}-1,3-dioxolan (Formel V)**

Die Suspension von 650 mg (1,75 mMol) 2,3,5-Triisopropyl-4-acetoxy-6-p-fluorphenyl-phenol (Verfahrensbeispiel 25), 652 mg (1,90 mMol) (3R,5S)-6-Methylsulfonyloxy-3,5-O-isopropyliden-3,5-dihydroxy-hexansäure-tert.-butylester (Verfahrensbeispiel 26), 518 mg (3,75 mMol) Kaliumcarbonat-Pulver und einem Kristall 18-Crown-6 in 9,6 ml absol. DMSO wurde 12 Std. bei 70 °C gerührt. Die Suspension wurde zähflüssig. Es wurden weitere 9,6 ml DMSO zugegeben und die Temperatur auf 75-80 °C erhöht. Nach 30 Min. wurden weitere 320 mg (0,95 mMol) des Mesylats und 240 mg (1,75 mMol) Kaliumcarbonat-Pulver zugegeben. Nach 10 Std. ließ man abkühlen, goß das Reaktionsgemisch auf wäßrige Natriumhydrogencarbonatlösung und extrahierte dreimal mit Ether. Die vereinigten Etherphasen wurden mit Natriumhydrogencarbonat-Lösung, dann mit Wasser, dann mit NaCl-Lösung gewaschen, getrocknet, filtriert und eingeengt. Der Rückstand wurde mit Cyclohexan/Ethylacetat 10:1 + 1 o/oo Triethylamin durch Kieselgel 35-70 µm chromatographiert. Man erhielt 770 mg (1,25 mMol, 71,4 % Ausbeute) eines farblosen Feststoffs (Titelverbindung), Schmp. 145-147 °C.

NMR (270 MHz, $C_6D_6$): δ = [1,08 (m), 1,22 (d), 1,27 (s), 1,32 (d), 1,41 (s), 1,47 (d), insgesamt 35H, $3xCH(\underline{CH_3})_2$, $CH_2$, $O-C(CH_3)_2-O$, tert.-Bu, offenbar gehinderte Rotation der Isopropylgruppen], 1,94 (s, 3H, OAc), 2,17 (dd, 1H, $CH_2CO_2$), 2,48 (dd, 1H, $CH_2CO_2$), 3,01 (sept., 1H, $C\underline{H}(CH_3)_2$), 3,2-4,0 (m, 5H, $OCH_2$, CH, $2xC\underline{H}(CH_3)_2$, Signale der Isopropylgruppen sehr breit, offenbar gehinderte Rotation), 4,16 (m, 1H, CH), 6,71-7,28 (m, 4H, arom.-H).

MS (DCI, Isobutan): m/e = 614 ($M^+$), 599 ($M^+-CH_3$), 572 ($M^+-CH_2=C=O$), 559, 557, ($M^+$-tert.-Bu), 501.

**Verfahrensbeispiel 41**

**3(R),5(S)-Dihydroxy-6-[(2,3,5-triisopropyl-4-acetoxy-6-p-fluorphenyl)-phenoxy]-hexansäure-tert.-butylester (Formel II/1)**

Die Lösung aus 765 mg (1,15 mMol) des Acetonids (Verfahrensbeispiel 40) in 13 ml Ethanol, 13 ml THF und 1,3 ml 2 N Salzsäure wurde 18 Std. bei Raumtemperatur gerührt. DC (Cyclohexan/Ethylacetat 2:1) zeigte saubere, praktisch quantitative Umsetzung des Acetonids ($R_f=0,63$) zum Produkt ($R_f=0,26$) an. Das Reaktionsgemisch wurde mit Kaliumhydrogencarbonat-Pulver neutralisiert, Ether und Wasser wurden zugefügt und nach kräftigem Schütteln wurde die Etherphase abgetrennt. Sie wurde mit Kochsalzlösung gewaschen, getrocknet, filtriert, eingeengt. Der Rückstand wurde mit Cyclohexan/Ethylacetat 2:1 + 1 o/oo Triethylamin über Kieselgel chromatographiert und ergab 632 mg (1,10 mMol, 95,6 % Ausbeute) farblosen Feststoff (Titelverbindung), Schmelzpunkt 119-122 °C.

NMR (270 MHz, $C_6D_6$): δ = 0,9-1,5 (m, 29H, $3xCH(\underline{CH_3})_2$, tert.-Bu, $CH_2$; offenbar gehinderte Rotation der Isopropylgruppen), 1,93 (s, 3H, OAc), 2,12 (AB-Teil von ABX-System, 2H, $CH_2CO_2$, [2,99 (m, 2H) und 3,38-4,12 (m, 7H), $3xC\underline{H}(CH_3)_2$, $OCH_2$, 2xCH, 2x0H, offenbar gehinderte Rotation der Isopropylgruppen], 6,78 (m, 2H, arom.-H), 7,03 (m, 2H, arom.-H).

MS (DCI, Isobutan): m/e = 575 ($M+H^+$), 574 ($M^+$), 519 ($M+H^+$ - > =), 330 ($M^+$ des Hydrochinon-Bausteins)

**Verfahrensbeispiel 42**

**{2,2-Dimethyl-4(S)-[2,5,6-triisopropyl-3-p-fluorphenyl-4-acetoxy-phenoxymethyl]-6(R)-tert.-butoxycarbonylmethyl}-1,3-dioxolan (Formel V)**

Die Suspension von 1,28 g (3,4 mMol) 2,5,6-Triisopropyl-3-p-fluorphenyl-4-acetoxy)-phenol (Verfahrensbeispiel 23), 1,28 g (3,8 mMol) (3R,5S)-6-Methylsulfonyloxy-3,5-O-isopropyliden-3,5-dihydroxyhexansäure-tert.-butylester (Verfahrensbeispiel 26), 1,02 g (7,6 mMol) Kaliumcarbonat-Pulver und einem Kristall 18-Crown-6 in 19 ml absol. DMSO wurde 12 Std. bei 70 °C gerührt, dann wurde die Temperatur auf 75-80 °C erhöht. Nach 30 Min. wurden weitere 575 mg (1,7 mMol) des Mesylats und 470 mg (3,4 mMol) Kaliumcarbonat-Pulver zugegeben. Nach 10 Std. ließ man abkühlen.

Aufarbeitung und Chromatographie erfolgten wie im Verfahrensbeispiel 40. Man erhielt 1,23 g (2,0 mMol, Ausbeute 60 %) farblosen Feststoff (Titelverbindung), Schmp. 151-153 °C.

NMR (270 MHz, $C_6D_6$): δ = 1,04-1,55 [m, 38H, 3xCH($\underline{CH_3}$)$_2$, O-C($CH_3$)$_2$-O, tert.-Bu, $CH_2$, OAc; offenbar gehinderte Rotation der Isopropylgruppen], 2,22 (dd, 1H, $CH_2CO_2$), 2,51 (dd, 1H, $CH_2CO_2$), 3,39 (m, breit 1H, C$\underline{H}$($CH_3$)$_2$), 3,58 (sept., 1H, C$\underline{H}$-($CH_3$)$_2$), 3,71 (AB-Teil von ABX-System, 2H, $OCH_2$), 4,08-4,37 (m, 3H, C$\underline{H}$, C$\underline{H}$($CH_3$)$_2$), 6,71-7,42 (m, 4H, arom.-H).

MS (DCI, Isobutan): m/e = 614 ($M^+$), 572, 559, 501

**Verfahrensbeispiel 43**

**3(R),5(S)-Dihydroxy-6-[(2,5,6-triisopropyl-3-p-fluorphenyl-4-acetoxy)-phenoxy]-hexansäure-tert.-butylester (Formel II/1)**

Die Lösung von 1,22 g (2,0 mMol) des Acetonids (Verfahrensbeispiel 42) in 25 ml Ethanol, 25 ml THF und 2,5 ml 2 N Salzsäure wurde 18 Std. bei Raumtemperatur gerührt. Aufarbeitung und Chromatographie wie in Verfahrensbeispiel 41 gab 1,03 g (1,8 mMol, Ausbeute 90 %) der Titelverbindung als farblosen Feststoff, Schmp. 61-63 °C. Gemäß $^1$H-NMR enthielt dieses Produkt ca. 5 % einer Verunreinigung.

NMR: Bei 27 °C ($C_6D_6$) waren die meisten Signale breit und komplex. Im gleichen Lösungsmittel bei 70 °C wurden die Signale definiert:
δ = 1,17 (2xd, 6H, CH($\underline{CH_3}$)$_2$), 1,37 (s, 9H, tert.-Bu), 1,42-1,47 (2xd + 1xs, 15H, 2xCH($\underline{CH_3}$)$_2$ + OAc), 1,69 (AB-System, 2H, $CH_2$), 2,25 (AB-Teil von ABX-System, 2H, $CH_2CO_2$), 3,03 (s, 1H, OH), 3,31 (s, 1H, OH), 3,35 (sept., 1H, C$\underline{H}$($CH_3$)$_2$), 3,56 (sept., 1H, C$\underline{H}$($CH_3$)$_2$), 3,77 (AB-Teil von ABX-System, 2H, $OCH_2$), 4,02 (sept., 1H, C$\underline{H}$($CH_3$)$_2$), 4,17 (~qui, 1H, CH), 4,28 (~qui, 1H, CH), 6,83 (m, 2H, arom.-H), 6,98-7,17 (m, 2H, arom.-H).

MS (DCI, Isobutan): m/e = 575 (M + $H^+$), 574 ($M^+$), 519 (M + $H^+$-> =), 330 ($M^+$ des Hydrochinonbausteins)

**Beispiel 1**

**3(R),5(S)-Dihydroxy-6-[2-p-fluorphenyl-4-p-fluorphenylthio-6-isopropyl)-phenoxy]-hexansäure-Natriumsalz (Formel II/2)**

Die Suspension von 3,07 g (5,50 mMol) des tert.-Butylesters (Verfahrensbeispiel 28) in 30 ml Ethanol und 5,56 ml (5,56 mMol, 1,01 Äquivalente) 1 N-Natronlauge wurde 3 Std. bei Raumtemperatur gerührt. Dabei entstand eine klare Lösung und DC (Chloroform/Methanol 4:1) zeigte vollständige Umsetzung des tert.-Butylesters ($R_f$ = 0,82) zum polaren Produkt ($R_f$ = 0,62) an. Die Lösungsmittel wurden im Vakuum entfernt. Zweimal wurde dem Rückstand Toluol zugesetzt und jeweil im Vakuum abgezogen, um Wasserreste azeotrop zu entfernen. Der kristalline Rückstand wurde mit Cyclohexan gewaschen, dann im Hochvakuum zur Gewichtskonstanz getrocknet.

Man erhielt 2,67 g (93 % Ausbeute) eines schwachgelben Feststoffs (Titelverbindung), der bei 192-195 °C unter Zersetzung (dunkelbraun-Färbung) schmilzt.

NMR (270 MHz, DMSO-$d_6$): δ = 1,18 (d, 6H, CH($\underline{CH_3}$)$_2$), 1,29 (t, 2H, $CH_2$), 1,40 (s, 1H, OH), 1,77 (dd, 1H, $CH_2CO_2$), 1,99 (dd, 1H, $CH_2CO_2$), 3,21 (AB-Teil von ABX-System, 2H, $OCH_2$), 3,46 (sept., 1H, C$\underline{H}$($CH_3$)$_2$), 3,66 (m, 2H, CH), 4,88 (s, br., 1H, OH), 7,04 (d, 1H, arom.-H), 7,18-7,28 (m, 5H, arom.-H), 7,37-7,57 (m,

4H, arom.-H).

**Beispiel 2**

**3(R),5(S)-Dihydroxy-6-[2-p-fluorphenyl-4-p-fluorphenylthio-6-cyclopropyl)-phenoxy]-hexansäure-Natriumsalz (Formel II/2)**

In Analogie zu Beispiel 1 erhielt man aus 1,63 g (2,93 mMol) des tert.-Butylesters (Verfahrensbeispiel 30) 1,25 g eines Feststoffs (Titelverbindung), der bei 190-197 °C unter Zersetzung schmilzt.

NMR (270 MHz, DMSO-$d_6$): δ = 0,78 (m, 4H, $CH_2$), 1,29 (t, 2H, $CH_2$), 1,41 (s, 1H, OH), 1,78 (dd, 1H, $CH_2CO_2$), 1,88 (qui, 1H, CH), 2,00 (dd, 1H, $CH_2CO_2$), 3,22 (AB-Teil von ABX-System, 2H, $OCH_2$), 3,67 (m, 2H, CH), 4,89 (s, br., 1H, OH), 7,05 (d, 1H, arom.-H), 7,16-7,29 (m, 5H, arom.-H), 7,36-7,58 (m, 4H, arom.-H).

**Beispiel 3**

**3(R),5(S)-Dihydroxy-6-[2-p-fluor-m-methyl-phenyl-4-p-fluorphenylthio-6-isopropyl)-phenoxy]-hexansäure-Natriumsalz (Formel II/2)**

In Analogie zu Beispiel 1 erhielt man aus 3,69 g (6,45 mMol) des tert.-Butylesters (Verfahrensbeispiel 32) 3,21 g farblosen Feststoff (Titelverbindung), der bei 197-201 °C unter Zersetzung schmilzt.

NMR (270 MHz, DMSO-$d_6$): δ = 1,19 (d, 6H, CH($CH_3$)$_2$), 1,29 (t, 2H, $CH_2$), 1,41 (s, 1H, OH), 1,78 (dd, 1H, $CH_2CO_2$), 1,98 (dd, 1H, $CH_2CO_2$), 2,35 (s, 3H, $CH_3$), 3,22 (AB-Teil von ABX-System, 2H, $OCH_2$), 3,47 (sept., 1H, CH($CH_3$)$_2$, 3,66 (m, 2H, CH), 4,90 (s, br., 1H, OH), 7,05 (d, 1H, arom.-H), 7,17-7,56 (m, 8H, arom.-H).

**Beispiel 4**

**3(R),5(S)-Dihydroxy-6-[(2-p-fluorphenyl-4-p-phenylthio-6-isopropyl)-phenoxy]-hexansäure-Natriumsalz (Formel II/2)**

In Analogie zu Beispiel 1 erhielt man aus 3,64 g (6,74 mMol) des tert.-Butylesters (Verfahrensbeispiel 34) 3,13 g blaßgelben Feststoff (Titelverbindung), der bei 190-193 °C unter Zersetzung schmilzt.

**Beispiel 5**

**3(R),5(S)-Dihydroxy-6-[(2-p-fluorphenyl-4-isopropylthio-6-isopropyl)-phenoxy]-hexansäure-Natriumsalz (Formel II/2)**

In Analogie zu Beispiel 1 erhielt man aus 65 mg (0,13 mMol) des tert.-Butylesters (Verfahrensbeispiel 36) 54 mg gelblichen Feststoff (Titelverbindung).

**Beispiel 6**

**3(R),5(S)-Dihydroxy-6-{2-isopropyl-4-[(3-isopropyl-4-natriumoxy-5-p-fluorphenyl-1-phenylthio)-2-propyl-2-thio] 6-p-fluorphenyl-phenoxy}-hexansäure-Natriumsalz (Formel II/2)**

Zur Lösung von 7,8 g (10,17 mMol) des tert.-Butylesters (Verfahrensbeispiel 38) in 75 ml Ethanol gab man 20,5 ml (20,5 mMol, 2,02 Äquivalente) 1 N-Natronlauge und rührte 2 Stunden bei Raumtemperatur. DC (Methylenchlorid/Methanol 10:1) zeigte vollständige Umsetzung des Esters ($R_f$ = 0,70) zum polaren Produkt ($R_f$ = 0,37) an. Lösungsmittel wurden im Vakuum entfernt. Man nahm viermal in Ethanol auf und zog das Lösungsmittel jeweils im Vakuum ab. Der Rückstand wurde mit Cyclohexan gewaschen, dann im Hochvakuum zur Gewichtskonstanz getrocknet. Man erhielt 7,35 g (9,74 mMol, 95,7 % Ausbeute) eines gelblichen Feststoffs (Titelverbindung), der bei 185 °C unter Zersetzung zu schmelzen beginnt und bei 200-210 °C in eine schwarze Schmelze übergeht.

NMR (270 MHz, DMSO-$d_6$): δ = 1,14 (d, fein aufgespalten, 6H, CH($CH_3$)$_2$), 1,22 (d, 6H, CH($CH_3$)$_2$)-,1,32 (t, 2H, $CH_2$), 1,40 (s, 6H, S-C($CH_3$)$_2$-S), 1,82 (dd, 1H, $CH_2CO_2$),

2,03 (dd, 1H, $CH_2CO_2$),3,15-3,55 (m, 6H, $OCH_2$, 2xOH, 2xC<u>H</u>($CH_3$)$_2$), 3,68 (~qui, 2H, CH), 6,86 (d, 1H, arom.-H), 7,00 (AA'BB', 2H, arom.-H), 7,07 (d, 1H, arom.-H), 7,22 (AA'BB', 2H, arom.-H), 7,33 (d, 1H, arom.-H), 7,50-7,62 (m, 3H, arom.-H).

**Beispiel 7**

**4,4-(Isopropylidendithio)-bis-⟨{1-{(2S,4R)-dihydroxy-5-natriumcarboxy]-pentoxy-2-isopropyl-6-p-fluorphenyl}-benzol⟩ (Formel II/2)**

Zur Lösung von 5,5 g (5,65 mMol) des tert.-Butylesters (Verfahrensbeispiel 39) in 73 ml Ethanol gab man 11,4 ml (11,4 mMol, 2,02 Äquivalente) 1 N-Natronlauge und rührte 2 Std. bei Raumtemperatur. Das Lösungsmittel wurde im Vakuum entfernt, der Rückstand fünfmal in Methanol aufgenommen und jeweils im Vakuum zur Trockne eingeengt. Der Rückstand wurde mit Cyclohexan gewaschen, dann im Hochvakuum zur Gewichtskonstanz getrocknet. Man erhielt 5,08 g (5,64 mMol, 100 % Ausbeute) eines farblosen Feststoffs, der sich bei 225-250 °C unter Dunkelfärbung zersetzt.

NMR (270 MHz, DMSO-$d_6$): $\delta$ = 1,21 (d, 12H, CH($CH_3$)$_2$), 1,32 (t, 2H, $CH_2$), 1,50 (s, 6H, S-C($CH_3$)$_2$-S)), 1,78 (dd, 1H, $CH_2CO_2$), 2,01 (dd, 1H, $CH_2CO_2$), 3,23 (m, 4H, $OCH_2$), 3,39-3,56 (m, 2H, C<u>H</u>($CH_3$)$_2$), 3,58-3,74 (m, 4H, CH), 4,90 (s, br., 2H, OH), 7,12-7,37 und 7,48-7,62 (m, 12H, arom.-H).

**Beispiel 8**

**3(R),5(S)-Dihydroxy-6-[(2,3,5-triisopropyl-4-hydroxy-6-p-fluorphenyl)-phenoxy]-hexansäure-Natriumsalz (Formel II/2)**

Die Suspension von 363 mg (0,63 mMol) des tert.-Butylesters (Verfahrensbeispiel 41) in 3,6 ml absol. Ethanol wurde in einem Eisbad gekühlt und mit einer Spritze 1,21 ml (1,21 mMol, 2,02 Äquivalente) 1 N-Natronlauge zugefügt. Das Reaktionsgemisch wurde bei Raumtemperatur gerührt und ging rasch in eine klare Lösung über. DC ((Chloroform/Methanol 5:1) nach 3 Stunden zeigte praktisch vollständige Umwandlung des Ausgangsmaterials ($R_f$ = 0,97) ins Produkt ($R_f$ = 0,28) an. Die Lösungsmittel wurden abgezogen, der Rückstand zweimal in Toluol aufgenommen und jeweils im Vakuum zur Trockne eingeengt. Der Rückstand wurde zweimal mit Diisopropylether, einmal mit Ether gewaschen und ergab 345 mg eines farblosen, feinen Pulvers, das sich bei 239-242 °C unter Braunfärbung zersetzte und schmolz. Dieses Material enthielt 1 Mol-Äquivalent Natriumacetat. Seine Bruttoformel betrug somit $C_{27}H_{36}FO_6Na$ x $C_2H_3O_2Na$ = $C_{29}H_{39}FO_8Na_2$ - (MG 580,60)
Die Ausbeute betrug 94 % an Titelverbindung.

**Beispiel 9**

**3(R),5(S)-Dihydroxy-6-[2,5,6-triisopropyl-3-p-fluorphenyl)-4-hydroxy)-phenoxy]-hexansäure-Natriumsalz (Formel II/2)**

Die Suspension von 549 mg (0,96 mMol) des tert.-Butylesters (Verfahrensbeispiel 43) in 5,8 ml absol. Ethanol wurde in einem Eisbad gekühlt und es wurden mit einer Spritze 1,94 ml (1,94 mMol, 2,02 Äquiv.) 1 N-Natronlauge zugefügt. Im Gegensatz zu Beispiel 8 blieb das Reaktionsgemisch eine Suspension. Trotzdem zeigte DC nach 3 Std. eine praktisch quantitative Umwandlung an. Aufarbeitung wie in Beispiel 8 gab 519 mg eines farblosen Feststoffs, der sich bei 239-240 °C unter Braunfärbung zersetzte und schmolz. Dieses Material enthielt 1 Mol-Äquivalent Natriumacetat. Seine Bruttoformel betrug somit $C_{29}H_{39}FO_8Na_2$ - (MG 580,60). Die Ausbeute an Titelverbindung betrug 93,6 %.

**Beispiel 10**

**4(R)-Hydroxy-6(S)-[(2-p-fluorphenyl-4-p-fluorphenylthio-6-isopropyl)-phenoxymethyl]-3,4,5,6-tetrahydro-2H-pyran-2-on (Formel I)**

Zur Lösung von 5,59 g (10,0 mMol) des tert.-Butylesters (Verfahrensbeispiel 28) in 20 ml Methylenchlorid wurden 5 ml Trifluoressigsäure zugetropft. Das Reaktionsgemisch wurde 2 Std. bei Raumtemperatur

gerührt. DC (Cyclohexan/Ethylacetat 1:1) zeigte quantitative Umwandlung des tert.-Butylesters ($R_f = 0,37$) zum Lakton ($R_f = 0,12$) und geringfügigen unpolareren Verunreinigungen an. Das Reaktionsgemisch wurde mit Natriumhydrogencarbonat-Pulver abgestumpft, dann mit Natriumcarbonat-Pulver neutral gestellt, anschließend in Wasser geschüttet und mehrmals ausgeethert. Die vereinigten organischen Phasen wurden mit Natriumhydrogencarbonat-Lösung, dann mit Kochsalzlösung gewaschen, getrocknet, filtriert und eingeengt. Der Rückstand wurde mit Cyclohexan/Ethylacetat 1:1 durch eine Kieselgelsäule chromatographiert und ergab 3,88 g (8,0 mMol, Ausbeute 80 %) eines farblosen Feststoffs (Titelverbindung), Schmp. 108-110 °C.

NMR (270 MHz): $\delta$ = 1,30 + 1,32 (2xd, 6H, $CH(CH_3)_2$), 1,72-1,94 (m, 3H, $CH_2$ und OH), 2,67 (AB-Teil von ABX-System, 2H, $CH_2CO_2$), 3,47 (sept., 1H, $CH(CH_3)_2$), 3,59 (AB-Teil von ABX-System, 2H, $OCH_2$), 4,40 (m, 1H, CH-OH), 4,72 (m, 1H, CH-OCO), 6,80-7,55 (m, 10H, arom.-H).

MS (DCI, Isobutan): m/e = 484 ($M^+$), 467 ($M^+$-OH),

129 ( $M^+$-
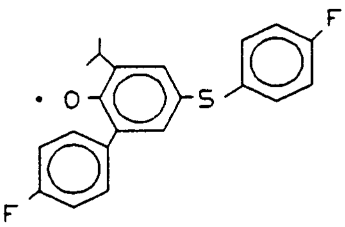
)

## Beispiel 11

**3(R),5(S)-Dihydroxy-6-[(2-p-fluorphenyl-4-p-fluorphenylthio-6-isopropyl)-phenoxy]-hexansäure-Natriumsalz (Formel II/2)**

Zur Lösung von 485 mg (1,0 mMol) des Laktons (Beispiel 10) in 10 ml Ethanol gab man unter Eiskühlung 1,0 ml (1,0 mMol) 1 N-Natronlauge und rührte 2 Std. bei 0 °C. Die Lösungsmittel wurden im Vakuum entfernt. Der Rückstand wurde zweimal in Toluol aufgenommen und jeweils im Vakuum zur Trockne eingeengt. Der Rückstand wurde mit n-Pentan gewaschen und im Hochvakuum zur Gewichtskonstanz getrocknet. Man erhielt 512 mg (0,98 mMol), 97,6 % Ausbeute) eines Feststoffs (Titelverbindung), der mit dem aus Beispiel 1 identisch ist.

## Beispiel 12

**4(R)-Hydroxy-6(S)-[(2-p-fluorphenyl-4-p-fluorphenylthio-6-isopropyl)-phenoxymethyl]-3,4,5,6-tetrahydro-2H-pyran-2-on (Formel I)**

1,05 g (2,0 mMol) des Natriumcarboxylats (Beispiel 1) wurden in 32 ml dest. Wasser weitgehend gelöst. Unter Eiskühlung wurden 2 ml 2 N-Salzsäure (4,0 mMol, 2 Äquivalente) zugegeben. Die kristallin ausgefallene Carbonsäure wurde mit Essigester (2x20 ml) extrahiert. Die Extrakte wurden zweimal mit gesättigter Kochsalzlösung gewaschen, kurz getrocknet, filtriert, im Vakuum eingeengt und der Rückstand wurde im Hochvakuum getrocknet. Ausbeute 1,00 g (1,99 mMol) farbloser Feststoff. Diese freie Carbonsäure wurde in 10 ml abs. THF gelöst und bei 0-10 °C wurde 302 µl (221,5 mg, 2,19 mMol, 1,1 Äquivalente) Triethylamin schnell zugetropft, 10 Min. bei 0 °C gerührt, dann auf -10 °C abgekühlt und 200 µl (226,8 mg, 2,09 mMol, 1,05 Äquivalente) Chlorameisensäureethylester langsam zugetropft. Das Reaktionsgemisch wurde 1 Std. bei -5 °C gerührt, zwischen Ether und halbgesättigter Kochsalzlösung verteilt und die Phasen wurden getrennt. Die wäßrige Phase wurde noch zweimal mit Ether extrahiert und die vereinigten Extrakte wurden nochmals mit Kochsalzlösung gewaschen.

Die Extrakte wurden getrocknet, filtriert, im Vakuum eingeengt und mit Cyclohexan/Ethylacetat 1:1 durch eine Kieselgelsäule chromatographiert.

Erhalten wurden 820 mg (1,69 mMol, 85 % Ausbeute) eines farblosen Feststoffs (Titelverbindung), der identisch mit dem aus Beispiel 10 war.

**Patentansprüche**

**Patentansprüche für folgende Vertragsstaaten : AT, BE, CH, DE, DK, FR, GB, IT, LI, LU, NL, SE**

**1.** 4-Hydroxytetrahydropyran-2-one der Formel I,

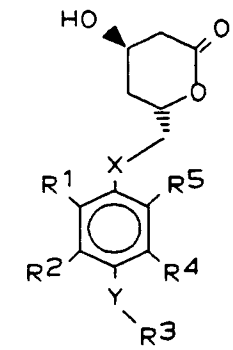

I

in der

| | |
|---|---|
| X und Y | gleich oder verschieden sind und ein Sauerstoffatom oder ein Schwefelatom bedeuten, |
| $R^1$ und $R^5$ | beide Isopropyl bedeuten oder verschieden sind und einen Isopropyl-, Cyclopropyl- oder Phenylrest bedeuten, wobei letzterer im Kern 1- bis 3-fach substituiert sein kann mit Fluor, Chlor, Brom, Trifluormethyl und/oder Alkyl oder Alkoxy mit je 1 bis 4 Kohlenstoffatomen. |
| $R^2$ und $R^4$ | gleich oder verschieden sind und Wasserstoff oder einen Isopropyl-, Cyclopropyl- oder Phenylrest bedeuten, wobei letzterer im Kern 1-3-fach substituiert sein kann mit Fluor, Chlor, Brom, Trifluormethyl und/oder Alkyl oder Alkoxy mit 1 bis 4 Kohlenstoffatomen, |
| $R^3$ | |

a) Wasserstoff, Methyl oder Ethyl

b) einen geradkettigen oder verzweigten Alkylrest mit 3 bis 8 Kohlenstoffatomen, der substituiert sein kann mit dem Rest der Formel

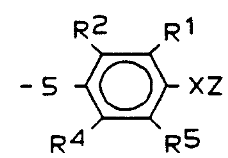

worin X, $R^1$, $R^2$, $R^4$, $R^5$ die oben angegebenen Bedeutungen haben und Z entweder ein Wasserstoffatom, ein pharmakologisch verträgliches Kation oder den 4(R)-Hydroxy-6(S)-methylen-3,4,5,6-tetrahydro-2H-pyran-2-on-Rest der Formel

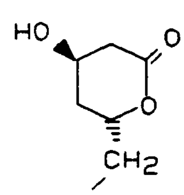

oder den entsprechenden 3(R),5(S)-Dihydroxyhexansäure-6-yl-Rest der Formel

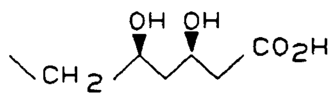

,

dessen pharmakologisch verträgliche Salze mit Basen oder dessen pharmakologisch verträgliche Ester bedeutet,

c) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 Kohlenstoffatomen, oder

d) Acetyl unter der Voraussetzung, daß Y Sauerstoff ist, bedeutet,

sowie die entsprechenden offenkettigen Dihydroxycarbonsäuren der Formel II

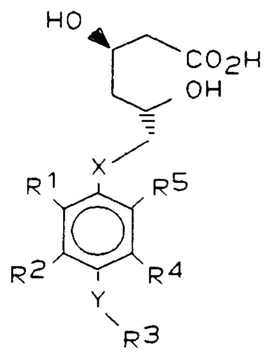

,

II

worin $R^1$, $R^2$, $R^3$, $R^4$, $R^5$, X und Y die angegebenen Bedeutungen haben, deren pharmakologisch verträgliche Salze mit Basen und deren pharmakologisch verträgliche Ester.

**2.** Verbindungen gemäß Anspruch 1, dadurch gekennzeichnet, daß in den Formeln I bzw. II

| | |
|---|---|
| X | Sauerstoff |
| Y | Sauerstoff oder Schwefel |
| $R^1$ | Isopropyl oder Cyclopropyl |
| $R^2$ | Wasserstoff, Isopropyl oder p-Fluorphenyl |
| $R^3$ | Wasserstoff, Acetyl, Isopropyl, p-Fluorphenyl oder einen der Reste |

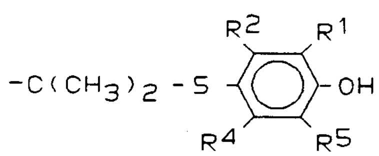

,

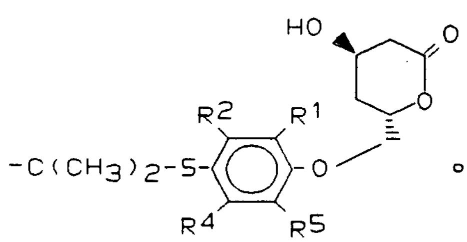

oder

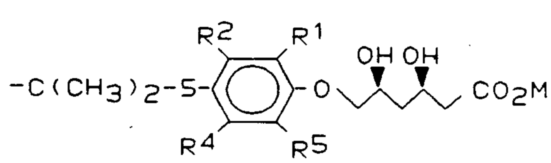

(M = Wasserstoff oder Natrium)

$R^4$ Wasserstoff, Isopropyl oder p-Fluorphenyl
$R^5$ Isopropyl oder p-Fluorphenyl
bedeuten.

**3.** Verbindungen gemäß Anspruch 1, dadurch gekennzeichnet, daß sie eine der folgenden Formeln (I)

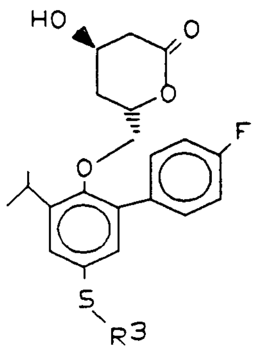

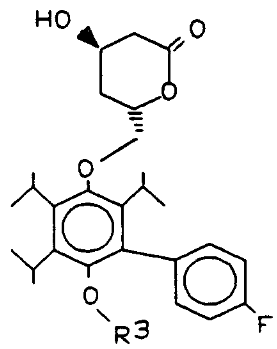

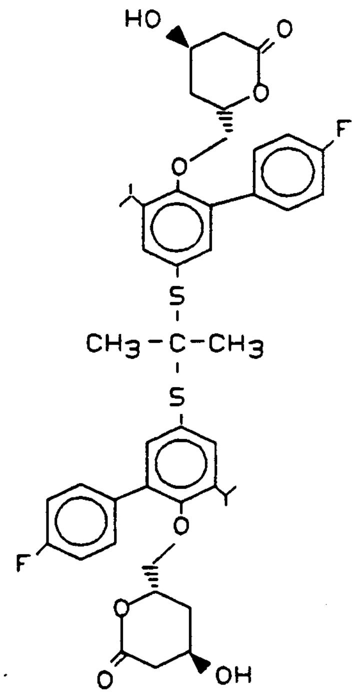

R3 = p-Fluorphenyl, p-Fluor-m-methyl-phenyl oder Isopropyl

R3 = Wasserstoff oder Acetyl

aufweisen, sowie die entsprechenden offenkettigen Dihydroxycarbonsäuren der allgemeinen Formel II, deren pharmakologisch verträgliche Salze mit Basen und deren pharmakologisch verträgliche Ester.

**4.** Verfahren zur Herstellung von Verbindungen gemäß Anspruch 1, dadurch gekennzeichnet, daß man

a) entsprechend substituierte Phenole oder Thiophenole der Formel III,

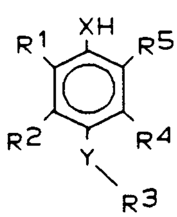

III

worin X, Y, $R^1$, $R^2$, $R^3$, $R^4$ und $R^5$ die zur Formel I angegebenen Bedeutungen haben, mit dem optisch reinen Mesylat der Formel IV

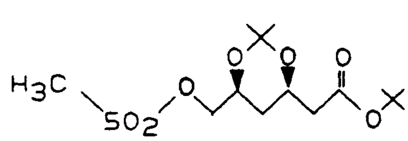

IV

zum Acetonid der Formel V

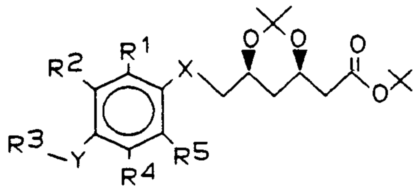

V

umsetzt,

b) Verbindungen der Formel V unter Abspaltung der Schutzgruppe in $\beta,\delta$-Dihydroxycarbonsäuretert.-butylester der Formel II/1,

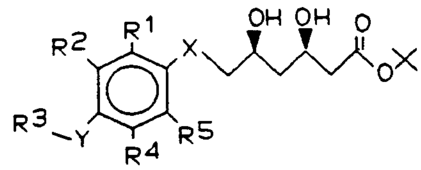

II / 1

worin X, Y, $R^1$, $R^2$, $R^3$, $R^4$ und $R^5$ die zur Formel I angegebenen Bedeutungen haben, überführt

c) die tert.-Butylester der Formel II/1 zu Salzen der Formel

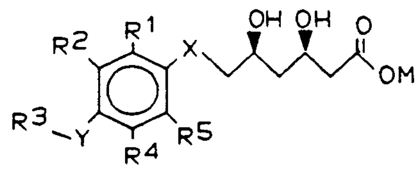

II / 2

II/2, in denen X, Y, $R^1$, $R^2$, $R^3$, $R^4$ und $R^5$ die zur Formel I angegebenen Bedeutungen haben und M ein pharmakologisch verträgliches Kation bedeutet, verseift, wobei gegebenenfalls vorhandene Schutzgruppen ebenfalls abgespaltet werden können,

d) die tert.-Butylester der Formel II/1 oder gegebenenfalls die Salze der Formel II/2 zu den $\beta$-Hydroxylaktonen der Formel I cyclisiert.

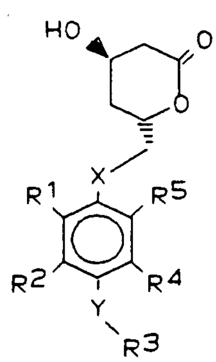

I

e) gegebenenfalls die Hydroxylaktone der Formel I in die entsprechenden offenkettigen Dihydroxycarbonsäuren der Formel II, deren Salze oder deren Ester überführt, gegebenenfalls die Salze oder

Ester in die freien Dihydroxycarbonsäuren der Formel II oder gegebenenfalls die Dihydroxycarbonsäuren II in die Salze oder Ester überführt.

5. Pharmazeutisches Präparat, dadurch gekennzeichnet, daß es eine Verbindung gemäß Anspruch 1 enthält.

6. Verbindungen gemäß Anspruch 1, zur Prophylaxe und Therapie der Arteriosklerose und Hypercholesterinämie.

7. Verbindungen der Formel III

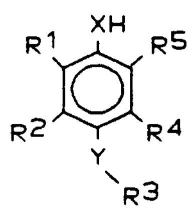

III

worin $R^1$, $R^2$, $R^3$, $R^4$, $R^5$, X und Y die in Anspruch 1 zu Formel I angegebenen Bedeutungen haben.

**Patentansprüche für folgenden Vertragsstaat: ES**

1. Verfahren zur Herstellung von 4-Hydroxytetrahydropyran-2-onen der Formel I

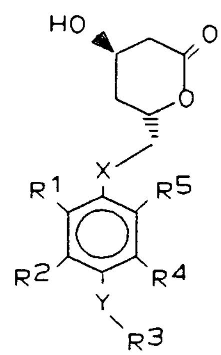

I

in der

| | |
|---|---|
| X und Y | gleich oder verschieden sind und ein Sauerstoffatom oder ein Schwefelatom bedeuten, |
| $R^1$ und $R^5$ | beide Isopropyl bedeuten oder verschieden sind und einen Isopropyl-, Cyclopropyl- oder Phenylrest bedeuten, wobei letzterer im Kern 1- bis 3-fach substituiert sein kann mit Fluor, Chlor, Brom, Trifluormethyl und/oder Alkyl oder Alkoxy mit je 1 bis 4 Kohlenstoffatomen. |
| $R^2$ und $R^4$ | gleich oder verschieden sind und Wasserstoff oder einen Isopropyl-, Cyclopropyl- oder Phenylrest bedeuten, wobei letzterer im Kern 1-3-fach substituiert sein kann mit Fluor, Chlor, Brom, Trifluormethyl und/oder Alkyl oder Alkoxy mit 1 bis 4 Kohlenstoffatomen, |
| $R^3$ | |

a) Wasserstoff, Methyl oder Ethyl

b) einen geradkettigen oder verzweigten Alkylrest mit 3 bis 8 Kohlenstoffatomen, der substituiert sein kann mit dem Rest der Formel

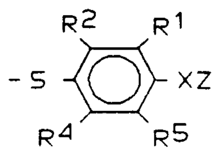

worin X, $R^1$, $R^2$, $R^4$, $R^5$ die oben angegebenen Bedeutungen haben und Z entweder ein Wasserstoffatom, ein pharmakologisch verträgliches Kation oder den 4(R)-Hydroxy-6(S)-methylen-3,4,5,6-tetrahydro-2H-pyran-2-on-Rest der Formel

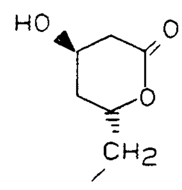

oder den entsprechenden 3(R),5(S)-Dihydroxyhexansäure-6-yl-Rest der Formel

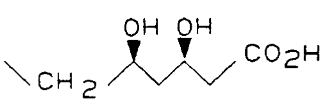 ,

dessen pharmakologisch verträgliche Salze mit Basen oder dessen pharmakologisch verträgliche Ester bedeutet,
c) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 Kohlenstoffatomen, oder
d) Acetyl unter der Voraussetzung, daß Y Sauerstoff ist, bedeutet,

von den entsprechenden offenkettigen Dihydroxycarbonsäuren der Formel II

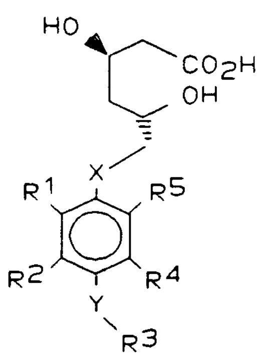 ,

II

worin $R^1$, $R^2$, $R^3$, $R^4$, $R^5$, X und Y die angegebenen Bedeutungen haben,
deren pharmakologisch verträglichen Salzen mit Basen und deren pharmakologisch verträglichen Estern, dadurch gekennzeichnet, daß man

a) ensprechend substituierte Phenole oder Thiophenole der Formel III,

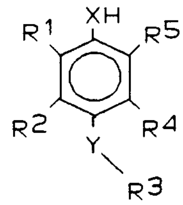

III

worin X, Y, $R^1$, $R^2$, $R^3$, $R^4$ und $R^5$ die zur Formel I angegebenen Bedeutungen haben, mit dem optisch reinen Mesylat der Formel IV

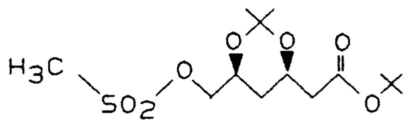

IV

zum Acetonid der Formel V

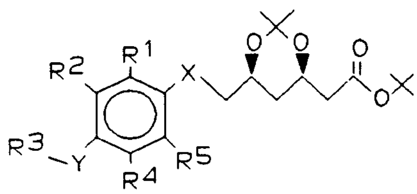

V

umsetzt,

b) Verbindungen der Formel V unter Abspaltung der Schutzgruppe in $\beta,\delta$-Dihydroxycarbonsäuretert.-butylester der Formel II/1,

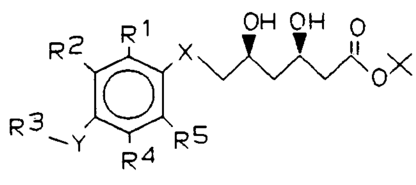

II / 1

worin X, Y, $R^1$, $R^2$, $R^3$, $R^4$ und $R^5$ die zur Formel I angegebenen Bedeutungen haben, überführt

c) die tert.-Butylester der Formel II/1 zu Salzen der Formel

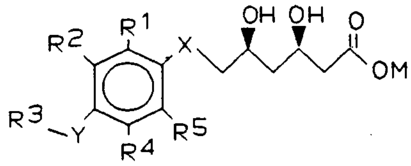

II/2

II/2, in denen X, Y, $R^1$, $R^2$, $R^3$, $R^4$ und $R^5$ die zur Formel I angegebenen Bedeutungen haben und M ein pharmakologisch verträgliches Kation bedeutet, verseift, wobei gegebenenfalls vorhandene Schutzgruppen ebenfalls abgespaltet werden können,
d) die tert.-Butylester der Formel II/1 oder gegebenenfalls die Salze der Formel II/2 zu den β-Hydroxylaktonen der Formel I cyclisiert.

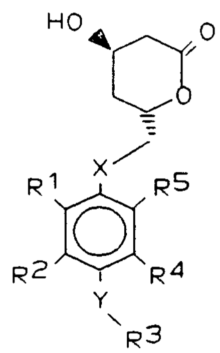

I

e) gegebenenfalls die Hydroxylaktone der Formel I in die entsprechenden offenkettigen Dihydroxycarbonsäuren der Formel II, deren Salze oder deren Ester überführt, gegebenenfalls die Salze oder Ester in die freien Dihydroxycarbonsäuren der Formel II oder gegebenenfalls die Dihydroxycarbonsäuren II in die Salze oder Ester überführt.

**2.** Verfahren gemäß Anspruch 1, dadurch gekennzeichnet, daß man Verbindungen der Formel I bzw. II, worin

X Sauerstoff
Y Sauerstoff oder Schwefel
$R^1$ Isopropyl oder Cyclopropyl
$R^2$ Wasserstoff, Isopropyl oder p-Fluorphenyl
$R^3$ Wasserstoff, Acetyl, Isopropyl, p-Fluorphenyl oder einen der Reste

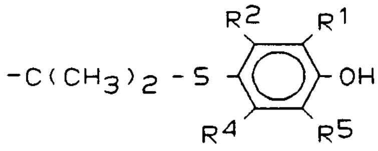,

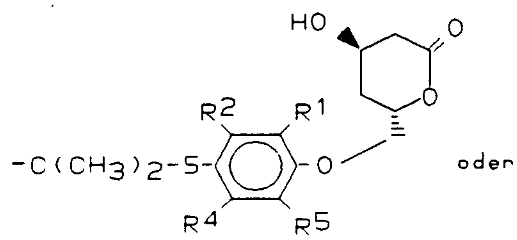

oder

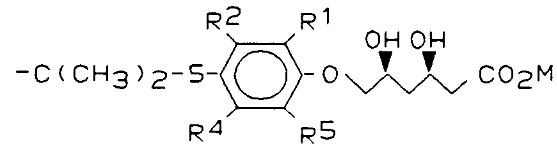

(M = Wasserstoff oder Natrium)

$R^4$ Wasserstoff, Isopropyl oder p-Fluorphenyl

$R^5$ Isopropyl oder p-Fluorphenyl

bedeuten, deren pharmakologisch verträgliche Salze mit Basen oder deren pharmakologisch verträglichen Ester herstellt.

3. Verfahren gemäß Anspruch 1, dadurch gekennzeichnet, daß man eine Verbindung der folgenden Formeln (I)

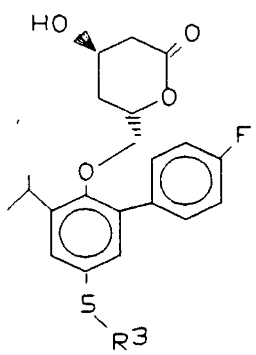

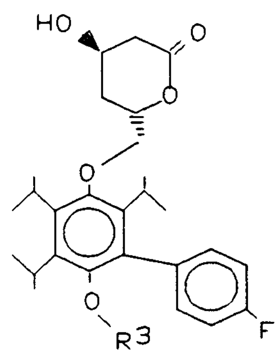

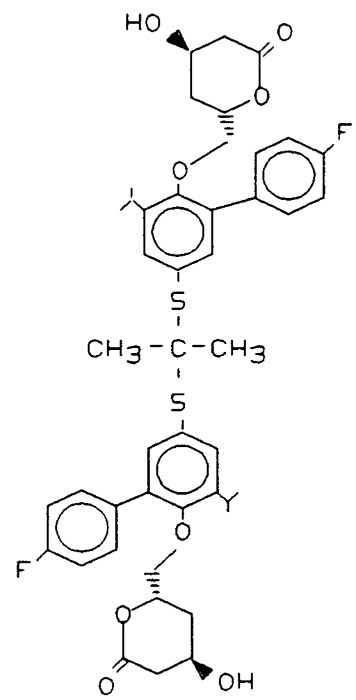

R3 = p-Fluorphenyl, p-Fluor-m-methyl-phenyl oder Isopropyl

R3 = Wasserstoff oder Acetyl

die entsprechenden offenkettigen Dihydroxycarbonsäuren der allgemeinen Formel II, deren pharmakologisch verträgliche Salze mit Basen oder deren pharmakologisch verträgliche Ester herstellt.

4. Verfahren zur Herstellung eines pharmazeutischen Präparates, dadurch gekennzeichnet, daß man eine Verbindung erhältlich gemäß Anspruch 1 in eine geeignete Darreichungsform überführt.

**5.** Verfahren gemäß Anspruch 1, dadurch gekennzeichnet, daß man Verbindungen der Formel III

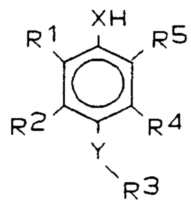

III

worin $R^1$, $R^2$, $R^3$, $R^4$, $R^5$, X und Y die in Anspruch 1 zu Formel I angegebenen Bedeutungen haben, isoliert.

## Patentansprüche für folgenden Vertragsstaat: GR

**1.** Verfahren zur Herstellung von 4-Hydroxytetrahydropyran-2-onen der Formel I

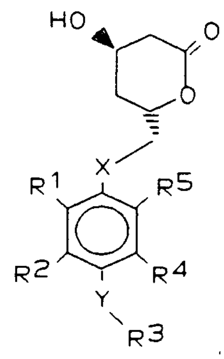

I

in der

X und Y gleich oder verschieden sind und ein Sauerstoffatom oder ein Schwefelatom bedeuten,

$R^1$ und $R^5$ beide Isopropyl bedeuten oder verschieden sind und einen Isopropyl-, Cyclopropyl- oder Phenylrest bedeuten, wobei letzterer im Kern 1- bis 3-fach substituiert sein kann mit Fluor, Chlor, Brom, Trifluormethyl und/oder Alkyl oder Alkoxy mit je 1 bis 4 Kohlenstoffatomen.

$R^2$ und $R^4$ gleich oder verschieden sind und Wasserstoff oder einen Isopropyl-, Cyclopropyl- oder Phenylrest bedeuten, wobei letzterer im Kern 1-3-fach substituiert sein kann mit Fluor, Chlor, Brom, Trifluormethyl und/oder Alkyl oder Alkoxy mit 1 bis 4 Kohlenstoffatomen,

$R^3$

a) Wasserstoff, Methyl oder Ethyl

b) einen geradkettigen oder verzweigten Alkylrest mit 3 bis 8 Kohlenstoffatomen, der substituiert sein kann mit dem Rest der Formel

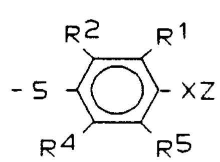

worin X, $R^1$, $R^2$, $R^4$, $R^5$ die oben angegebenen Bedeutungen haben und Z entweder ein Wasserstoffatom, ein pharmakologisch verträgliches Kation oder den 4(R)-Hydroxy-6(S)-methylen-3,4,5,6-tetrahydro-2H-pyran-2-on-Rest der Formel

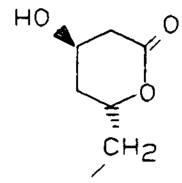

oder den entsprechenden 3(R),5(S)-Dihydroxyhexansäure-6-yl-Rest der Formel

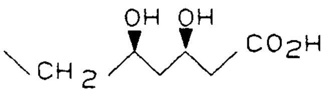

,

dessen pharmakologisch verträgliche Salze mit Basen oder dessen pharmakologisch verträgliche Ester bedeutet,

c) Cycloalkyl mit 3 bis 8 Kohlenstoffatomen oder einen Phenylrest, der im Kern 1- bis 3-fach substituiert sein kann mit Halogen, Trifluormethyl und/oder Alkyl oder Alkoxy mit je 1 bis 4 Kohlenstoffatomen, oder

d) Acetyl unter der Voraussetzung, daß Y Sauerstoff ist, bedeutet,

von den entsprechenden offenkettigen Dihydroxycarbonsäuren der Formel II

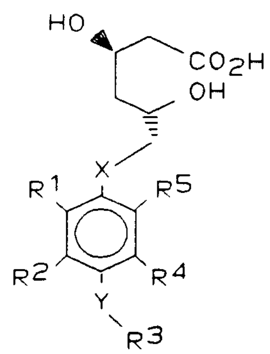

,

worin $R^1$, $R^2$, $R^3$, $R^4$, $R^5$, X und Y die angegebenen Bedeutungen haben,
deren pharmakologisch verträglichen Salzen mit Basen und deren pharmakologisch verträgliche Estern, dadurch gekennzeichnet, daß man

a) ensprechend substituierte Phenole oder Thiophenole der Formel III,

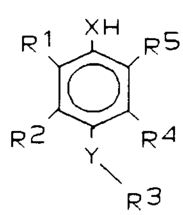

III

worin X, Y, $R^1$, $R^2$, $R^3$, $R^4$ und $R^5$ die zur Formel I angegebenen Bedeutungen haben, mit dem optisch reinen Mesylat der Formel IV

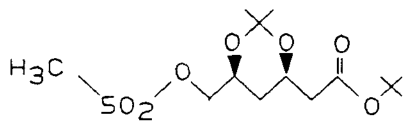

IV

zum Acetonid der Formel V

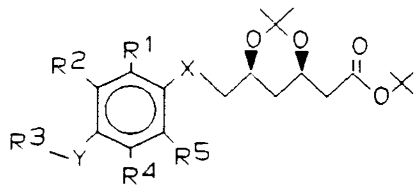

V

umsetzt,
b) Verbindungen der Formel V unter Abspaltung der Schutzgruppe in $\beta$,$\delta$-Dihydroxycarbonsäuretert.-butylester der Formel II/1,

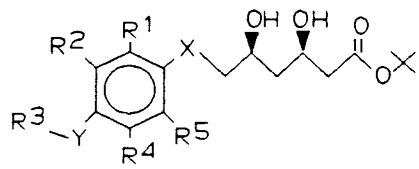

II / 1

worin X, Y, $R^1$, $R^2$, $R^3$, $R^4$ und $R^5$ die zur Formel I angegebenen Bedeutungen haben, überführt
c) die tert.-Butylester der Formel II/1 zu Salzen der Formel

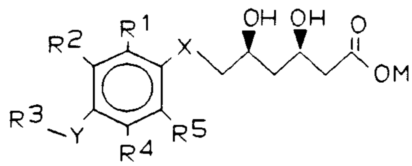

II / 2

II/2, in denen X, Y, $R^1$, $R^2$, $R^3$, $R^4$ und $R^5$ die zur Formel I angegebenen Bedeutungen haben und M ein pharmakologisch verträgliches Kation bedeutet, verseift, wobei gegebenenfalls vorhandene Schutzgruppen ebenfalls abgespaltet werden können,
d) die tert.-Butylester der Formel II/1 oder gegebenenfalls die Salze der Formel II/2 zu den $\beta$-Hydroxylaktonen der Formel I cyclisiert.

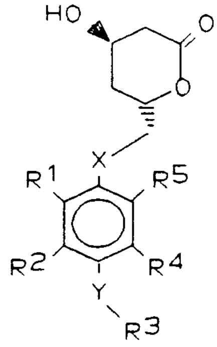

I

e) gegebenenfalls die Hydroxylaktone der Formel I in die entsprechenden offenkettigen Dihydroxycarbonsäuren der Formel II, deren Salze oder deren Ester überführt, gegebenenfalls die Salze oder Ester in die freien Dihydroxycarbonsäuren der Formel II oder gegebenenfalls die Dihydroxycarbonsäuren II in die Salze oder Ester überführt.

**2.** Verfahren gemäß Anspruch 1, dadurch gekennzeichnet, daß man Verbindungen der Formel I bzw. II, worin

X Sauerstoff
Y Sauerstoff oder Schwefel
$R^1$ Isopropyl oder Cyclopropyl
$R^2$ Wasserstoff, Isopropyl oder p-Fluorphenyl
$R^3$ Wasserstoff, Acetyl, Isopropyl, p-Fluorphenyl oder einen der Reste

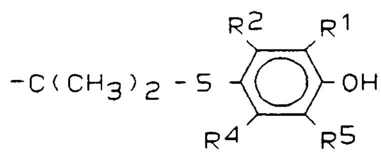
,

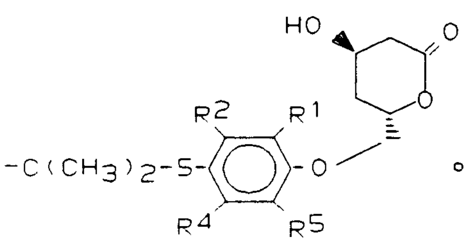
oder
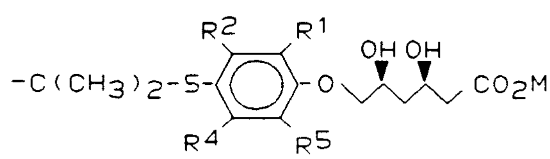

(M = Wasserstoff oder Natrium)

$R^4$ Wasserstoff, Isopropyl oder p-Fluorphenyl
$R^5$ Isopropyl oder p-Fluorphenyl
bedeuten, deren pharmakologisch verträgliche Salze mit Basen oder deren pharmakologisch verträglichen Ester herstellt.

**3.** Verfahren gemäß Anspruch 1, dadurch gekennzeichnet, daß man eine Verbindung der folgenden Formeln (I)

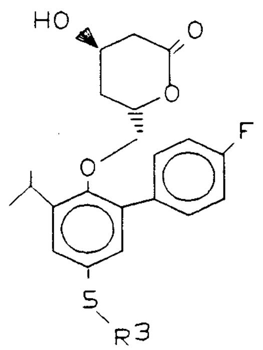

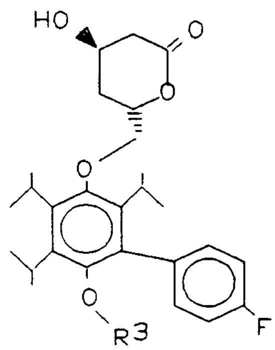

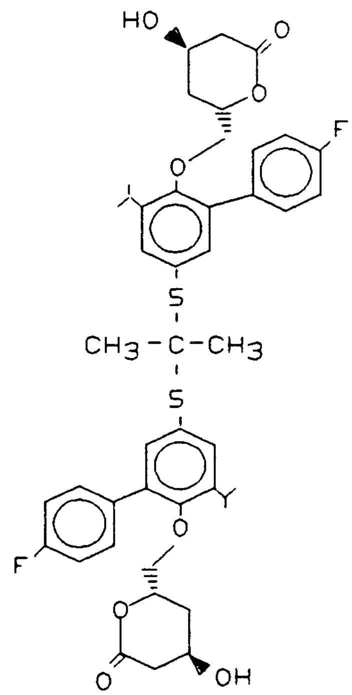

R3 = p-Fluorphenyl, p-Fluor-m-methyl-phenyl oder Isopropyl

R3 = Wasserstoff oder Acetyl

die entsprechenden offenkettigen Dihydroxycarbonsäuren der allgemeinen Formel II, deren pharmakologisch verträgliche Salze mit Basen oder deren pharmakologisch verträgliche Ester herstellt.

**4.** Verfahren zur Herstellung eines pharmazeutischen Präparates, dadurch gekennzeichnet, daß man eine Verbindung erhältlich gemäß Anspruch 1 in eine geeignete Darreichungsform überführt.

**5.** Verbindungen der Formel III

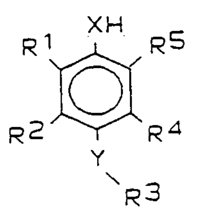

III ,

worin $R^1$, $R^2$, $R^3$, $R^4$, $R^5$, X und Y die in Anspruch 1 zu Formel I angegebenen Bedeutungen haben.

## Claims

**Claims for the following Contracting States : AT, BE, CH, DE, DK, FR, GB, IT, LI, LU, NL, SE**

**1.** A 4-hydroxytetrahydropyran-2-one of the formula I,

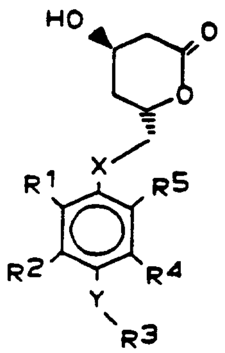

I

in which

| | |
|---|---|
| X and Y | are identical or different and are an oxygen atom or a sulfur atom, |
| $R^1$ and $R^5$ | are both isopropyl or are different and are an isopropyl, cyclopropyl or phenyl radical, it being possible for the latter to be monosubstituted to trisubstituted in the nucleus by fluorine, chlorine, bromine, trifluoromethyl and/or alkyl or alkoxy each having 1 to 4 carbon atoms, |
| $R^2$ and $R^4$ | are identical or different and are hydrogen or an isopropyl, cyclopropyl or phenyl radical, it being possible for the latter to be monosubstituted to trisubstituted in the nucleus by fluorine, chlorine, bromine, trifluoromethyl and/or alkyl or alkoxy having 1 to 4 carbon atoms, |
| $R^3$ | is |

a) hydrogen, methyl or ethyl

b) a straight-chain or branched alkyl radical having 3 to 8 carbon atoms, which can be substituted by the radical of the formula

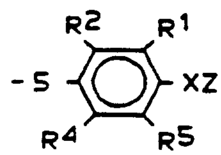

in which X, $R^1$, $R^2$, $R^4$ and $R^5$ have the abovementioned meanings and Z is either a hydrogen atom, a pharmacologically tolerable cation or the 4(R)-hydroxy-6-(S)-methylene-3,4,5,6-tetrahydro-2H-pyran-2-one radical of the formula

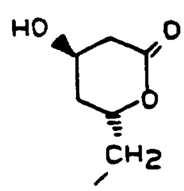

or the corresponding 3(R),5(S)-dihydroxyhexanoic acid-6-yl radical of the formula

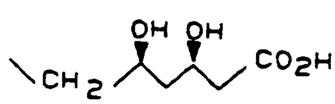

its pharmacologically tolerable salts with bases or its pharmacologically tolerable

esters,

c) cycloalkyl having 3 to 8 carbon atoms or a phenyl radical which can be monosubstituted to trisubstituted in the nucleus by halogen, trifluoromethyl and/or alkyl or alkoxy each having 1 to 4 carbon atoms, or

d) acetyl with the condition that Y is oxygen,

or the corresponding open-chain dihydroxycarboxylic acid of the formula II

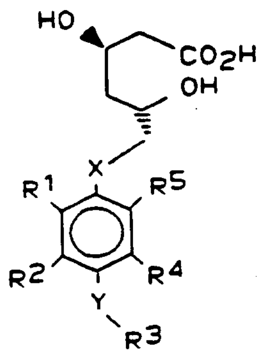

II,

in which $R^1$, $R^2$, $R^3$, $R^4$, $R^5$, X and Y have the meanings indicated,
their pharmacologically tolerable salts with bases and their pharmacologically tolerable esters.

**2.** A compound as claimed in claim 1, wherein in the formulae I or II

| | |
|---|---|
| X | is oxygen |
| Y | is oxygen or sulfur |
| $R^1$ | is isopropyl or cyclopropyl |
| $R^2$ | is hydrogen, isopropyl or p-fluorophenyl |
| $R^3$ | is hydrogen, acetyl, isopropyl, p-fluorophenyl, or one of the radicals |

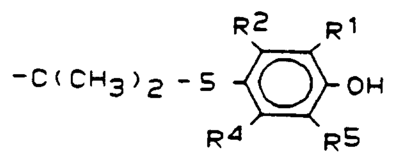
,

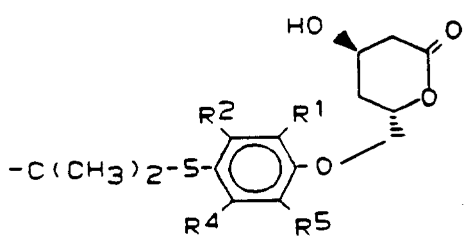
or
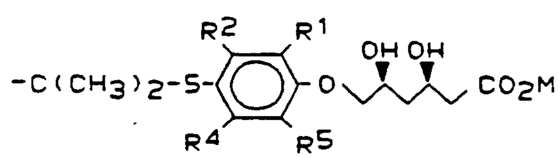

(M = hydrogen or sodium)

| | |
|---|---|
| $R^4$ | is hydrogen, isopropyl or p-fluorophenyl |
| $R^5$ | is isopropyl or p-fluorophenyl. |

**3.** A compound as claimed in claim 1, which has one of the following formulae (I)

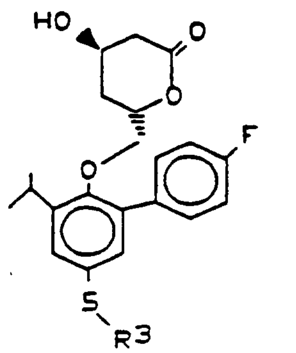

$R^3$ = p-fluorophenyl, p-fluoro-m-methylphenyl or isopropyl

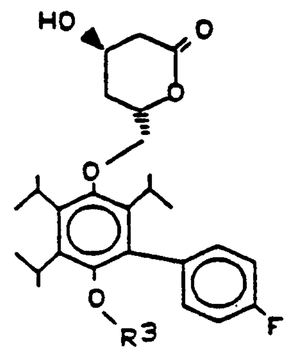

$R^3$ = hydrogen or acetyl

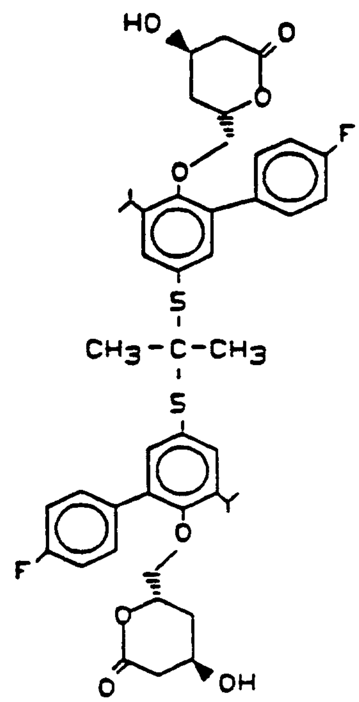

or the corresponding open-chain dihydroxycarboxylic acid of the formula II, their pharmacologically tolerable salts with bases and their pharmacologically tolerable esters.

**4.** A process for the preparation of a compound as claimed in claim 1, which comprises

a) reacting appropriately substituted phenols or thiophenols of the formula III

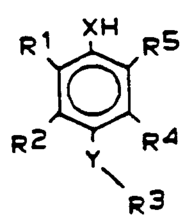

III

in which X, Y, $R^1$, $R^2$, $R^3$, $R^4$ and $R^5$ have the meanings indicated for formula I, with the optically pure mesylate of the formula IV

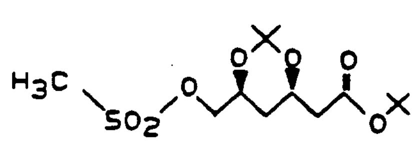

IV

to give the acetonide of the formula V

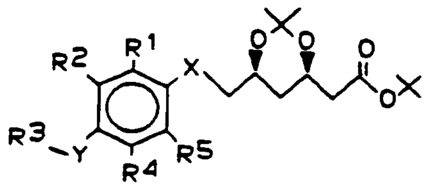

V,

b) converting compounds of the formula V with removal of the protective group into tert.-butyl $\beta,\delta$-dihydroxycarboxylates of the formula II/1

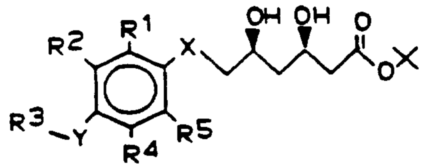

II/1

in which X, Y, $R^1$, $R^2$, $R^3$, $R^4$ and $R^5$ have the meanings indicated for formula I,
c) hydrolyzing the tert.-butyl esters of the formula II/1 to give salts of the formula II/2

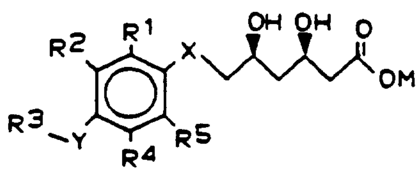

II/2

in which X, Y, $R^1$, $R^2$, $R^3$, $R^4$ and $R^5$ have the meanings indicated for formula I and M is a pharmacologically tolerable cation, it also being possible to remove protective groups which may be present,
d) cyclizing the tert.-butyl esters of the formula II/1 or, if appropriate, the salts of the formula II/2 to the $\beta$-hydroxylactones of the formula I

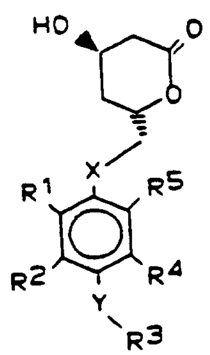

I

e) if appropriate converting the hydroxylactones of the formula I into the corresponding open-chain dihydroxycarboxylic acids of the formula II, their salts or their eaters, if desired converting the salts or eaters into the free dihydroxycarboxylic acids of the formula II or if desired converting the dihydroxycarboxylic acids II into the salts or eaters

**5.** A pharmaceutical preparation, which contains a compound as claimed in claim 1.

**6.** A compound as claimed in claim 1 for the prophylaxis and treatment of arteriosclerosis and hypercholesterolemia.

**7.** A compound of the formula III

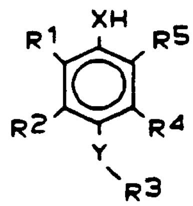

III

in which $R^1$, $R^2$, $R^3$, $R^4$, $R^5$, X and Y have the meanings indicated in claim 1 for formula I.

**Claims for the following Contracting State: ES**

**1.** A process for the preparation of 4-hydroxytetrahydropyran-2-ones of the formula I,

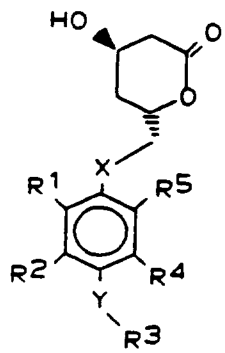

I

in which

X and Y are identical or different and are an oxygen atom or a sulfur atom,

$R^1$ and $R^5$ are both isopropyl or are different and are an isopropyl, cyclopropyl or phenyl radical, it being possible for the latter to be monosubstituted to trisubstituted in the nucleus by fluorine, chlorine, bromine, trifluoromethyl and/or alkyl or alkoxy each having 1 to 4 carbon atoms,

$R^2$ and $R^4$ are identical or different and are hydrogen or an isopropyl, cyclopropyl or phenyl radical, it being possible for the latter to be monosubstituted to trisubstituted in the nucleus by fluorine, chlorine, bromine, trifluoromethyl and/or alkyl or alkoxy having 1 to 4 carbon atoms,

$R^3$ is

a) hydrogen, methyl or ethyl

b) a straight-chain or branched alkyl radical having 3 to 8 carbon atoms, which can be substituted by the radical of the formula

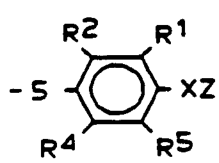

in which X, $R^1$, $R^2$, $R^4$ and $R^5$ have the abovementioned meanings and Z is either a hydrogen atom, a pharmacologically tolerable cation or the 4(R)-hydroxy-6-(S)-methylene-3,4,5,6-tetrahydro-2H-pyran-2-one radical of the formula

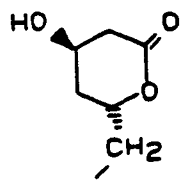

or the corresponding 3(R),5(S)-dihydroxyhexanoic acid-6-yl radical of the formula

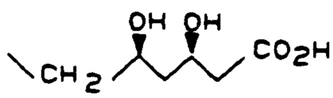

its pharmacologically tolerable salts with bases or its pharmacologically tolerable esters,

c) cycloalkyl having 3 to 8 carbon atoms or a phenyl radical which can be monosubstituted to trisubstituted in the nucleus by halogen, trifluoromethyl and/or alkyl or alkoxy each having 1 to 4 carbon atoms, or

d) acetyl with the condition that Y is oxygen,

or of the corresponding open-chain dihydroxycarboxylic acids of the formula II

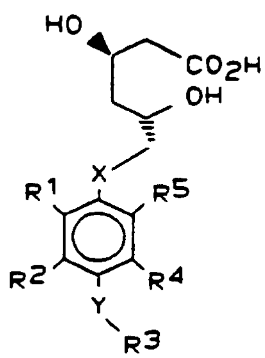

II,

in which $R^1$, $R^2$, $R^3$, $R^4$, $R^5$, X and Y have the meanings indicated,
their pharmacologically tolerable salts with bases and their pharmacologically tolerable esters, which comprises

a) reacting appropriately substituted phenols or thiophenols of the formula III

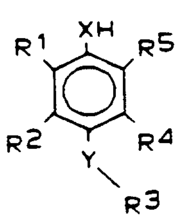

III

in which X, Y, $R^1$, $R^2$, $R^3$, $R^4$ and $R^5$ have the meanings indicated for formula I, with the optically pure mesylate of the formula IV

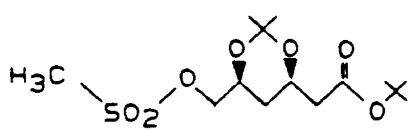

IV

to give the acetonide of the formula V

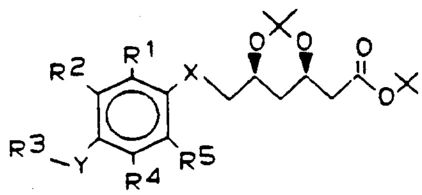

V,

b) converting compounds of the formula V with removal of the protective group into tert.-butyl $\beta,\delta$-dihydroxycarboxylates of the formula II/1

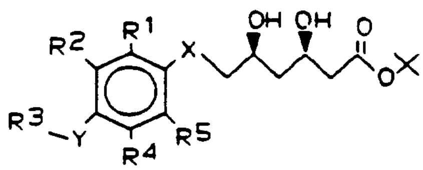

II/1

in which X, Y, $R^1$, $R^2$, $R^3$, $R^4$ and $R^5$ have the meanings indicated for formula I,

c) hydrolyzing the tert.-butyl esters of the formula II/1 to give salts of the formula II/2

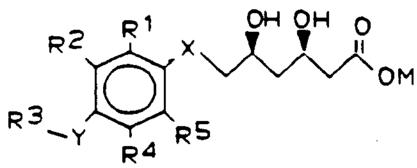

II/2

in which X, Y, $R^1$, $R^2$, $R^3$, $R^4$ and $R^5$ have the meanings indicated for formula I and M is a pharmacologically tolerable cation, it also being possible to remove protective groups which may be present,

d) cyclizing the tert.-butyl esters of the formula II/1 or, if appropriate, the salts of the formula II/2 to the $\beta$-hydroxylactones of the formula I

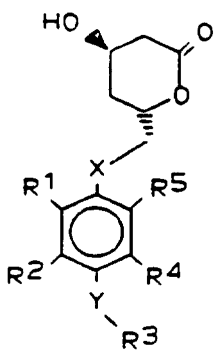

I

e) if appropriate converting the hydroxylactones of the formula I into the corresponding open-chain dihydroxycarboxylic acids of the formula II, their salts or their esters, if desired converting the salts or esters into the free dihydroxycarboxylic acids of the formula II or if desired converting the dihydroxycarboxylic acids II into the salts or esters.

**2.** The process as claimed in claim 1, wherein compounds of the formula I or II in which

X is oxygen

Y is oxygen or sulfur

$R^1$ is isopropyl or cyclopropyl
$R^2$ is hydrogen, isopropyl or p-fluorophenyl
$R^3$ is hydrogen, acetyl, isopropyl, p-fluorophenyl, or one of the radicals

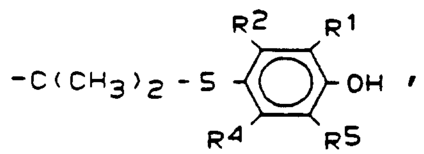

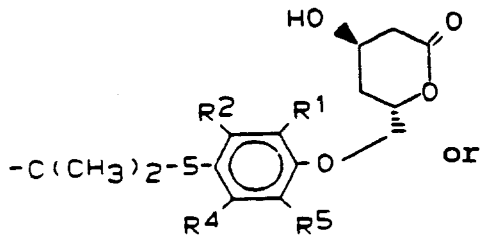

or

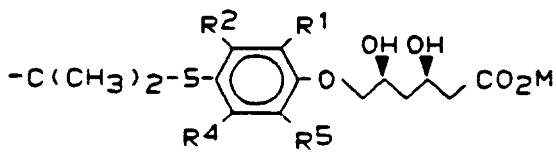

(M = hydrogen or sodium)

$R^4$ is hydrogen, isopropyl or p-fluorophenyl
$R^5$ is isopropyl or p-fluorophenyl,

their pharmacologically tolerable salts with bases or their pharmacologically tolerable esters are prepared.

**3.** The process as claimed in claim 1, wherein a compound of the following formulae (I)

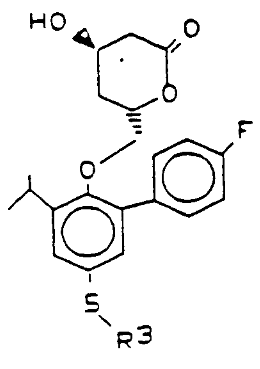

$R^3$ = p-fluorophenyl,
p-fluoro-m-methylphenyl
or
isopropyl

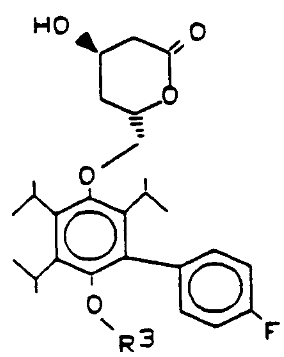

$R^3$ = hydrogen
or
acetyl

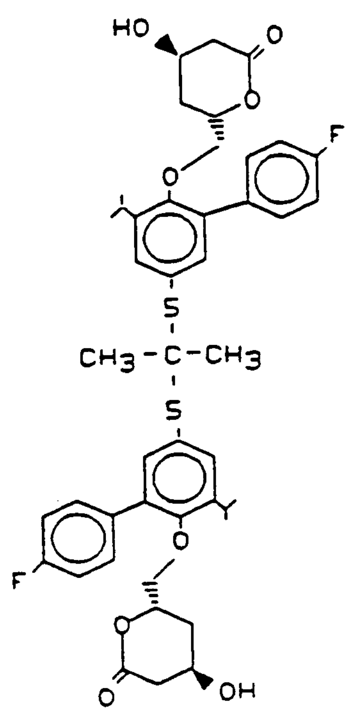

the corresponding open-chain dihydroxycarboxylic acids of the formula II, their pharmacologically tolerable salts with bases or their pharmacologically tolerable esters are prepared.

4. A process for the production of a pharmaceutical preparation, which comprises converting a compound obtainable as claimed in claim 1 into a suitable administration form.

5. The process as claimed in claim 1, wherein compounds of the formula III

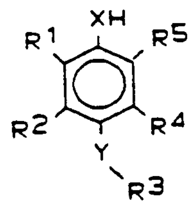

III

in which $R^1$, $R^2$, $R^3$, $R^4$, $R^5$, X and Y have the meanings indicated in claim 1 for formula I, are isolated.

**Claims for the following Contracting State : GR**

1. A process for the preparation of 4-hydroxytetrahydropyran-2-ones of the formula I,

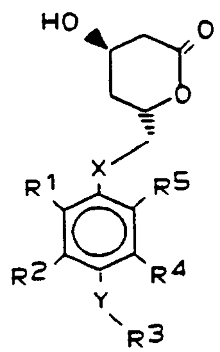

I

in which

X and Y — are identical or different and are an oxygen atom or a sulfur atom,

$R^1$ and $R^5$ — are both isopropyl or are different and are an isopropyl, cyclopropyl or phenyl radical, it being possible for the latter to be monosubstituted to trisubstituted in the nucleus by fluorine, chlorine, bromine, trifluoromethyl and/or alkyl or alkoxy each having 1 to 4 carbon atoms,

$R^2$ and $R^4$ — are identical or different and are hydrogen or an isopropyl, cyclopropyl or phenyl radical, it being possible for the latter to be monosubstituted to trisubstituted in the nucleus by fluorine, chlorine, bromine, trifluoromethyl and/or alkyl or alkoxy having 1 to 4 carbon atoms,

$R^3$ — is

a) hydrogen, methyl or ethyl

b) a straight-chain or branched alkyl radical having 3 to 8 carbon atoms, which can be substituted by the radical of the formula

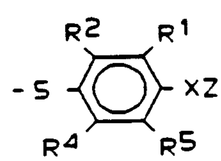

in which X, $R^1$, $R^2$, $R^4$ and $R^5$ have the abovementioned meanings and Z is either a hydrogen atom, a pharmacologically tolerable cation or the 4(R)-hydroxy-6-(S)-methylene-3,4,5,6-tetrahydro-2H-pyran-2-one radical of the formula

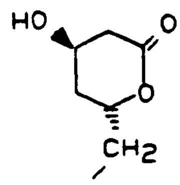

or the corresponding 3(R),5(S)-dihydroxyhexanoic acid-6-yl radical of the formula

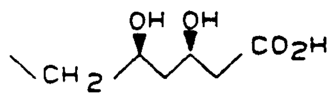

its pharmacologically tolerable salts with bases or its pharmacologically tolerable esters,

c) cycloalkyl having 3 to 8 carbon atoms or a phenyl radical which can be monosubstituted to trisubstituted in the nucleus by halogen, trifluoromethyl and/or alkyl or alkoxy each having 1 to 4 carbon atoms, or

d) acetyl with the condition that Y is oxygen,

or of the corresponding open-chain dihydroxycarboxylic acids of the formula II

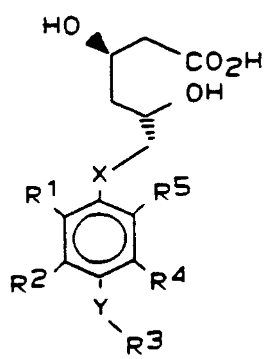

II,

in which $R^1$, $R^2$, $R^3$, $R^4$, $R^5$, X and Y have the meanings indicated, their pharmacologically tolerable salts with bases and their pharmacologically tolerable esters, which comprises

a) reacting appropriately substituted phenols or thiophenols of the formula III

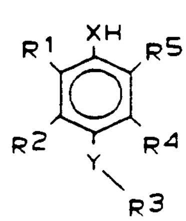

III

in which X, Y, $R^1$, $R^2$, $R^3$, $R^4$ and $R^5$ have the meanings indicated for formula I, with the optically pure mesylate of the formula IV

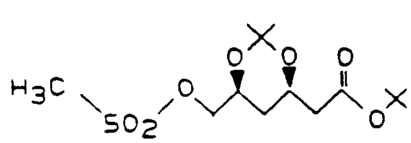

IV

to give the acetonide of the formula V

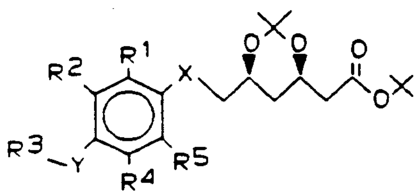

V,

b) converting compounds of the formula V with removal of the protective group into tert.-butyl $\beta,\delta$-dihydroxycarboxylates of the formula II/1

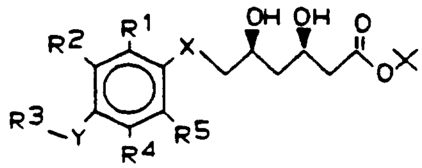

II/1

in which X, Y, $R^1$, $R^2$, $R^3$, $R^4$ and $R^5$ have the meanings indicated for formula I,
c) hydrolysing the tert.-butyl esters of the formula II/1 to give salts of the formula II/2

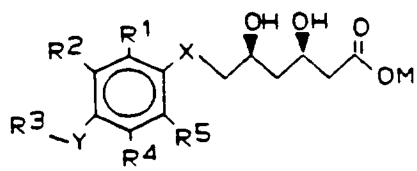

II/2

in which X, Y, $R^1$, $R^2$, $R^3$, $R^4$ and $R^5$ have the meanings indicated for formula I and M is a pharmacologically tolerable cation, it also being possible to remove protective groups which may be present,
d) cyclizing the tert.-butyl esters of the formula II/1 or, if appropriate, the salts of the formula II/2 to the $\beta$-hydroxylactones of the formula I

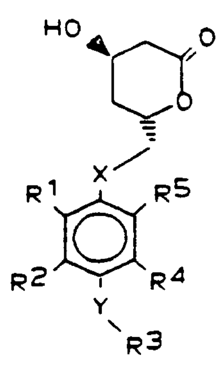

I

e) if appropriate converting the hydroxylactones of the formula I into the corresponding open-chain dihydroxycarboxylic acids of the formula II, their salts or their esters, if desired converting the salts or esters into the free dihydroxycarboxylic acids of the formula II or if desired converting the dihydroxycarboxylic acids II into the salts or esters.

**2.** The process as claimed in claim 1, wherein compounds of the formula I or II in which

X is oxygen
Y is oxygen or sulfur

$R^1$ is isopropyl or cyclopropyl
$R^2$ is hydrogen, isopropyl or p-fluorophenyl
$R^3$ is hydrogen, acetyl, isopropyl, p-fluorophenyl, or one of the radicals

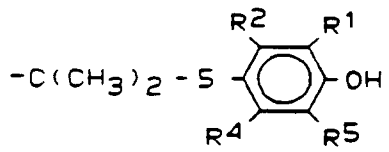
,

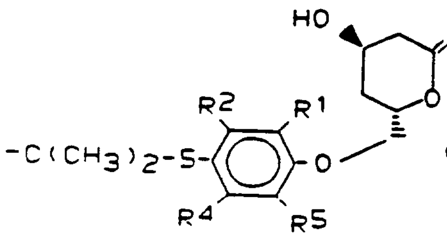
or
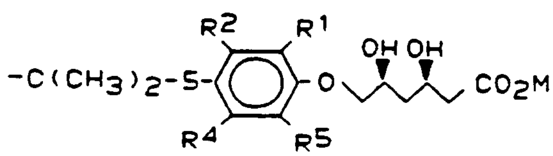

(M = hydrogen or sodium)

$R^4$ is hydrogen, isopropyl or p-fluorophenyl
$R^5$ is isopropyl or p-fluorophenyl,

their pharmacologically tolerable salts with bases or their pharmacologically tolerable esters are prepared.

**3.** The process as claimed in claim 1, wherein a compound of the following formulae (I)

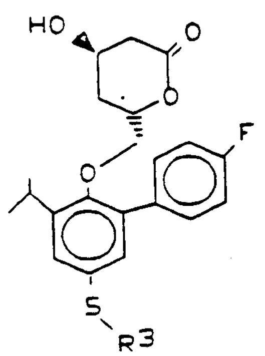

$R^3$ = p-fluorophenyl,
p-fluoro-m-methylphenyl
or
isopropyl

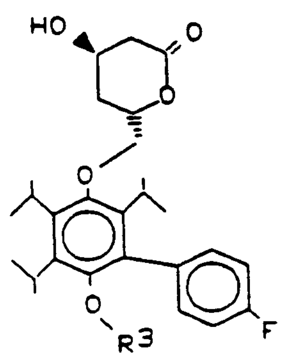

$R^3$ = hydrogen
or
acetyl

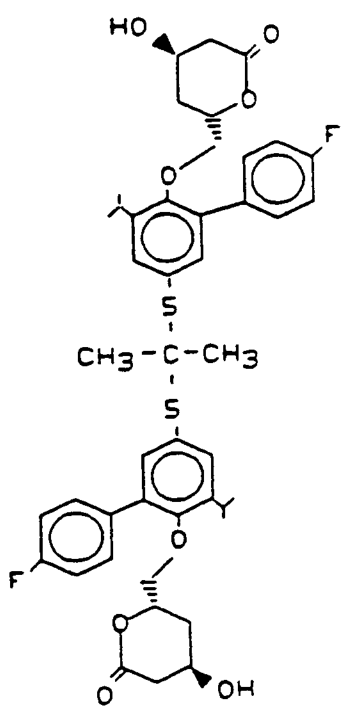

the corresponding open-chain dihydroxycarboxylic acids of the formula II, their pharmacologically tolerable salts with bases or their pharmacologically tolerable esters are prepared.

**4.** A process for the production of a pharmaceutical preparation, which comprises converting a compound obtainable as claimed in claim 1 into a suitable administration form.

**5.** A compound of the formula III

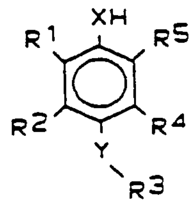

III

in which $R^1$, $R^2$, $R^3$, $R^4$, $R^5$, X and Y have the meanings indicated in claim 1 for formula I.

**Revendications**

**Revendications pour les Etats contractants suivants : AT, BE, CH, DE, DK, FR, GB, IT, LI, LU, NL, SE**

**1.** 4-hydroxytétrahydropyranne-2-ones de formule I

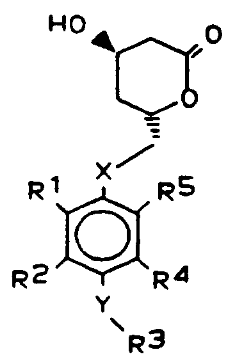

I

dans laquelle

X et Y sont identiques ou différents et représentent un atome d'oxygène ou un atome de soufre,

$R^1$ et $R^5$ représentent chacun le radical isopropyle ou sont différents et représentent un radical isopropyle, cyclopropyle ou phényle, ce dernier pouvant être 1 à 3 fois substitué sur le noyau par un ou des atomes de fluor, chlore ou brome ou groupes trifluorométhyle et/ou alkyle ou alcoxy ayant chacun de 1 à 4 atomes de carbone,

$R^2$ et $R^4$ sont identiques ou différents et représentent un atome d'hydrogène ou un radical isopropyle, cyclopropyle ou phényle, ce dernier pouvant être 1 à 3 fois substitué sur le noyau par un ou des atomes de fluor, chlore ou brome ou groupes trifluorométhyle et/ou alkyle ou alcoxy ayant de 1 à 4 atomes de carbone,

$R^3$ représente

a) un atome d'hydrogène ou le groupe méthyle ou éthyle,

b) un radical alkyle à chaîne droite ou ramifiée ayant de 3 à 8 atomes de carbone, qui peut être substitué par un groupe de formule

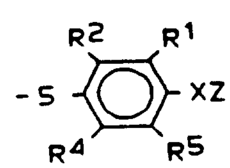

dans laquelle X, $R^1$, $R^2$, $R^4$, $R^5$ ont les significations données ci-dessus, et Z représente un atome d'hydrogène ou un cation pharmacologiquement acceptable ou le radical 4(R)-hydroxy-6-(S)-méthylène-3,4,5,6-tétrahydro-2H-pyranne-2-one de formule

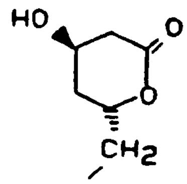

ou le radical 3(R),5(S)-dihydroxyhexane-6-oyle correspondant de formule

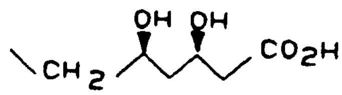

,

ses sels pharmaceutiquement acceptables avec des bases ou ses esters pharmaceutiquement acceptables,

c) un radical cycloalkyle ayant de 3 à 8 atomes de carbone, ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou

d) le radical acétyle, avec la condition que Y soit un atome d'hydrogène,

et acides dihydroxycarboxyliques à chaîne ouverte correspondants, de formule II

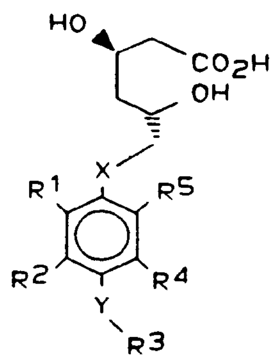

II

dans laquelle $R^1$, $R^2$, $R^3$, $R^4$, $R^5$, X et Y ont les significations données,

leurs sels pharmacologiquement acceptables avec des bases et leurs esters pharmacologiquement acceptables.

**2.** Composés selon la revendication 1, caractérisés en ce que, dans les formules I ou II,

X représente un atome d'oxygène,

Y représente un atome d'oxygène ou de soufre,

$R^1$ représente le radical isopropyle ou cyclopropyle,

$R^2$ représente un atome d'hydrogène ou le radical isopropyle ou p-fluorophényle,

$R^3$ représente un atome d'hydrogène, le radical acétyle, isopropyle, p-fluorophényle ou l'un des radicaux

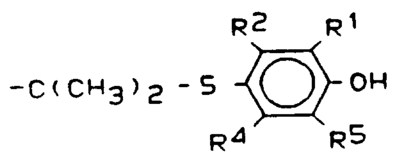

,

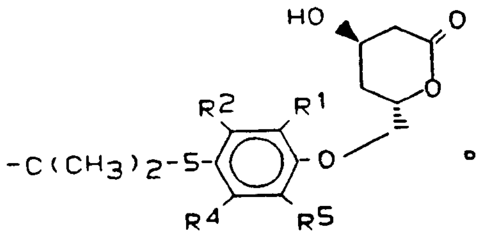

ou

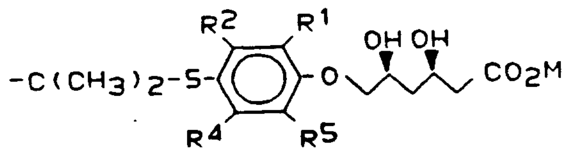

(M étant un atome d'hydrogène ou de sodium)

$R^4$ représente un atome d'hydrogène ou le groupe isopropyle ou p-fluorophényle,

$R^5$ représente le groupe isopropyle ou p-fluorophényle.

**3.** Composés selon la revendication 1, caractérisés en ce qu'ils possèdent l'une des formules (I) suivantes

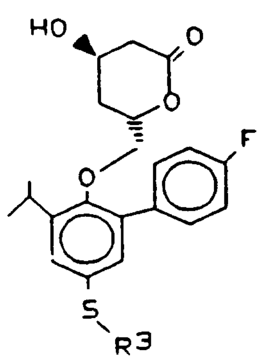

$R^3$ = p-fluorophényle, p-fluoro-m-méthyl-phényle ou isopropyle

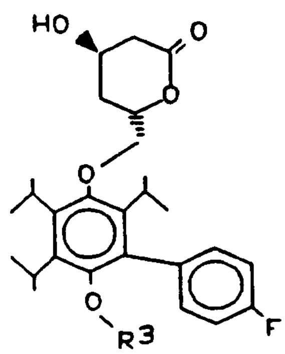

$R^3$ = H ou acétyle

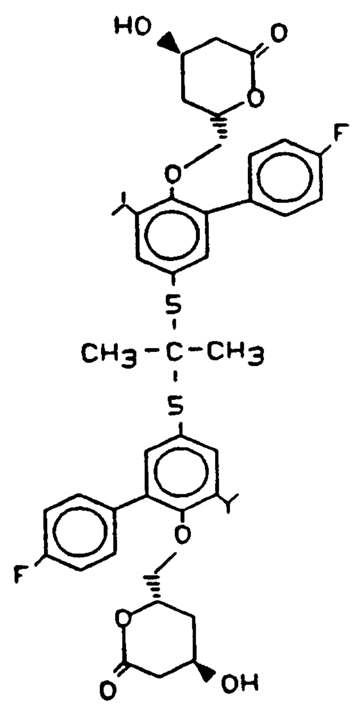

et acides dihydroxycarboxyliques à chaîne ouverte correspondants, de formule générale II, leurs sels pharmacologiquement acceptables avec des bases ou leurs esters pharmacologiquement acceptables.

**4.** Procédé pour la préparation de composés selon la revendication 1, caractérisé en ce que

a) on fait réagir des phénols ou thiophénols convenablement substitués, de formule III

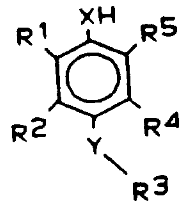

III

dans laquelle X, Y, $R^1$, $R^2$, $R^3$, $R^4$ et $R^5$ ont les significations données à propos de la formule I, avec le mésylate optiquement pur de formule IV

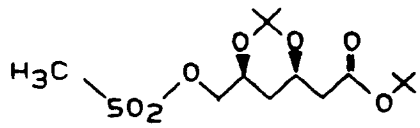

IV

pour aboutir à l'acétonide de formule V

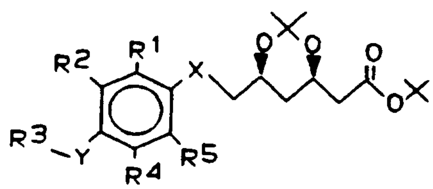

V

b) on convertit des composés de formule V, par élimination du groupe protecteur, en $\beta,\delta$-dihydroxycarboxylates de tert-butyle, de formule II/1

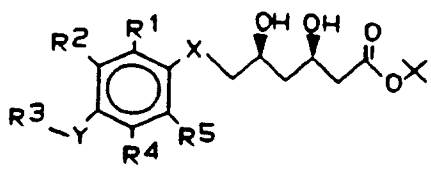

II/1

dans laquelle X, Y, $R^1$, $R^2$, $R^3$, $R^4$ et $R^5$ ont les significations données à propos de la formule I,

c) on saponifie les esters tert-butyliques de formule II/1 en sels de formule II/2

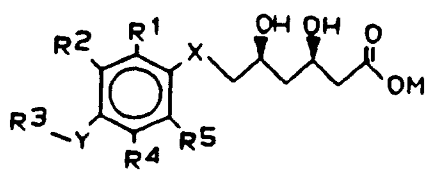

II/2

dans laquelle X, Y, $R^1$, $R^2$, $R^3$, $R^4$ et $R^5$ ont les significations données à propos de la formule I, et M représente un cation pharmacologiquement acceptable, des groupes protecteurs éventuellement présents pouvant également être éliminés,

d) on soumet à une cyclisation les esters tert-butyliques de formule II/1, ou éventuellement les sels de formule II/2, pour aboutir aux $\beta$-hydroxylactones de formule I

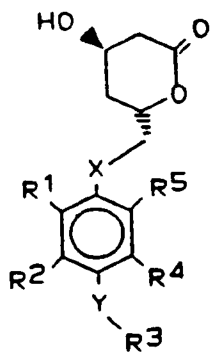

I

e) éventuellement on convertit les hydroxylactones de formule I en les acides dihydroxycarboxyliques à chaîne ouverte correspondants, de formule II, leurs sels ou leurs esters, éventuellement on convertit les sels ou esters en les acides dihydroxycarboxyliques libres de formule II ou éventuellement on convertit les acides dihydroxycarboxyliques II en les sels ou esters.

**5.** Composition pharmaceutique caractérisée en ce qu'elle contient un composé selon la revendication 1.

**6.** Composés selon la revendication 1, pour la prophylaxie et le traitement de l'artériosclérose et de l'hypercholestérolémie.

**7.** Composés de formule III

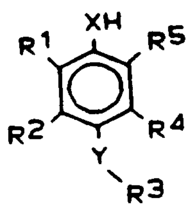

III

dans laquelle $R^1$, $R^2$, $R^3$, $R^4$, $R^5$, X et Y ont les significations données à propos de la formule I dans la revendication 1.

**Revendications pour l'Etat contractant suivant : ES**

**1.** Procédé pour la préparation de 4-hydroxytetrahydropyranne-2-ones de formule I

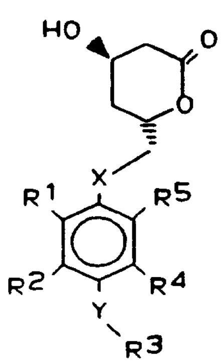

I

dans laquelle

X et Y sont identiques ou différents et représentent un atome d'oxygène ou un atome de soufre,

$R^1$ et $R^5$ représentent chacun le radical isopropyle ou sont différents et représentent un radical isopropyle, cyclopropyle ou phényle, ce dernier pouvant être 1 à 3 fois substitué sur le

noyau par un ou des atomes de fluor, chlore ou brome ou groupes trifluorométhyle et/ou alkyle ou alcoxy ayant chacun de 1 à 4 atomes de carbone,

$R^2$ et $R^4$ sont identiques ou différents et représentent un atome d'hydrogène ou un radical isopropyle, cyclopropyle ou phényle, ce dernier pouvant être 1 à 3 fois substitué sur le noyau par un ou des atomes de fluor, chlore ou brome ou groupes trifluorométhyle et/ou alkyle ou alcoxy ayant de 1 à 4 atomes de carbone,

$R^3$ représente

a) un atome d'hydrogène ou le groupe méthyle ou éthyle,

b) un radical alkyle à chaîne droite ou ramifiée ayant de 3 à 8 atomes de carbone, qui peut être substitué par un groupe de formule

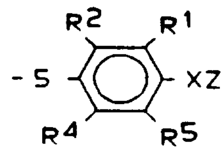

dans laquelle X, $R^1$, $R^2$, $R^4$, $R^5$ ont les significations données ci-dessus, et Z représente un atome d'hydrogène ou un cation pharmacologiquement acceptable ou le radical 4(R)-hydroxy-6-(S)-méthylène-3,4,5,6-tétrahydro-2H-pyranne-2-one de formule

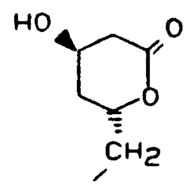

ou le radical 3(R),5(S)-dihydroxyhexane-6-oyle correspondant de formule

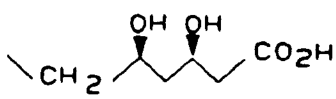 ,

ses sels pharmaceutiquement acceptables avec des bases ou ses esters pharmaceutiquement acceptables,

c) un radical cycloalkyle ayant de 3 à 8 atomes de carbone, ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou

d) le radical acétyle, avec la condition que Y soit un atome d'hydrogène,

des acides dihydroxycarboxyliques à chaîne ouverte correspondants, de formule II

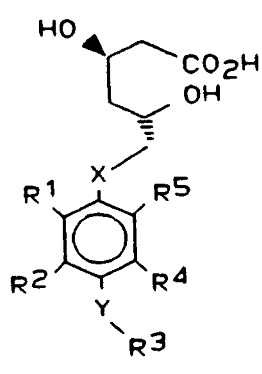

II

dans laquelle $R^1$, $R^2$, $R^3$, $R^4$, $R^5$, X et Y ont les significations données,
de leurs sels pharmacologiquement acceptables avec des bases et de leurs esters pharmacologiquement acceptables, caractérisé en ce que

a) on fait réagir des phénols ou thiophénols convenablement substitués, de formule III

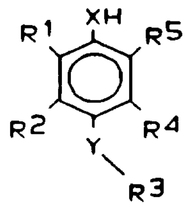

III

dans laquelle X, Y, $R^1$, $R^2$, $R^3$, $R^4$ et $R^5$ ont les significations données à propos de la formule I, avec le mésylate optiquement pur de formule IV

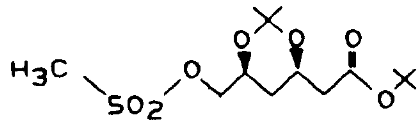

IV

pour aboutir à l'acétonide de formule V

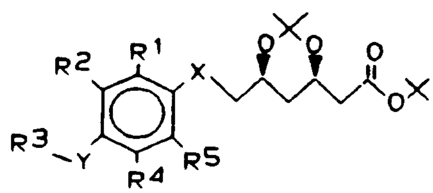

V

b) on convertit des composés de formule V, par élimination du groupe protecteur, en $\beta,\delta$-dihydroxycarboxylates de tert-butyle, de formule II/1

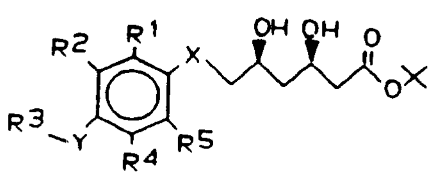

II/1

dans laquelle X, Y, $R^1$, $R^2$, $R^3$, $R^4$ et $R^5$ ont les significations données à propos de la formule I,

c) on saponifie les esters tert-butyliques de formule II/1 en sels de formule II/2

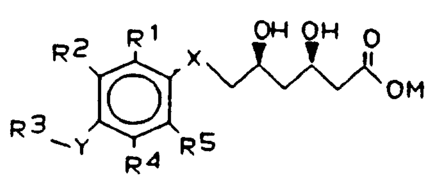

II/2

dans laquelle X, Y, $R^1$, $R^2$, $R^3$, $R^4$ et $R^5$ ont les significations données à propos de la formule I, et M représente un cation pharmacologiquement acceptable, des groupes protecteurs éventuellement présents pouvant également être éliminés,

d) on soumet à une cyclisation les esters tert-butyliques de formule II/1, ou éventuellement les sels de formule II/2, pour aboutir aux $\beta$-hydroxylactones de formule I

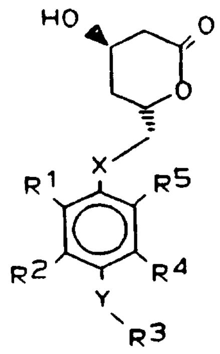

I

e) éventuellement on convertit les hydroxylactones de formule I en les acides dihydroxycarboxyliques à chaîne ouverte correspondants, de formule II, leurs sels ou leurs esters, éventuellement on convertit les sels ou esters en les acides dihydroxycarboxyliques libres de formule II ou éventuellement on convertit les acides dihydroxycarboxyliques II en les sels ou esters.

**2.** Procédé selon la revendication 1, caractérisé en ce que l'on prépare des composés de formules I ou II dans lesquels

X représente un atome d'oxygène,
Y représente un atome d'oxygène ou de soufre,
$R^1$ représente le radical isopropyle ou cyclopropyle,
$R^2$ représente un atome d'hydrogène ou le radical isopropyle ou p-fluorophényle,
$R^3$ représente un atome d'hydrogène, le radical acétyle, isopropyle, p-fluorophényle ou l'un des radicaux

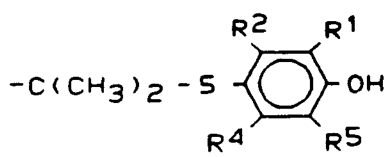

,

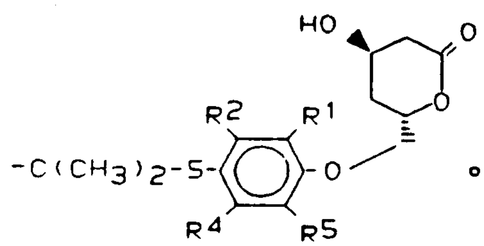

ou

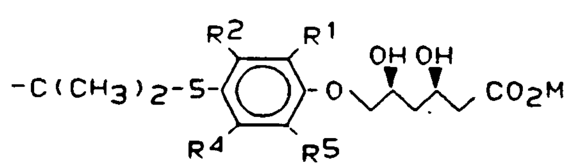

(M étant un atome d'hydrogène ou de sodium)

$R^4$ représente un atome d'hydrogène ou le groupe isopropyle ou p-fluorophényle,
$R^5$ représente le groupe isopropyle ou p-fluorophényle,

leurs sels pharmacologiquement acceptables avec des bases ou leurs esters pharmacologiquement acceptables.

**3.** Procédé selon la revendication 1, caractérisé en ce l'on prépare un composé de formules (I) suivantes

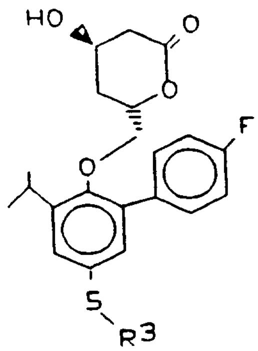

$R^3$ = p-fluorophényle, p-fluoro-m-méthylphényle ou isopropyle

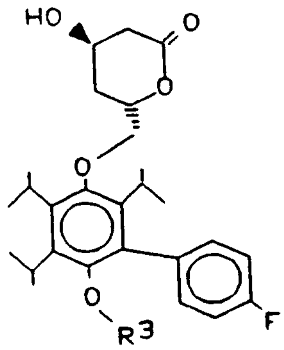

$R^3$ = H ou acétyle

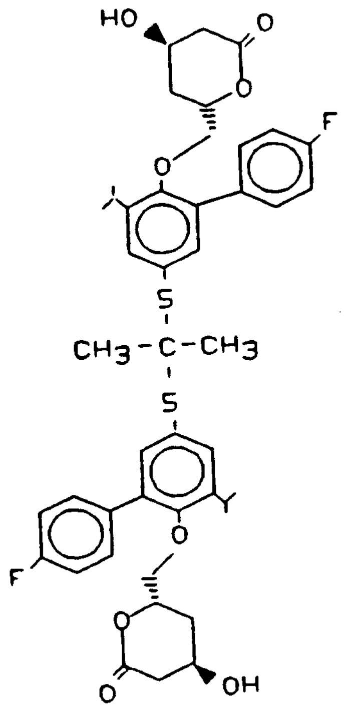

les acides dihydroxycarboxyliques à chaîne ouverte correspondants, de formule générale II, leurs sels pharmacologiquement acceptables avec des bases ou leurs esters pharmacologiquement acceptables.

**4.** Procédé pour la préparation d'une composition pharmaceutique, caractérisé en ce que l'on met sous une forme d'administration appropriée un composé préparable selon la revendication 1.

**5.** Procédé selon la revendication 1, caractérisé en ce que l'on isole des composés de formule III

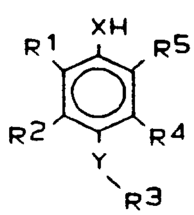

III

dans laquelle $R^1$, $R^2$, $R^3$, $R^4$, $R^5$, X et Y ont les significations données à propos de la formule I dans la revendication 1.

**Revendications pour l'Etat contractant suivant : GR**

1. Procédé pour la préparation de 4-hydroxytétrahydropyranne-2-ones de formule I

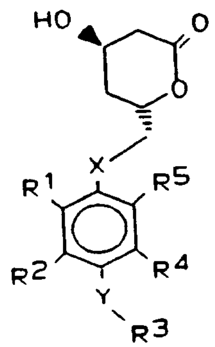

I

dans laquelle

X et Y sont identiques ou différents et représentent un atome d'oxygène ou un atome de soufre,

$R^1$ et $R^5$ représentent chacun le radical isopropyle ou sont différents et représentent un radical isopropyle, cyclopropyle ou phényle, ce dernier pouvant être 1 à 3 fois substitué sur le noyau par un ou des atomes de fluor, chlore ou brome ou groupes trifluorométhyle et/ou alkyle ou alcoxy ayant chacun de 1 à 4 atomes de carbone,

$R^2$ et $R^4$ sont identiques ou différents et représentent un atome d'hydrogène ou un radical isopropyle, cyclopropyle ou phényle, ce dernier pouvant être 1 à 3 fois substitué sur le noyau par un ou des atomes de fluor, chlore ou brome ou groupes trifluorométhyle et/ou alkyle ou alcoxy ayant de 1 à 4 atomes de carbone,

$R^3$ représente

a) un atome d'hydrogène ou le groupe méthyle ou éthyle,

b) un radical alkyle à chaîne droite ou ramifiée ayant de 3 à 8 atomes de carbone, qui peut être substitué par un groupe de formule

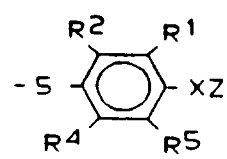

dans laquelle X, $R^1$, $R^2$, $R^4$, $R^5$ ont les significations données ci-dessus, et Z représente un atome d'hydrogène ou un cation pharmacologiquement acceptable ou le radical 4(R)-hydroxy-6-(S)-méthylène-3,4,5,6-tétrahydro-2H-pyranne-2-one de formule

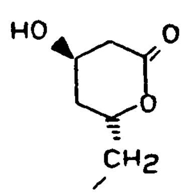

ou le radical 3(R),5(S)-dihydroxyhexane-6-oyle correspondant de formule

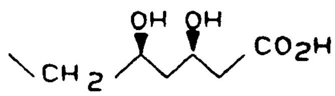

,

ses sels pharmaceutiquement acceptables avec des bases ou ses esters pharmaceutiquement acceptables,

c) un radical cycloalkyle ayant de 3 à 8 atomes de carbone, ou un radical phényle qui peut être 1 à 3 fois substitué sur le noyau par un ou des atomes d'halogène ou groupes trifluorométhyle et/ou alkyle ou alcoxy ayant chacun de 1 à 4 atomes de carbone, ou

d) le radical acétyle, avec la condition que Y soit un atome d'hydrogène,

des acides dihydroxycarboxyliques à chaîne ouverte correspondants, de formule II

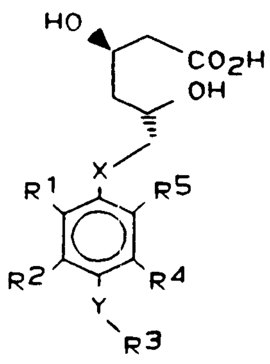

II

dans laquelle $R^1$, $R^2$, $R^3$, $R^4$, $R^5$, X et Y ont les significations données,

de leurs sels pharmacologiquement acceptables avec des bases et de leurs esters pharmacologiquement acceptables, caractérisé en ce que

a) on fait réagir des phénols ou thiophénols convenablement substitués, de formule III

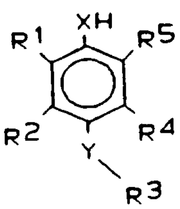

III

dans laquelle X, Y, $R^1$, $R^2$, $R^3$, $R^4$ et $R^5$ ont les significations données à propos de la formule I, avec le mésylate optiquement pur de formule IV

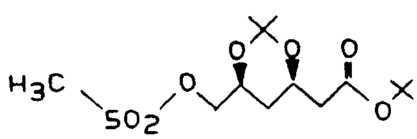

IV

pour aboutir à l'acétonide de formule V

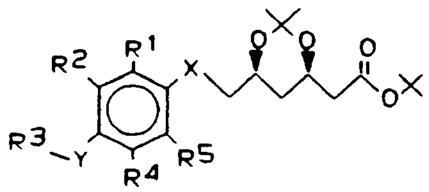

V

b) on convertit des composés de formule V, par élimination du groupe protecteur, en $\beta,\delta$-dihydroxycarboxylates de tert-butyle, de formule II/1

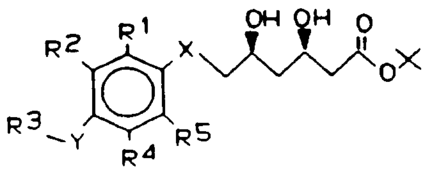

II/1

dans laquelle X, Y, $R^1$, $R^2$, $R^3$, $R^4$ et $R^5$ ont les significations données à propos de la formule I,
c) on saponifie les esters tert-butyliques de formule II/1 en sels de formule II/2

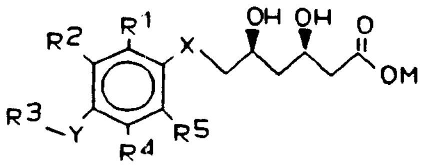

II/2

dans laquelle X, Y, $R^1$, $R^2$, $R^3$, $R^4$ et $R^5$ ont les significations données à propos de la formule I, et M représente un cation pharmacologiquement acceptable, des groupes protecteurs éventuellement présents pouvant également être éliminés,
d) on soumet à une cyclisation les esters tert-butyliques de formule II/1, ou éventuellement les sels de formule II/2, pour aboutir aux $\beta$-hydroxylactones de formule I

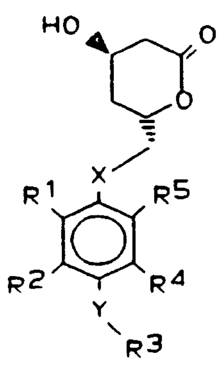

I

e) éventuellement on convertit les hydroxylactones de formule I en les acides dihydroxycarboxyliques à chaîne ouverte correspondants, de formule II, leurs sels ou leurs esters, éventuellement on convertit les sels ou esters en les acides dihydroxycarboxyliques libres de formule II ou éventuellement on convertit les acides dihydroxycarboxyliques II en les sels ou esters.

**2.** Procédé selon la revendication 1, caractérisé en ce que, l'on prépare des composés de formules I ou II dans lesquels

X    représente un atome d'oxygène,

Y représente un atome d'oxygène ou de soufre,

$R^1$ représente le radical isopropyle ou cyclopropyle,

$R^2$ représente un atome d'hydrogène ou le radical isopropyle ou p-fluorophényle,

$R^3$ représente un atome d'hydrogène, le radical acétyle, isopropyle, p-fluorophényle ou l'un des radicaux

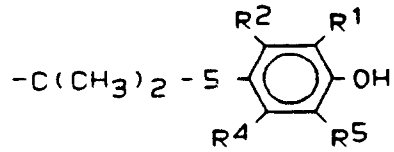

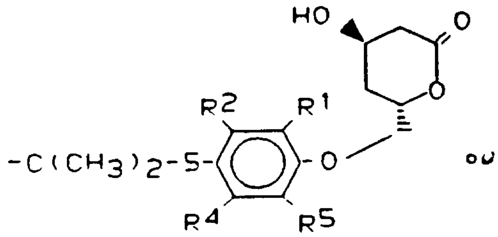

ou

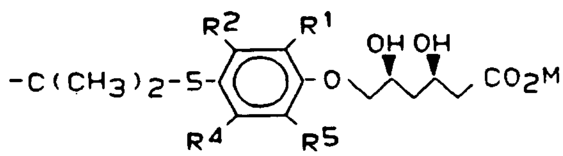

(M étant un atome d'hydrogène ou de sodium)

$R^4$ représente un atome d'hydrogène ou le groupe isopropyle ou p-fluorophényle,

$R^5$ représente le groupe isopropyle ou p-fluorophényle,

leurs sels pharmacologiquement acceptables avec des bases ou leurs esters pharmacologiquement acceptables.

**3.** Procédé selon la revendication 1, caractérisé en ce l'on prépare un composé de formules (I) suivantes

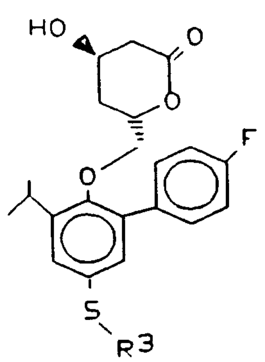

$R^3$ = p-fluorophényle, p-fluoro-m-méthylphényle ou isopropyle

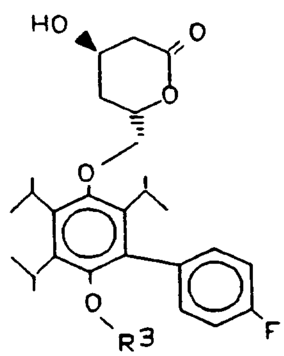

$R^3$ = H ou acétyle

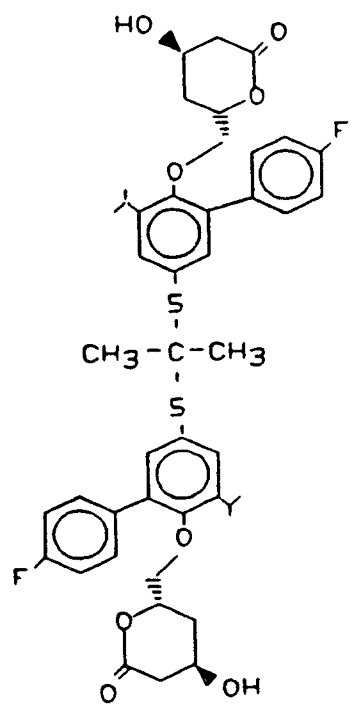

les acides dihydroxycarboxyliques à chaîne ouverte correspondants, de formule générale II, leurs sels pharmacologiquement acceptables avec des bases ou leurs esters pharmacologiquement acceptables.

**4.** Procédé pour la préparation d'une composition pharmaceutique, caractérisé en ce que l'on met sous une forme d'administration appropriée un composé préparable selon la revendication 1.

**5.** Composés de formule III

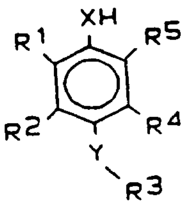

III

dans laquelle $R^1$, $R^2$, $R^3$, $R^4$, $R^5$, X et Y ont les significations données à propos de la formule I dans la revendication 1.